# EUROPÄISCHE PATENTSCHRIFT

(11) **EP 1 307 237 B1**
(45) Veröffentlichungstag und Bekanntmachung des Hinweises auf die Patenterteilung: **11.04.2007**
(21) Anmeldenummer: 01958005.9
(22) Anmeldetag: 23.07.2001
(51) Int. Cl.: A61K 49/08, A61K 49/06, A61K 51/00

(54) **PERFLUORALKYLHALTIGE KOMPLEXE MIT POLAREN RESTEN, VERFAHREN ZU DEREN HERSTELLUNG UND IHRE VERWENDUNG**
COMPLEXES CONTAINING PERFLUOROALKYL WITH POLAR RADICALS, METHOD FOR THE PRODUCTION AND USE THEREOF
COMPLEXES PERFLUOROALKYLES A RESIDUS POLAIRES, LEUR PROCEDE DE FABRICATION ET LEUR UTILISATION

(30) Priorität: 11.08.2000 DE 10040858
(43) Veröffentlichungstag der Anmeldung: 07.05.2003
(73) Patentinhaber: Bayer Schering Pharma Aktiengesellschaft, 13353 Berlin (DE)
(72) Erfinder: PLATZEK, Johannes, 12621 Berlin (DE); MARESKI, Peter, 13407 Berlin (DE); NIEDBALLA, Ulrich, 14195 Berlin (DE); RADÜCHEL, Bernd, 13465 Berlin (DE); WEINMANN, Hanns-Joachim, 14129 Berlin (DE); MISSELWITZ, Bernd, 16548 Glienicke (DE)
(86) Internationale Anmeldenummer: PCT/EP2001/008500
(87) Internationale Veröffentlichungsnummer: WO 2002/013875

(56) Entgegenhaltungen:
- WO-A-99/16474
- DE-A- 19 603 033
- DE-A- 19 608 278
- DE-A- 19 729 013

## Beschreibung

Die Erfindung betrifft die in den Patentansprüchen gekennzeichneten Gegenstände, nämlich perfluoralkylhaltige Metallkomplexe mit polaren Resten der allgemeinen Formel I, Verfahren zu deren Herstellung und ihre Verwendung in der NMR-und Röntgendiagnostik, Radiodiagnostik und Radiotherapie, in der MRT-Lymphographie sowie als blood-pool-agents. Die erfindungsgemäßen Verbindungen sind in ganz besonderer Weise für die intravenöse Lymphographie, für die Tumor-Diagnostik und für das Infarkt- und Nekrose-Imaging geeignet.

In der kernmagnetischen Resonanz kommt nach dem Element Wasserstoff dem Element Fluor die größte Bedeutung zu.
1) Fluor hat eine hohe Empfindlichkeit von 83 % der des Wasserstoffs.
2) Fluor hat nur ein NMR-aktives Isotop.
3) Fluor hat eine dem Wasserstoff ähnliche Resonanzfrequenz - Fluor und Wasserstoff können mit der gleichen Anlage gemessen werden.
4) Fluor ist biologisch inert.
5) Fluor kommt in biologischem Material (Ausnahme: Zähne) nicht vor und kann deshalb als Sonde oder Kontrastmittel vor einem störsignalfreiem Untergrund eingesetzt werden.

Diese Eigenschaften führten dazu, daß Fluor einen breiten Raum in der diagnostischen Patentliteratur mit der magnetischen Kernresonanz als Grundlage einnimmt: Fluor-19-imaging, Funktionsdiagnose, Spektroskopie.

So werden in dem US Patent 4,639,364 (Mallinckrodt) Trifluormethansulfonamide als Kontrastmittel für das Fluor-19-imaging vorgeschlagen:

CF₃SO₂NH₂

CF₃SO₂NH-CH₂-(CHOH) ₄-CH₂OH

Ebenfalls mit dem Fluor-19-imaging beschäftigt sich das deutsche Patent DE 4203254 (Max-Planck-Gesellschaft), in dem ein Anilinderivat vorgeschlagen wird:

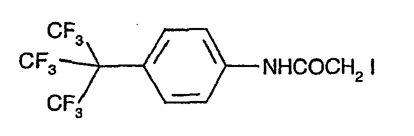

Das Fluor-19-imaging ist Gegenstand der Anmeldung WO 93/07907 (Mallinckrodt), in der ebenfalls Phenylderivate als Kontrastmittel beansprucht werden:

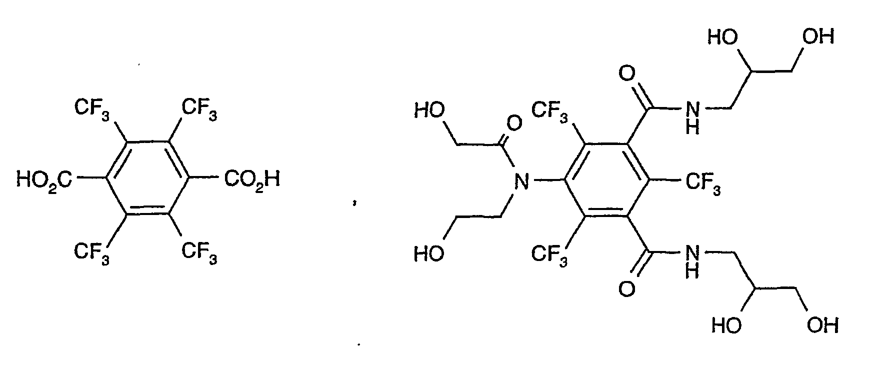

Für das Fluor-19-imaging werden auch erheblich einfacher gebaute Verbindungen beansprucht. So wird im Patent US 4,586,511 (Children's Hospital Medical Center) das Perfluoroctylbromid

CF₃(CF₂)₇-Br

genannt,
im Europäischen Patent EP 307863 (Air Products) der Perfluor-15-krone-5-ether 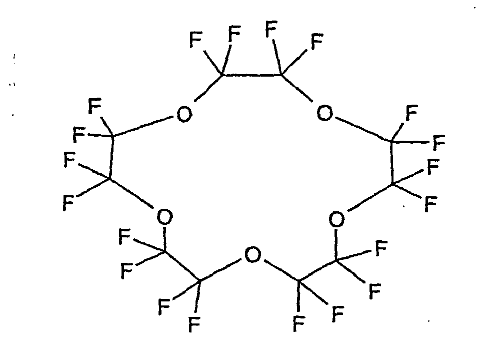 und im amerikanischen Patent US 4,588,279 (University of Cincinnati, Children's Hospital Research Foundation) Perfluorkohlenstoffverbindungen wie Perfluorcyclononan oder -octan, perfluorierte Ether wie das Tetrahydrofuran 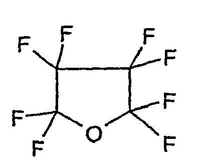 oder Diether wie der Perfluor-propylenglycol-diether

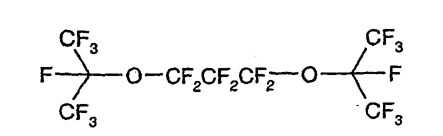

Ebenfalls für das Fluor-19-imaging dienen die in der Anmeldung WO 94/22368 (Molecular Biosystems) genannten Verbindungen z.B. 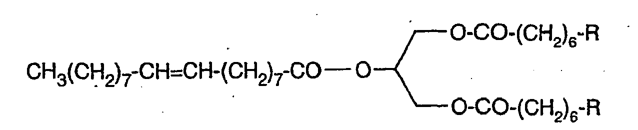 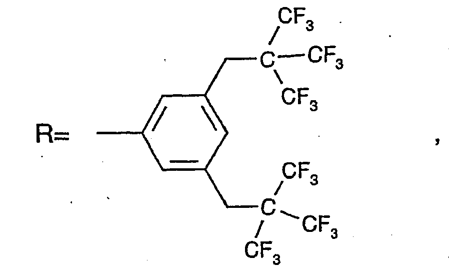 die als fluorhaltigen Rest die Perfluor-1H, 1H-neöpentylgruppe besitzen.

Einen weiteren Strukturtyp mit erweiterter diagnostischer Nutzung zeigt das amerikanische Patent US 5,362,478 (VIVORX) auf, in dem die Kombination Fluorkohlenstoff / polymere Hülle für imaging-Zwecke beansprucht wird. Genannt werden Perfluornonan und Humanserumalbumin. Diese Kombination erweist sich darüberhinaus als geeignet, das Fluoratom als Sonde für lokale Temperaturmessung und zur Bestimmung des Sauerstoffpartialdruckes einzusetzen.

Perfluorkohlenstoffe werden auch im US Patent 4,586,511 zur Sauerstoffbestimmung beansprucht.

In der deutschen Patentschrift DE 4008179 (Schering) werden fluorhaltige Benzolsulfonamide als pH-Sonden beansprucht:

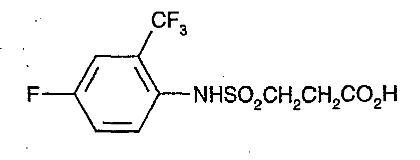

Für die NMR-Diagnostik werden auch Verbindungen, die Jod- und Fluoratome enthalten, als kontrastverstärkende Mittel beansprucht, so in WO 94/05335 und WO 94/22368 (beide Molecular Biosystems):

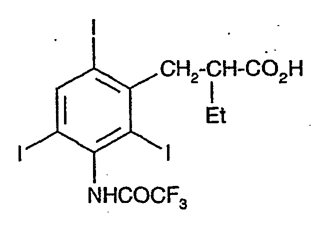

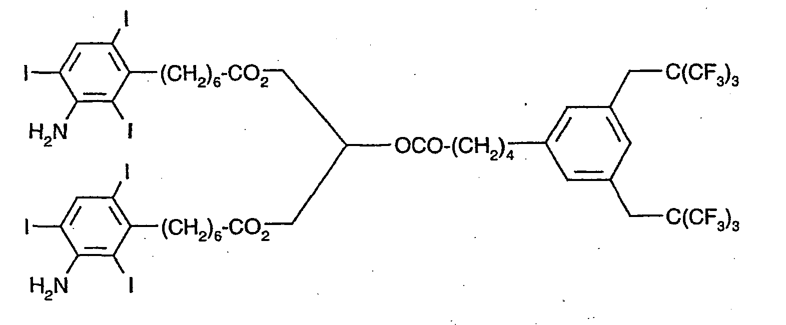

Auch die Kombination Fluor-paramagnetisches Metallion wird für das Fluor-19-imaging beansprucht, und zwar für offenkettige Komplexe in WO 94/22368 (Molecular Biosystems) mit z.B.: 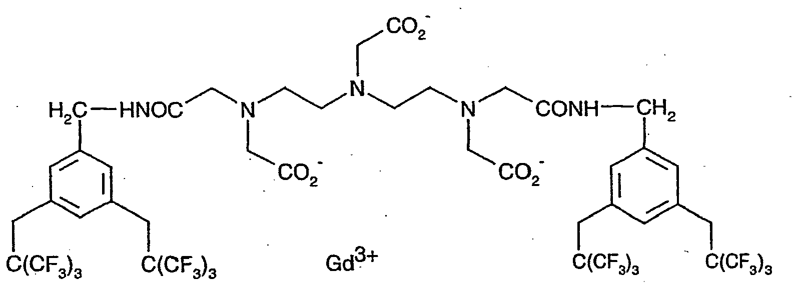 und in EP 292 306 (TERUMO Kabushiki Kaisha) mit z.B.: 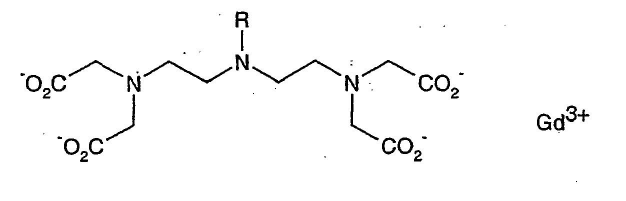 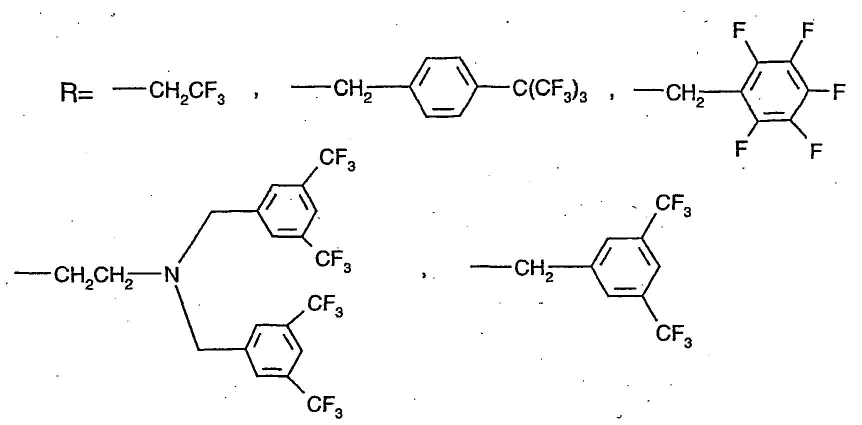 aber auch für cyclische Verbindungen, wie sie in EP 628 316 (TERUMO Kabushiki Kaisha) 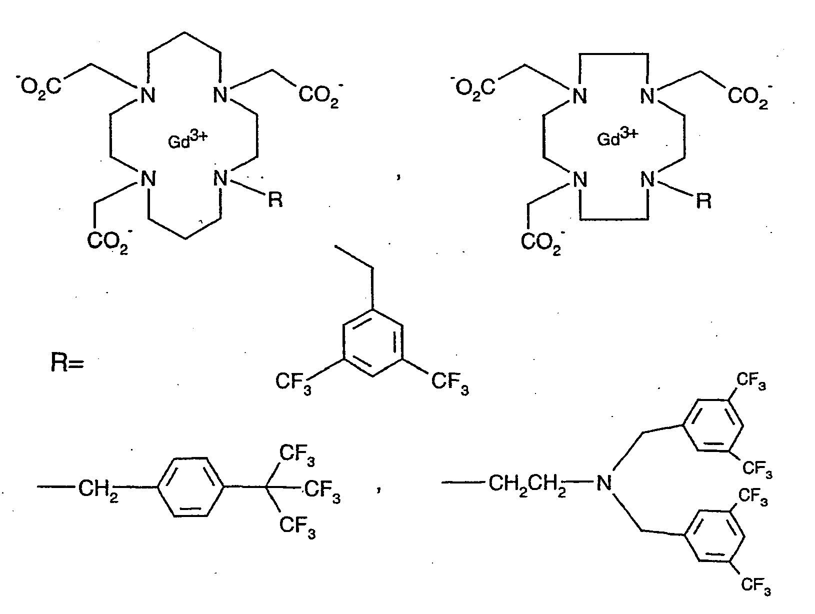 genannt werden.

Die Kombination Fluoratom-Seltenes Erdmetall wird auch für die NMR-spektroskopischen Temperaturmessungen in DE 4317588 (Schering) beansprucht:

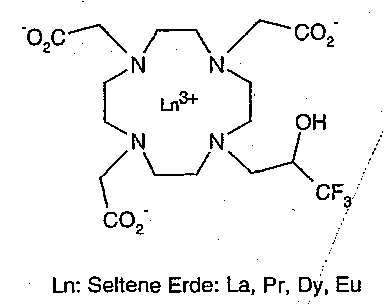

Während bei Verbindungen, die die Elemente Fluor und Jod enthalten, keine Wechselwirkungen zwischen den beiden Kernen stattfinden, findet in Verbindungen, die Fluor und paramagnetische Zentren (Radikale, Metallionen) enthalten, eine intensive Wechselwirkung statt, die sich in einer Verkürzung der Relaxationszeit des Fluorkernes äußern. Die Größe dieses Effekts hängt von der Anzahl der ungepaarten Elektronen des Metallions (Gd³⁺ > Mn²⁺ > Fe³⁺ > Cu²⁺) und von der Entfernung zwischen dem paramagnetischen Ion und dem ¹⁹F-Atom ab.

Je mehr ungepaarte Elektronen des Metallions vorhanden sind und je näher diese an das Fluor gebracht werden, desto größer ist die Verkürzung der Relaxationszeit des Fluorkernes.

Die Verkürzung der Relaxationszeit als Funktion des Abstandes vom paramagnetischen Ion macht sich bei allen Kernen mit ungerader Spinzahl bemerkbar, so auch beim Proton, und Gadoliniumverbindungen finden deshalb breite Anwendung als Kontrastmittel in der Kernspintomographie (Magnevist^{®}, Prohance^{®}, Omniscan^{®}, Dotarem^{®}).

Beim ¹H-MR-imaging (¹H-MRI) wird jedoch die Relaxationszeit T¹ oder T² der Protonen, das heißt vor allem der Protonen des Wassers, und nicht die Relaxationszeit der Fluorkerne gemessen und für die Bildgebung verwendet. Das quantitative Maß für die Verkürzung der Relaxationszeit ist die Relaxivity [L/mmol·s].

Die Kombination Fluoratom-Seltenes Erdmetall wird auch für die NMR-spektroskopischen Temperaturmessungen in DE 4317588 (Schering) beansprucht:

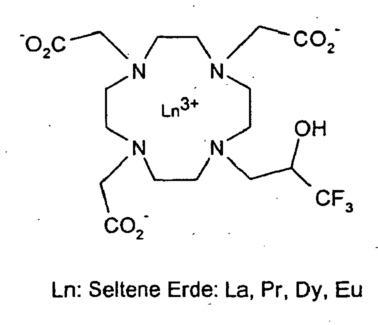

Die in DE 196 03 033, DE 196 08 278, DE 197 29 013 und WO 99/16474 offenbarten Substanzen weisen im Gegensatz zu denen der vorliegenden Erfindung keine polare Seitenkette auf.

Während bei Verbindungen, die die Elemente Fluor und Jod enthalten, keine Wechselwirkungen zwischen den beiden Kernen stattfinden, findet in Verbindungen, die Fluor und paramagnetische Zentren (Radikale, Metallionen) enthalten, eine intensive Wechselwirkung statt, die sich in einer Verkürzung der Relaxationszeit des Fluorkernes äußern. Die Größe dieses Effekts hängt von der Anzahl der ungepaarten Elektronen des Metallions (Gd³⁺ > Mn²⁺ > Fe³⁺ > Cu²⁺) und von der Entfernung zwischen dem paramagnetischen Ion und dem ¹⁹F-Atom ab.

Je mehr ungepaarte Elektronen des Metallions vorhanden sind und je näher diese an das Fluor gebracht werden, desto größer ist die Verkürzung der Relaxationszeit des Fluorkernes.

Die Verkürzung der Relaxationszeit als Funktion des Abstandes vom paramagnetischen Ion macht sich bei allen Kernen mit ungerader Spinzahl bemerkbar, so auch beim Proton, und Gadoliniumverbindungen finden deshalb breite An wendung als Kontrastmittel in der Kernspintomographie (Magnevist ^{®} Prohance^{®}, Omniscan^{®}, Dotarem^{®}).

Beim ¹H-MR-imaging (¹H-MRI) wird jedoch die Relaxationszeit T¹ oder T²der Protonen, das heißt vor allem der Protonen des Zur Verkürzung der Relaxationszeiten werden mit Erfolg Komplexe paramagnetischer Ionen eingesetzt. In der folgenden Tabelle ist die Relaxivity einiger Handelspräparate angegeben:

| | | |
|---|---|---|
| | **T¹-relaxivity in Wasser** [l/mmol·s, 39°C, 0.47 T] | **T¹-relaxivity in Plasma** [l/mmol·s, 39°C, 0.47 T] |
| **MAGNEVIST^{®}** | 3,8 | 4,8 |
| **DOTAREM^{®}** | 3,5 | 4,3 |
| **OMNISCAN^{®}** | 3,8 | 4,4 |
| **PRO HANCE^{®}** | 3,7 | 4,9 |

In diesen Verbindungen finden nur Wechselwirkungen zwischen Protonen und dem Gadoliniumion statt. Für diese Kontrastmittel in Wasser wird also eine Relaxivity von ca. 4 [1/mmol·s] beobachtet.

Es werden also erfolgreich für das MR-imaging sowohl Fluor-Verbindungen für Fluor-19-imaging, bei dem die verkürzte Relaxationszeit des Fluor-Kernes ausgenutzt wird, als auch nicht Fluor-haltige Verbindungen, bei denen die Relaxationszeit der Protonen des Wassers gemessen wird, verwendet.

Bei der Einführung eines perfluorkohlenstoffhaltigen Restes in ein paramagnetisches Kontrastmittel, das heißt bei der Kombination von Eigenschaften, die bisher nur für Fluorimaging-Verbindungen als geeignet bekannt waren, mit Verbindungen, die für Protonen-imaging verwendet wurden, steigt überraschenderweise auch die Relaxivity betreffend die Protonen des Wassers rapide an. Sie erreicht nun Werte von 10-50 [1/mmol·s] im Vergleich zu Werten zwischen 3,5 und 3,8 [1/mmol·s] wie sie für einige Handelsprodukte in obiger Tabelle bereits aufgeführt wurden.

Aus DE 196 03 033.1 sind bereits perfluoralkylhaltige Metallkomplexe bekannt. Diese Verbindungen sind jedoch nicht für alle Anwendungen befriedigend einsetzbar. So besteht nach wie vor ein Bedarf an Kontrastmitteln für die Darstellung von malignen Tumoren, von Lymphknoten und von nekrotischem Gewebe.

Maligne Tumoren metastasieren gehäuft in regionale Lymphknoten, wobei auch mehrere Lymphknotenstationen beteiligt sein können. So werden Lymphknotenmetastasen in etwa 50 - 69% aller Patienten mit malignen Tumoren gefunden (Elke, Lymphographie, in: Frommhold, Stender, Thurn (eds.), Radiologische Diagnostik in Klinik und Praxis, Band IV, Thieme Verlag Stuttgart, 7th ed., 434-496, 1984).). Die Diagnose eines metastatischen Befalls von Lymphknoten ist im Hinblick auf die Therapie und Prognose maligner Erkrankungen von großer Bedeutung. Mit den modernen bildgebenden Methoden (CT, US und MRI) werden lymphogene. Absiedlungen von malignen Tumoren nur unzureichend erkannt, da zumeist nur die Größe des Lymphknotens als Diagnosekriterium herangezogen werden kann. Damit sind kleine Metastasen in nicht-vergrößerten Lymphknoten (< 2 cm) nicht von Lymphknotenhyperplasien ohne malignen Befall zu unterscheiden (Steinkamp et al., . Sonographie und Kernspintomographie: Differentialdiagnostik von reaktiver Lymphknoten-vergrößerung und Lymphknotenmetastasen am Hals, Radiol.diagn. 33:158, 1992).

Wünschenswert wäre es, daß bei Einsatz von spezifischen Kontrastmitteln Lymphknoten mit metastatischem Befall und hyperplastische Lymphknoten unterschieden werden können.

Bekannt ist die direkte Röntgen-Lymphographie (Injektion einer öligen Kontrastmittelsuspension in ein präpariertes Lymphgefäß) als eine nur noch selten benutzte invasive Methode, die nur wenige Lymphabflußstationen darstellen kann.

Experimentell werden in Tierexperimenten auch Fluoreszenzmarkierte Dextrane verwendet, um nach deren interstitieller Applikation den Lymphabfluß beobachten zu können. Allen gebräuchlichen Markern für die Darstellung von Lymphwegen und Lymphknoten nach interstitieller/intrakutaner Applikation ist also gemein, daß es sich um Substanzen mit partikulärem Charakter ("particulates", z.B. Emulsionen und Nanokristallsuspensionen) oder große Polymere handelt (s.a. WO 90/14846). Die bisher beschriebenen Zubereitungen erwiesen sich jedoch aufgrund ihrer mangelnden lokalen und systemischen Verträglichkeit sowie ihrer geringen Lymphgängigkeit, die eine unzureichende diagnostischen Effizienz bedingt, als noch nicht optimal für die indirekte Lymphographie geeignet.

Da die Darstellung von Lymphknoten von zentraler Bedeutung für die frühe Erkennung des metastatischen Befalls bei Krebspatienten ist, besteht ein großer Bedarf an lymphspezifischen Kontrastmittelzubereitungen zur Diagnose entsprechender Veränderungen des Lymphsystems.

Möglichst hohe Kontrastmittelbeladung und hohe Stabilität sind ebenso wünschenswert wie diagnostisch relevante, möglichst gleichmäßige Lymphanreicherung über mehrere Lymphstationen hinweg. Die Belastung des Gesamtorganismus sollte durch rasche und vollständige Ausscheidung des Kontrastmittels gering gehalten werden. Ein rascher Wirkungseintritt möglichst bereits innerhalb weniger Stunden nach Kontrastmittelapplikation ist für die radiologische Praxis von Bedeutung. Eine gute Verträglichkeit ist notwendig.

Nicht zuletzt ist es wünschenswert, lymphspezifische Kontrastmittel zur Verfügung zu haben, die es erlauben, in einer diagnostischen Sitzung sowohl den Primärtumor als auch eine mögliche Lymphknotenmetastasierung zur Darstellung zu bringen.

Ein anderer wichtiger Bereich in der Medizin ist die Detektion, Lokalisierung und Überwachung von Nekrosen oder Infarkten. So ist der Myokardinfarkt nicht ein stationärer Vorgang, sondern ein dynamischer Prozeß, der sich über einen längeren Zeitraum (Wochen bis Monate) erstreckt. Die Erkrankung verläuft in etwa drei Phasen, die nicht scharf voneinander getrennt, sondern überlappend sind. Die erste Phase, die Entwicklung des Myokardinfarktes, umfaßt die 24 Stunden nach dem Infarkt, in .denen die Zerstörung wie eine Stoßwelle (Wellenfrontphänomen) vom Subendokard zum Myokard fortschreitet. Die zweite Phase, der bereits bestehende Infarkt, umfaßt die Stabilisierung des Bereiches, in dem Faserbildung (Fibrose) als Heilprozeß erfolgt. Die dritte Phase, der ausgeheilte Infarkt, beginnt, nachdem alles zerstörte Gewebe durch fibröses Narbengewebe ersetzt ist. Während dieser Periode findet eine umfangreiche Restrukturierung statt.
Bis heute ist kein präzises und zuverlässiges Verfahren bekannt, das die aktuelle Phase eines Myokardinfarktes am lebenden Patienten diagnostizierbar macht. Für die Beurteilung eines Myokardinfarktes ist es von entscheidender Bedeutung, zu wissen, wie groß der Anteil des bei dem Infarkt verlorenen Gewebes ist und an welcher Stelle der Verlust erfolgte, denn von dieser Kenntnis hängt die Art der Therapie ab.
Infarkte erfolgen nicht nur im Myokard, sondern auch in anderen Geweben, besonders im Hirn.
Während der Infarkt in gewissem Umfang heilbar ist, können bei einer Nekrose, dem lokal begrenzten Gewebetod, nur die schädlichen Folgen für den Restorganismus verhindert oder wenigstens gemildert werden. Nekrosen können auf vielfache Weise entstehen: durch Verletzungen, Chemikalien, Sauerstoffdefizit oder duch Strahlung. Wie beim Infarkt ist die Kenntnis von Umfang und Art einer Nekrose wichtig für das weitere ärztliche Vorgehen.
Schon früh erfolgten daher Versuche, die Lokalisation von Infarkten und Nekrosen durch Einsatz von Kontrastmitteln bei nichtinvasiven Verfahren wie Szintigraphie oder Kernspintomographie zu verbessern. In der Literatur nehmen die Versuche, Porphyrine für das Nekroseimaging einzusetzen, einen großen Raum ein. Die erzielten Resultate ergeben jedoch ein widersprüchliches Bild. So beschreiben Winkelman und Hoyes in Nature, 200, 903 (1967), daß sich Mangan-5,10,15,20-Tetrakis(4-sulfonatophenyl)-porphyrin (TPPS) selektiv im nekrotischen Teil eines Tumors anreichert.
Lyon et al. (Magn. Res. Med. 4, 24 (1987)) dagegen beobachteten, daß sich Mangan-TPPS im Körper verteilt, und zwar in Niere, Leber, Tumor und nur zu einem geringen Teil in den Muskeln. Interessant ist dabei, daß die Konzentration im Tumor erst am 4. Tag ihr Maximum erreicht und das auch nur, nachdem, die Autoren die Dosis von 0.12 mmol/kg auf 0.2 mmol/kg gesteigert hatten. Die Autoren sprechen daher auch von einer nichtspezifischen Aufnahme des TPPS in den Tumor. Bockhurst et al. wiederum berichten in Acta Neurochir 60, 347 (1994, Suppl.), daß MnTPPS selektiv an Tumorzellen bindet.
Foster et al. (J. Nucl. Med. 26, 756 (1985)) ihrerseits fanden, daß sich 111 In-5,10,15,20-Tetrakis-(4-N-methyl-pyridinium)-Porphyrin (TMPyP) nicht im nekrotischen Teil, sondern in den lebenden Randschichten anreichert. Daraus zu folgern, daß eine Porphyrin-Gewebe-Wechselwirkung besteht, ist naheliegend, aber nicht zwingend.
In Circulation Vol. 90, No. 4, Teil 2, Seite 1468, Abstract No. 2512 (1994) berichten Ni et al., daß sie mit einem Mangan-Tetraphenyl-Porphyrin (Mn-TPP) und einem Gadolinium-Mesoporphyrin (Gd-MP) Infarktbereiche darstellen können. In der internationalen Patentanmeldung WO 95/31219 wurden beide Substanzen zum Infarkt- und Nekroseimaging eingesetzt. Die Autoren Marchal und Ni schreiben (siehe Beispiel 3), daß für die Verbindung Gd-MP der Metallgehalt der Infarkt-Niere ähnlich hoch war wie der des nichtinfarzierten Organs, daß er jedoch für das Myokard beim infarzierten Gewebe (Beispiel 1) neunmal so groß war. Erstaunlich war, daß das Verhältnis der Signalintensitäten beim MRI für infarziertes im Vergleich zum gesunden Gewebe in beiden Fällen mit 2.10 bzw. 2.19 vergleichbar hoch war. Weitere Metalloporphyrine sind in der Anmeldung DE 19835082 (Schering AG) beschrieben werden.
Porphyrine neigen dazu, sich in der Haut abzulagern, was zu einer Photosensibilisierung führt. Die Sensibilisierung kann Tage, ja sogar Wochen andauern. Dies ist ein unerwünschter Nebeneffekt bei der Verwendung von Porphyrinen als Diagnostika. Außerdem ist der therapeutische Index für die Porphyrine nur sehr klein, da z.B. für Mn-TPPS eine Wirkung erst bei einer Dosis von 0.2 mmol/kg einsetzt, die LD₅₀ aber bereits bei 0.5 mmol/kg liegt.

Nicht vom Porphyringerüst abgeleitete Kontrastmittel für das Nekrose- und Infarkt-Imaging sind in DE 19744003 (Schering AG), DE 19744004 (Schering AG) und WO 99/17809 (EPIX) beschrieben. Bisher gibt es aber noch keine Verbindungen, die befriedigend als Kontrastmittel beim Infarkt- und Nekrose-imaging eingesetzt werden können.

Aufgabe der Erfindung war es deshalb, Kontrastmittel zur Verfügung zu stellen, die insbesondere für die MRT-Lymphographie, aber auch für die Tumor-Diagnostik und das Nekrose-und Infarkt-Imaging einsetzbar sind.

Die Aufgabe der Erfindung wird durch die perfluoralkylhaltigen Komplexe mit polaren Resten der allgemeinen Formel I 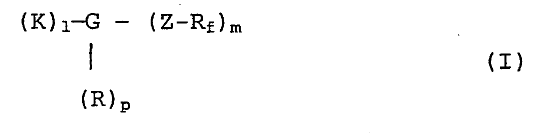 in der
- R_{f}: eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
- K: für einen Metallkomplex der allgemeinen Formel II steht, 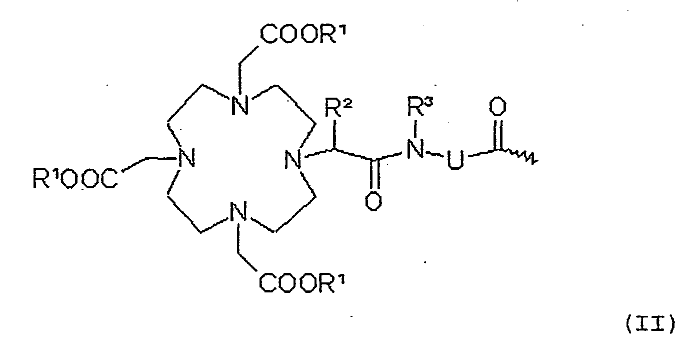
in der
- R¹: ein Wasserstoffatom oder ein Metallionenäquivalent der Ordnungszahlen 21-29, 31-33, 37-39, 42-44, 49 oder 57-83 bedeutet,
mit der Maßgabe, daß mindestens zwei R¹ für Metallionenäquivalente stehen,
- R² und R³: unabhängig voneinander Wasserstoff, C₁-C₇-Alkyl, Benzyl, Phenyl, -CH₂OH oder -CH₂OCH₃ darstellen und
- U: -C₆H₄-O-CH₂-ω-, -(CH₂)₁₋₅-ω, eine Phenylengruppe, -CH₂-NHCO-CH₂-CH(CH₂COOH) -C₆H₄-ω-, -C₆H₄- (OCH₂CH₂) ₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3 - (CH₂) ₀₋₅COOH-Gruppen substituierte C₁-C₁₂-Alkylen-oder C₇-C₁₂-C₆H₄-0-Gruppe darstellt, wobei ω für die Bindungsstelle an -CO- steht,
oder
der allgemeinen Formel III 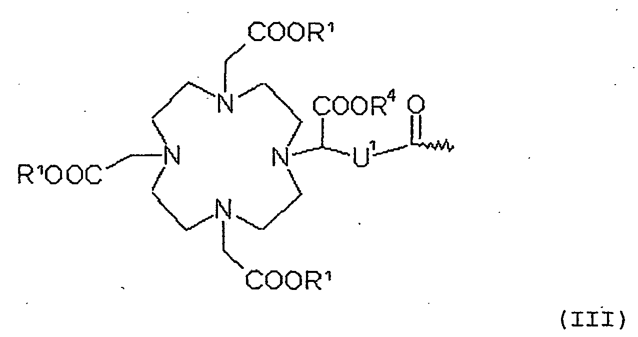 in der R¹ die oben genannte Bedeutung hat, R⁴ Wasserstoff oder ein unter R¹ genanntes Metallionenäquivalent darstellt und U¹ - C₆H₄-O-CH₂-ω- darstellt, wobei ω die Bindungsstelle an -CObedeutet
oder der allgemeinen Formel IV 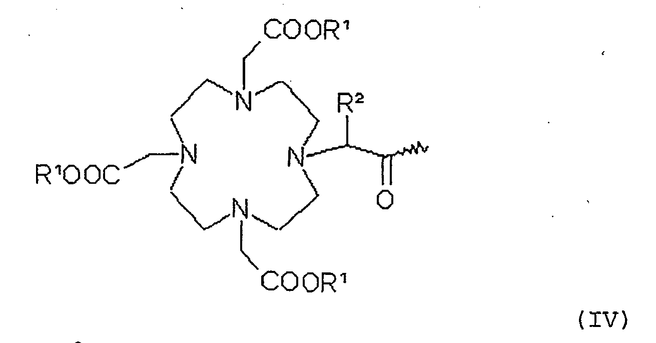 in der R¹ und R² die oben genannte Bedeutung haben
oder der allgemeinen Formel V A oder V B 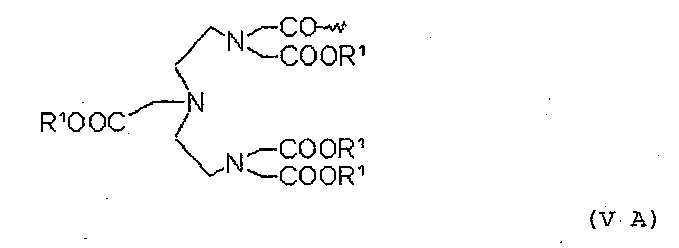 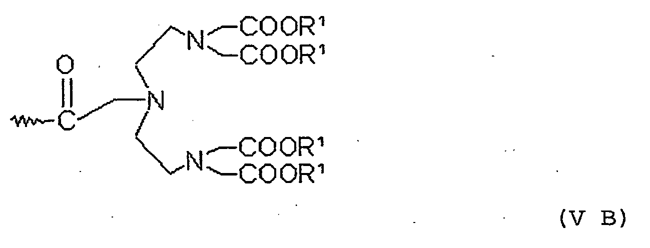 in der R¹ die oben genannte Bedeutung hat,
oder der allgemeinen Formel VI 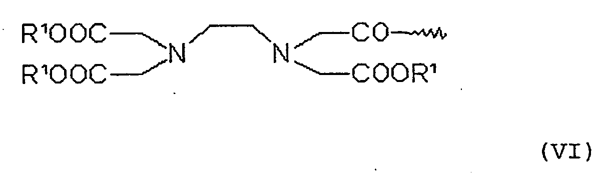 in der R¹ die oben genannte Bedeutung hat,
oder der allgemeinen Formel VII 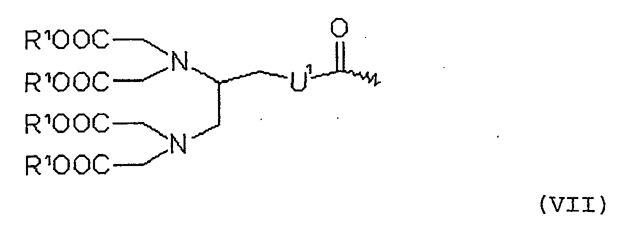 in der R¹ die oben genannte Bedeutung hat und
- U¹: -C₆H₄-O-CH₂-ω- darstellt, wobei ω die Bindungsstelle an -CO-bedeutet
und im Rest K gegebenenfalls vorhandene freie Säuregruppen gegebenenfalls als Salze organischer und/oder anorganischer Basen oder Aminosäuren oder Aminosäureamide vorliegen können,
- G: einen mindestens dreifach funktionalisierten Rest ausgewählt aus den nachfolgenden Resten a) bis g) darstellt

(a) 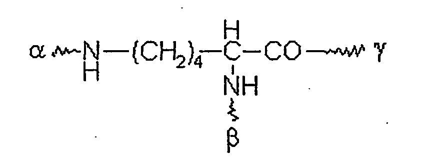
(b) 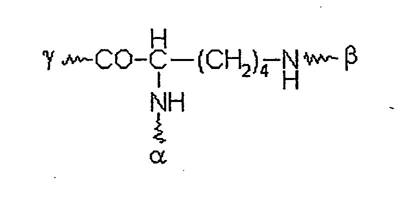
(c) 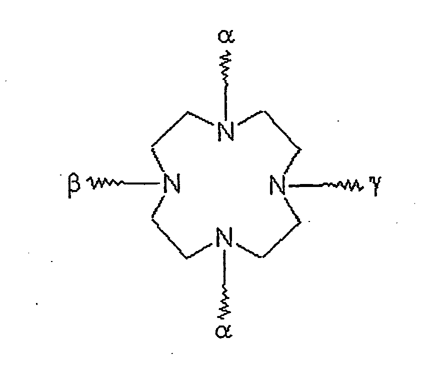
(d) 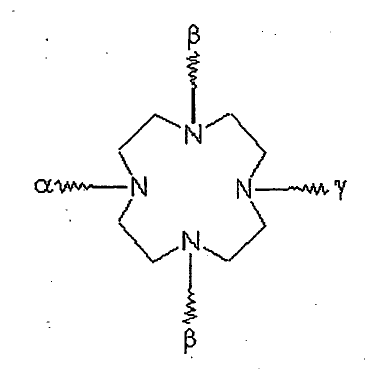
(e) 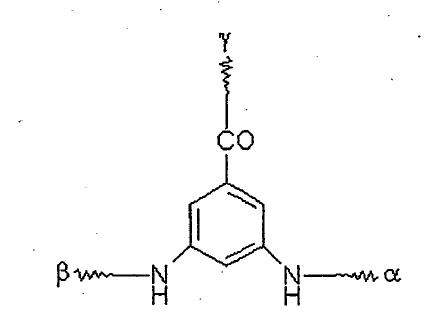
(f) 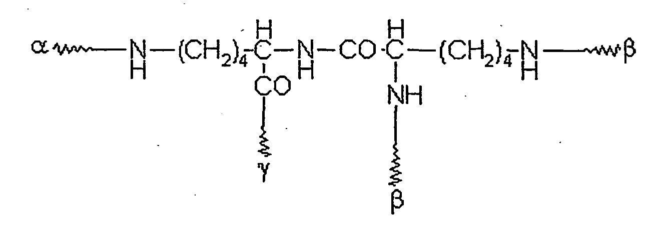
(g) 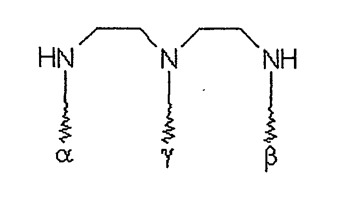
(h) 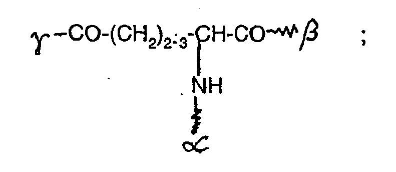
(i)

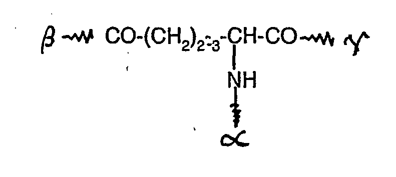

(h) dass G den Rest (c) oder (d) und R einen Komplex ausgewählt aus den allgemeinen Formeln II und V darstellen, darf R nicht identisch mit dem Rest K der allgemeinen Formel I sein, wenn Z für δ-C(O)CH₂O(CH₂)₂-ε steht,
wobei α die Bindungsstelle von G an den Komplex K bedeutet, β die Bindungsstelle von G zum Rest R ist und γ die Bindungsstelle von G zum Rest Z darstellt
- Z: für

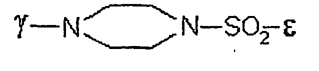

γ-C(O)CH₂O(CH₂)₂-ε,

steht, wobei γ die Bindungsstelle von Z zum Rest G darstellt und ε die Bindungsstelle von Z an den perfluorierten Rest R_{f} bedeutet
- R: einen polaren Rest ausgewählt aus den Komplexen K der allgemeinen Formeln II bis VII darstellt, wobei R¹ hier ein Wasserstoffatom oder ein Metallionenäquivalent der Ordnungszahlen 20-29, 31-33, 37-39, 42-44, 49 oder 57-83 bedeutet,
und die Reste R², R³, R⁴, U und U¹ die oben angegebene Bedeutung aufweisen, wobei für den Fall, dass G den Rest (c) oder (d) und R einen Komplex ausgewählt aus den allgemeinen Formeln II und V darstellen, darf R nicht identisch mit dem Rest K der allgemeinen Formel I sein, wenn Z für δ-C (O) CH₂O(CH₂)₂-ε steht,
oder
den Folsäurerest
oder
eine über -CO- , SO₂- oder eine direkte Bindung an den Rest G gebundene Kohlenstoffkette mit 2-30 C-Atomen bedeutet, geradlinig oder verzweigt, gesättigt oder ungesättigt,
gegebenenfalls unterbrochen durch 1-10 Sauerstoffatome, 1-5 -NHCO-Gruppen, 1-5 -CONH-Gruppen, 1-2 Schwefelatome, 1-5 -NH-Gruppen oder 1-2 Phenylengruppen, die gegebenenfalls mit 1-2 OH-Gruppen, 1-2 NH₂-Gruppen, 1-2 -COOH-Gruppen, oder 1-2 -SO₃H-Gruppen substituiert sein können
oder
gegebenenfalls substituiert mit 1-8 OH-Gruppen, 1-5-COOH-Gruppen, 1-2 SO₃H-Gruppen, 1-5 NH₂-Gruppen, 1-5 C₁-C₄-Alkoxygruppen,
und
- l, m, p: unabhängig voneinander die ganzen Zahlen 1 oder 2 bedeuten.

Ist die erfindungsgemäße Verbindung zur Anwendung in der NMR-Diagnostik bestimmt, so muß das Metallion der signalgebenden Gruppe paramagnetisch sein. Dies sind insbesondere die zwei-und dreiwertigen Ionen der Elemente der Ordnungszahlen 21-29, 42, 44 und 58-70. Geeignete Ionen sind beispielsweise das Chrom(III)-, Eisen(II)-, Kobalt(II)-, Nickel(II)-, Kupfer (II)-, Praseodym(III)-, Neodym(III)-, Samarium(III)- und Ytterbium(III)-ion. Wegen ihres starken magnetischen Moments sind besonders bevorzugt Gadolinium(III)-, Terbium(III)-, Dysprosium(III)-, Holmium(III)-, Erbium(III)-, Eisen(III)- und Mangan(II)-ionen.

Für die Verwendung der erfindungsgemäßen Verbindungen in der Nuklearmedizin (Radiodiagnostik und Radiotherapie) muß das Metallion radioaktiv sein. Geeignet sind zum Beispiel Radioisotope der Elemente mit den Ordnungszahlen 27, 29, 31-33, 37-39, 43, 49, 62, 64, 70, 75 und 77. Bevorzugt sind Technetium, Gallium, Indium, Rhenium und Yttrium.

Ist die erfindungsgemäße Verbindung zur Anwendung in der Röntgen-Diagnostik bestimmt, so leitet sich das Metallion vorzugsweise von einem Element höherer Ordnungszahl ab, um eine ausreichende Absorption der Röntgenstraheln zu erzielen. Es wurde gefunden, daß zu diesem Zweck diagnostische Mittel, die ein physiologisch verträgliches Komplexsalz mit Metallionen von Elementen der Ordnungszahlen 25, 26 und 39 sowie 57-83 enthalten, geeignet sind.

Bevorzugt sind Mangan(II)-, Eisen(II)-, Eisen(III)-, Praseodym(III)-, Neodym(III)-, Samarium(III)-, Gadolinium(III)-, Ytterbium(III)- oder Bismut(III)-ionen, insbesondere Dysprosium(III)-ionen und Yttrium(III)-ionen.

In R¹ gegebenenfalls vorhandene acide Wasserstoffatome, das heißt diejenigen, die nicht durch das Zentralion substituiert worden sind, können gegebenenfalls ganz oder teilweise durch Kationen anorganischer und/oder organischer Basen oder Aminosäuren oder Aminosäureamide ersetzt sein.

Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Besonders bevorzugte Verbindungen der allgemeinen Formel I sind solche mit dem Makrocyclus K der allgemeinen Formeln II, III, VB oder VII.

Der Rest U im Metallkomplex K bedeutet vorzugsweise -CH₂- oder C₆H₄-O-CH₂-ω, wobei ω für die Bindungsstelle an -CO- steht.

Die Alkylgruppen R² und R³ im Makrocyclus der allgemeinen Formel II können geradkettig oder verzweigt sein. Beispielhaft seien Methyl, Ethyl, Propyl, Isopropyl, n-Butyl, 1-Methylpropyl, 2-Methylpropyl, n-Pentyl, 1-Methylbutyl, 2-Methylbutyl, 3-Methylbutyl, 1,2-Dimethylpropyl genannt. Vorzugsweise bedeuten R² und R³ unabhängig voneinander Wasserstoff oder C₁-C₄-Alkyl.

In einer ganz besonders bevorzugten Ausführungsform steht R² für Methyl und R³ für Wasserstoff.

Die Benzylgruppe oder die Phenylgruppe R² oder R³ im Makrocyclus K der allgemeinen Formel II kann auch im Ring substituiert sein.

Der polare Rest R in der allgemeinen Formel I bedeutet in einer bevorzugten Ausführungsform den Komplex K, wobei dieser vorzugsweise neben einem Gd³⁺- oder Mn²⁺-Komplex auch ein Ca²⁺-Komplex sein kann. Besonders bevorzugt sind als polare Reste R die Komplexe K der allgemeinen Formeln II, III, VA oder VII. Ganz besonders bevorzugt weisen diese als R¹ ein Metallionenäquivalent der Ordnungszahlen 20, 25 oder 64 auf.

In einer anderen bevorzugten Ausführungsform hat der polare Rest R die folgenden Bedeutungen:
-C (O) CH₂CH₂SO₃H
-C(O) CH₂OCH₂CH₂OCH₂CH₂OH
-C(O) CH₂OCH₂CH₂OH
-C(O)CH₂OCH₂CH(OH)CH₂OH
-C(O)CH₂NH-C(O)CH₂COOH
-C(O)CH₂CH(OH)CH₂OH
-C(O)CH₂OCH₂COOH
-SO₂CH₂CH₂COOH
-C(O)-C₆H₃-(m-COOH)₂
-C (O) CH₂O (CH₂) ₂-C₆H₃- (m-COOH)₂
-C (O) CH₂O-C₆H₄-m-SO₃H
-C (O)CH₂NHC (O) CH₂NHC (O) CH₂OCH₂COOH
-C (O) CH₂OCH₂CH₂OCH₂COOH
-C (O) CH₂OCH₂CH (OH) CH₂O-CH₂CH₂OH
-C(O) CH₂OCH₂CH (OH) CH₂OCH₂-CH (OH)-CH₂OH
-C(O)CH₂SO₃H
-C(O)CH₂CH₂COOH
-C(O)CH(OH)CH(OH)CH₂OH
-C(O)CH₂O[(CH₂)_{2O}]₁-₉-CH₃
-C(O)CH₂O[(CH₂)₂O]₁₋₉-H
-C(O)CH₂OCH(CH₂OH)₂
-C(O)CH₂OCH (CH₂OCH₂COOH)₂
-C(O)-C₆H₃- (m-OCH₂COOH)₂
-CO-CH₂O-(CH₂)₂O (CH₂)₂O- (CH₂)₂O (CH₂)₂OCH₃
vorzugsweise -C(O)CH₂O[(CH₂)₂O]₄-CH₃.

In einer weiteren bevorzugten Ausführungsform bedeutet der polare Rest R den Folsäurerest.

Von den erfindungsgemäßen Verbindungen der allgemeinen Formel I sind weiterhin solche bevorzugt, in denen R_{f} -CₙF₂ₙ₊₁ bedeutet. n steht vorzugsweise für die Zahlen 4-15. Ganz besonders bevorzugt sind die Reste -C₄F₉, -C₆F₁₃, -C₈F₁₇, -C₁₂F₂₅ und -C₁₄F₂₉ sowie die Reste der in den Beispielen genannten Verbindungen.

Der mindestens dreifach funktionalisierte Rest G in der allgemeinen Formel I, der das "Gerüst" darstellt, bedeutet in einer bevorzugten Ausführungsform der Erfindung den Lysinrest (a) oder (b).

Z bedeutet die in der allgemeinen Formel I angegebenen Linker, wobei der Rest 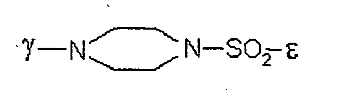 bevorzugt ist.

Die perfluoralkylhaltigen Metallkomplexe mit polaren Resten der allgemeinen Formel I 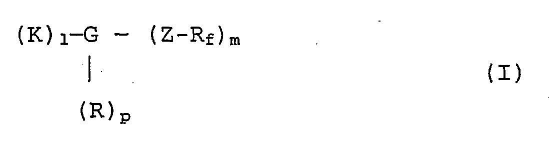 in der K, G, R, Z, R_{f}, l, m und p die oben angegebene Bedeutung haben,
werden hergestellt, indem man in an sich bekannter Weise eine Carbonsäure der allgemeinen Formel IIa 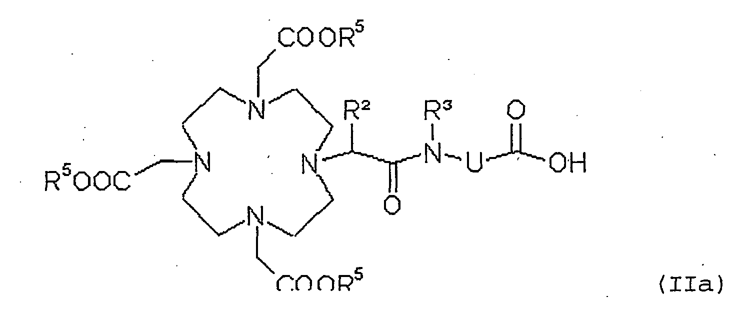 worin R⁵ ein Metallionenäquivalent der Ordnungszahlen 21-29, 31-33, 37-39, 42-44, 49 oder 57-83 oder eine Carboxylschutzgruppe bedeutet, und R², R³ und U die genannte Bedeutung haben
oder eine Carbonsäure der allgemeinen Formel IIIa 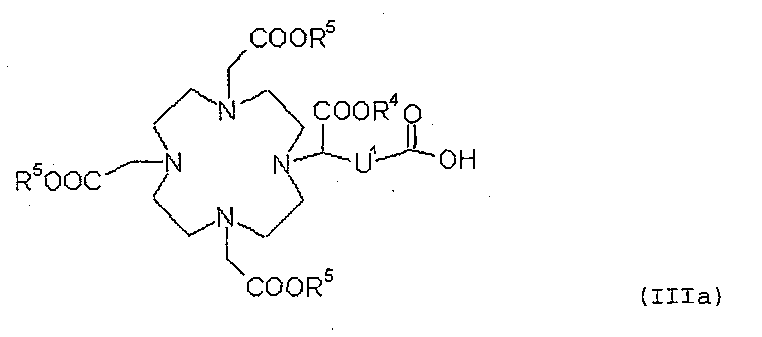 worin R⁴, R⁵ und U¹ die genannte Bedeutung haben
oder eine Carbonsäure der allgemeinen Formel IVa 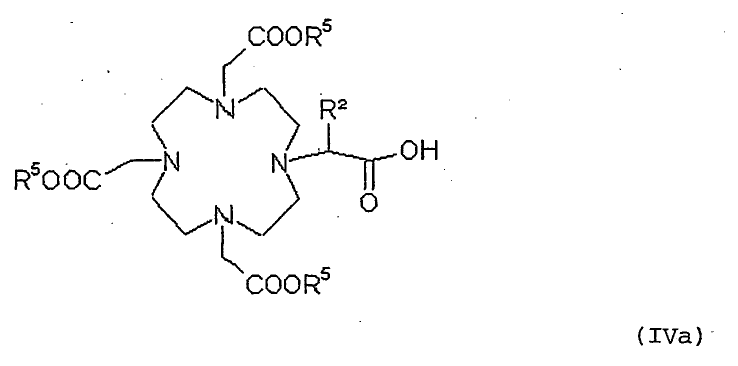 worin R⁵ und R² die genannte Bedeutung haben
oder eine Carbonsäure der allgemeinen Formel Va oder Vb 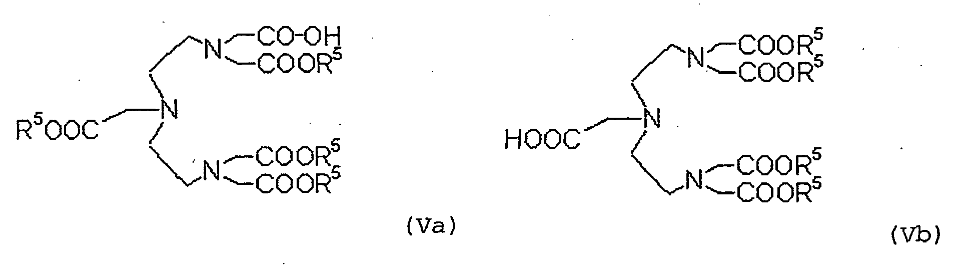 worin R⁵ die genannte Bedeutung hat
oder eine Carbonsäure der allgemeinen Formel VIa 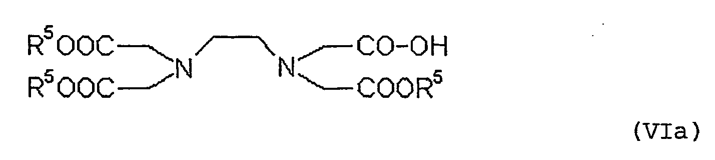 worin R⁵ die genannte Bedeutung hat
oder eine Carbonsäure der allgemeinen Formel VIIa 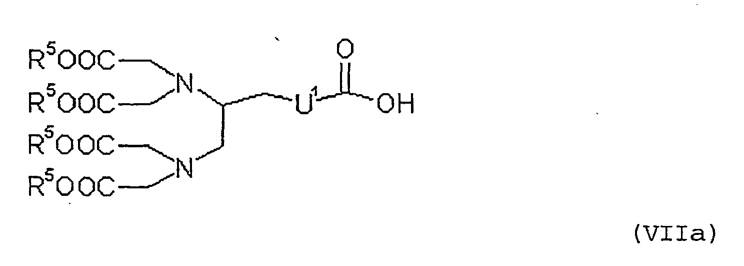 worin R⁵ und U¹ die genannten Bedeutungen haben,
in gegebenenfalls aktivierter Form mit einem Amin der allgemeinen Formel VIII 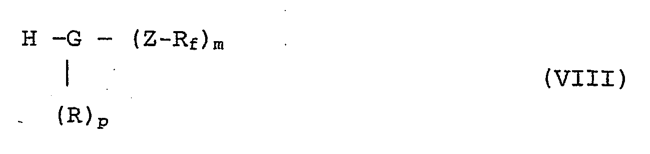 in der G, R, Z, R_{f}, m und p die angegebene Bedeutung haben, in einer Kupplungsreaktion und gegebenenfalls nachfolgender Abspaltung gegebenenfalls vorhandener Schutzgruppen zu einem Metallkomplex der allgemeinen Formel I umsetzt oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

Die eingesetzten Carbonsäuren der allgemeinen Formeln IIa bis VIIa sind entweder bekannte Verbindungen oder werden nach den in den Beispielen beschriebenen Verfahren hergestellt. So ist die Herstellung der Carbonsäuren der allgemeinen Formel IIa aus DE 196 52 386 bekannt. Die Herstellung von Carbonsäuren der allgemeinen Formel IIIa kann in Analogie zu Beispiel 4 der vorliegenden Anmeldung erfolgen. Die Herstellung der Carbonsäuren der allgemeinen Formel IVa ist DE 197 28 954 entnehmbar.

Vorstufe für Verbindungen der allgemeinen Formel VA ist die N³-(2,6-Dioxomorpholinoethyl)-N⁶-(ethoxycarbonylmethyl)-3,6-diazaoctandisäure, die in EP 263 059 beschrieben ist.

Die Verbindungen der allgemeinen Formel VB leiten sich von der isomeren Diethylentriamin-pentaessigsäure ab, die über die am mittleren N-Atom stehende Essigsäure bindet. Diese DTPA ist in den Patenten DE 195 07 819 und DE 195 08 058 beschrieben.

Verbindungen der allgemeinen Formel VI leiten sich vom N-(Carboxymethyl)-N-[2-(2,6-dioxo-4-morpholinyl)-ethyl]-glycin ab, dessen Herstellung in J. Am. Oil. Chem. Soc. (1982), 59 (2), 104-107, beschrieben ist.

Verbindungen der allgemeinen Formel VII leiten sich von der 1-(4-Carboxymethoxybenzyl)-ethylendiamin-tetraessigsäure ab, deren Herstellung im Patent US 4,622,420 beschrieben worden ist.

Die Herstellung der Amine der allgemeinen Formel VIII ist in den Beispielen der vorliegenden Anmeldung detalliert beschrieben und kann analog den in den Beispielen beschriebenen Verfahren vorgenommen werden.

Es hat sich gezeigt, daß die erfindungsgemäßen Metallkomplexe insbesondere für die NMR- und Röntgen-Diagnostik, aber auch und für die Radiodiagnostik und Radiotherapie geeignet sind. Gegenstand der Erfindung ist deshalb auch die Verwendung der erfindungsgemäßen perfluoralkylhaltigen Metallkomplexe mit polaren Resten zur Herstellung von Kontrastmitteln zur Anwendung in der NMR- und Röntgendiagnostik, insbesondere zur Lymphographie, zur Tumordiagnostik und zum Infarkt- und Nekrose-Imaging sowie in der Radiodiagnostik und Radiotherapie. Hervorragend geeignet sind die erfindungsgemäßen Verbindungen zur Anwendung in der interstitiellen und besonders in der intravenösen Lymphographie. Daneben können sie auch zur Darstellung des Vasalraumses (blood-pool-agents) dienen.

Gegenstand der Erfindung sind auch pharmazeutische Mittel, die mindestens eine physiologisch verträgliche erfindungsgemäße Verbindung enthalten, gegebenenfalls mit den in der Galenik üblichen Zusätzen.

Die Verbindungen der vorliegenden Erfindung zeichnen sich durch eine sehr gute systemische Verträglichkeit und eine hohe Lymphknotenanreicherung in drei aufeinanderfolgenden Lymphknotenstationen aus (was besonders für die i.v. Lymphographie wichtig ist). Sie sind damit besonders gut geeignet für die Anwendung in der MRT-Lymphographie.

Die erfindungsgemäßen Verbindungen sind auch in hervorragender Weise zur Erkennung und Lokalisierung von Gefäßkrankheiten geeignet, da sie sich bei der Applikation in den Intravasalraum ausschließlich in diesem verteilen. Die erfindungsgemäßen Verbindungen ermöglichen es, mit Hilfe der Kernspintomographie gut durchblutetes von schlecht durchblutetem Gewebe abzugrenzen und somit eine Ischämie zu diagnostizieren. Auch infarziertes Gewebe läßt sich aufgrund seiner Anämie vom umliegenden gesunden oder ischämischen Gewebe abgrenzen, wenn die erfindungsgemäßen Kontrastmittel angewandt werden. Dies ist von besonderer Bedeutung, wenn es z.B. darum geht, einen Herzinfarkt von einer Ischämie zu unterscheiden.

Gegenüber den bisher als blood-pool-agents eingesetzten makromolekularen Verbindungen, wie beispielsweise Gd-DTPA-Polylysin, zeigen die erfindungsgemäßen Verbindungen ebenfalls eine höhere T¹-Relaxivity und zeichnen sich somit durch eine Steigerung der Signalintensität bei der NMR-Bildgebung aus. Da sie daneben eine verlängerte Retention im Blutraum haben, können sie auch in relativ kleinen Dosierungen (von z.B. ≤ 50 µmol Gd/l Körpergewicht) appliziert werden: Vor allem aber werden die erfindungsgemäßen Verbindungen rasch und weitgehend vollständig aus dem Körper eliminiert.

Ferner zeigte sich, daß sich die erfindungsgemäßen Verbindungen in Gebieten mit erhöhter Gefäßpermeabilität, wie z.B. in Tumoren, anreichern; sie erlauben Aussagen über die Perfusion von Geweben, geben die Möglichkeit, das Blutvolumen in Geweben zu bestimmen, die Realaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA (Magnevist^{®}) zu erhalten. -Aus diesen Gesichtspunkten ergeben sich auch die Einsatzgebiete bei den modernen bildgebenden Verfahren Kernspintomographie und Computertomographie: spezifische Diagnose von malignen Tumoren, frühe Therapiekontrolle bei zytostatischer, antiphlogistischer oder vaso-dilatativer Therapie, frühe Erkennung von minderperfundierten Gebieten (z.B. im Myokard), Angiographie bei Gefäßerkrankungen, und Erkennung und Diagnose von sterilen oder infektiösen Entzündungen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern (wie zum Beispiel Diethylentriaminpentaessigsäure) oder schwachen Komplexen oder die zu den erfindungsgemäßen Metallkomplexen korrespondierenden Ca-Komplexe oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methyl-cellulose, Lactose, Mannit] und/oder Tensid(en) [zum Beispiel Lecithine, Tween^{®}, Myrj^{®}] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Humanserumalbumin (HSA) verabreicht werden.

Die erfindungsgemäßen Mittel werden üblicherweise parenteral, vorzugsweise i.v., appliziert. Sie können auch intravasal oder interstitiell/intrakutan appliziert werden, je nachdem, ob Körpergefäße oder -gewebe untersucht werden sollen.

Die erfindungsgemäßen pharmazeutischen Mittel enthalten vorzugsweise 0,1µMol 2 Mol/l des Komplexes und werden in der Regel in Mengen von 0,0001 - 5 mMol/kg dosiert.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität invitro auf, sondern auch eine überraschend hohe Stabilität in-vivo, so daß eine Freigabe oder ein Austausch der in den Komplexen gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001-5 mMol/kg, vorzugsweise 0,005 - 0,5 mMol/kg, dosiert.

Ferner können die erfindungsgemäßen Komplexverbindungen vorteilhaft als Suszeptibilitäts-Reagenzien und als shift-Reagenzien für die in-vivo-NMR-Spektroskopie verwendet werden:

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details einer solchen Anwendung und Dosierung werden z.B. in "Radiotracers for Medical Applications", CRC-Press, Boca Raton, Florida, beschrieben.
Die erfindungsgemäßen Verbindungen und Mittel können auch in der Positronen-Emissions-Tomographie, die positronenemittierende Isotope wie z.B. ⁴³Sc, ⁴⁴Sc, ⁵²Fe, ⁵⁵Co_{,}⁶⁸Ga und ⁸⁶Y. verwendet (Heiss, W.D.; Phelps, M.E.; Positron Emission Tomography of Brain, Springer Verlag Berlin, Heidelberg, New York 1983), eingesetzt werden.

Die erfindungsgemäßen Verbindungen sind überraschenderweise auch zur Differenzierung von malignen und benignen Tumoren in Bereichen ohne Blut-Hirn-Schranke geeignet.

Sie zeichnen sich auch dadurch aus, daß sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strahlentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronen-emittierendes Ion ist ¹⁵⁸Gd, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen Mittel werden üblicherweise parenteral, vorzugsweise i.v., appliziert. Sie können auch - wie bereits erörtert - intravasal oder interstitiell/intrakutan appliziert werden, je nachdem, ob Körpergefäße oder -gewebe untersucht werden sollen.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zum Beispiel Meglumin-Diatrizoat in Mengen von 0,1 - 5 mMol/kg, vorzugsweise 0,25 - 1 mMol/kg, dosiert.

Insbesondere werden mit den erfindungsgemäßen Verbindungen höhere Blutkonzentrationen erreicht als mit extrazellulären Kontrastmitteln. Sie verteilen sich nach i.v. Applikation nur im Intravasalraum und haben damit einen entscheidenden Vorteil gegenüber den extrazellulären Kontrastmitteln.

### Ausführungsbeispiele

### Beispiel 1a

### 2-N-Trifluoracetyl-6-N-benzyloxycarbonyl-lysin

100 g (356,7 mmol) 6-N-Benzyloxycarbonyl-lysin werden in einer Mischung aus 1000 ml Trifluoressigsäureethylester / 500 ml Ethanol gelöst und 24 Stunden bei Raumtemperatur gerührt. Man dampft zur Trockene ein und kristallisiert den Rückstand aus Diisopropylether. Ausbeute: 128,9 g (96 % der Theorie) eines farblosen kristallienen Pulvers.

| Elementaranalyse: | | | | |
|---|---|---|---|---|
| ber. | C 51,07 | H 5,09 | F 15,14 | N 7,44 |
| gef. | C 51,25 | H 5,18 | F 15,03 | N 7,58 |

### Beispiel 1b

### 2-N-Trifluoracetyl-6-N-benzyloxycarbonyl-lysin - [1-(4-perfluoroctylsulfonyl) piperazin]-amid

Zu 125 g (332 mmol) der Titelverbindung aus Beispiel 1a und 188,7g (332 mmol) 1-Perfluoroctylsulfonyl-piperazin, (hergestellt nach DE 19603033) in 800 ml Tetrahydrofuran, gibt man bei 0° C 164,2 g (0,664 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel : Dichlormethan / Methanol =20:1)
Ausbeute: 286 g (93 % der Theorie) eines farblosen Feststoffs.

| Elementaranalyse: | | | | | |
|---|---|---|---|---|---|
| ber. | C 36,30 | H 2,83 | F 41,01 | N 6,05 | S 3,46 |
| gef. | C 36,18 | H 2,94 | F 40,87 | N 5,98 | S 3,40 |

### Beispiel 1c

### 6-N- Benzyloxycarbonyl- lysin - [1- (4-perfluoroctylsulfonyl)- piperazin] - amid

In eine Lösung aus 280 g (302,2 mmol) der Titelverbindung aus Beispiel 1b in 2000 ml Ethanol, leitet man bei 0° C für eine Stunde Ammoniak-Gas ein. Man rührt anschließend 4 Stunden bei 0°C. Es wird zur Trockene eingedampft und der Rückstand aus Wasser ausgerührt. Man filtriert den Feststoff ab und trocknet im Vakuum (50° C).
Ausbeute : 243,5 g (97 % der Theorie) eines amorphen Feststoffs.

| Elementaranalyse: | | | | | |
|---|---|---|---|---|---|
| ber. | C 37,60 | H 3,28 | F 38,89 | N 6,75 | S 3,86 |
| gef. | C 37,15 | H 3,33 | F 38,78 | N 6,68 | S 3,81 |

### Beispiel 1d

### 6-N- Benzyloxycarbonyl- 2-N- (3,6,9,12,15- pentaoxahexadecanoyl) - lysin [1- (4-perfluoroctylsulfonyl)-piperazin]-amid

Zu 50 g (60,20 mmol) der Titelverbindung aus Beispiel 1c und 7,10 g (70 mmol) Triethylamin, gelöst in 350 ml Dichlormethan, tropft man bei 0° C eine Lösung aus 19,93 g ( 70 mmol) 3,6,9,12,15 Pentaoxahexadecansäurechlorid in 50 ml Dichlormethan und rührt 3 Stunden bei 0° C. Man gibt 200 ml 5% ige aqu Salzsäure zu und rührt 5 Minuten bei Raumtemperatur. Die organische Phase wird abgetrennt, über Magnesiumsulfat getrocknet und im Vakuum zur Trockene eingedampft. Der Rückstand wird an Kieselgel chromatographiert (Laufmittel: Dichlormethan / Aceton = 15:1 1
Ausbeute: 53,7 g (93% der Theorie) eines farblosen, zähen Ö1s.

| Elementaranalyse: | | | | | |
|---|---|---|---|---|---|
| ber. | C 33,83 | H 4,94 | F 3,34 | N 5,84 | S 33,69 |
| gef. | C 33,75 | H 5,05 | F 3,29 | N 5,78 | S 33,75 |

### Beispiel 1e

### 2-N-(3,6,9,12,15- pentaoxahexadecanoyl)-lysin [1-(4- perfluoroctylsulfonyl)-piperazin]-amid

50 g (52,15 mmol) der Titelverbindung aus Beispiel 1d werden in 500 ml Ethanol gelöst und 6 g Palladium-Katalysator (10 % Pd/C) zugegeben. Man hydriert bei Raumtemperatur. Man filtriert vom Katalysator ab und dampft das Filtrat im Vakuum zur Trockene ein. Ausbeute: 43,0 g (quantitativ) eines farblosen Feststoffs.

| Elementaranalyse: | | | | | |
|---|---|---|---|---|---|
| ber. | C 27,68 | H 5,01 | F 39,17 | N 6,79 | S 3,89 |
| gef. | C 27,60 | H 5,13 | F 39,09 | N 6,68 | S 3,81 |

### Beispiel 1f

### 6-N-[1,4,7-Tris (carboxylatomethyl) -1,4,7,10- tetraazacyclododecan-10 -(pentanoyl- 3-aza-4- oxo- 5- methyl- 5-yl)] - 2-N- (3,6,9,12,15- pentaoxahexadecanoyl)-lysin [1-(4-perfluoroctylsulfonyl)-piperazin]-amid, Gd-Komplex

20 g (24,25 mmol) der Titelverbindung aus Beispiel 1e, 2,79 g (24,25 mmol) N-Hydroxysuccinimid, 2,12 g (50 mmol) Lithiumchlorid und 15,27g (24,25 mmol) 1,4,7-Tris (carboxylatomethyl) -10 -[(3-aza- 4- oxo- 5- methyl- 5-yl)]-pentansäure]-1,4,7,10- tetraazacyclododecan, Gd-Komplex werden unter leichter Erwärmung in 200 ml Dimethylsulfoxid gelöst. Bei 10°C gibt man 8,25 g (40 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt ( Kieselgel RP-18, Laufmittel: Gradient aus Wasser / Ethanol / Acetonitril).Ausbeute: 28,21 g (81 % der Theorie) eines farblosen Feststoffs.
Wassergehalt: 11,0 %

| Elementaranalyse (auf wasserfreie Substanz berechnet) | | | | | | |
|---|---|---|---|---|---|---|
| ber. | C 31,78 | H 4,84 | F 22,49 | N 8,78 | S 2,23 | Gd 10,95 |
| gef. | C 31,74 | H 4,98 | F 22,39 | N 8,69 | S 2,15 | Gd 10,87 |

### Beispiel 2a

### 6-N-[3,9 - Bis(t butyloxycarbonylmethyl)-3,6,9- triazaundecan -1,11- dicarbonsäure bis (t butylester) - 6- carbonylmethyl] -2-N- [3,6,9,12,15- pentaoxahexadecanoyl)- lysin- [1-(4-perfluoroctylsulfonyl)- piperazin] - amid

Zu einer Lösung aus 20 g (24,08 mmol) der Titelverbindung aus Beispiel 1e , 14,88, g ( 24,08 mmol) 3,9- bis (t butyloxycarbonylmethyl - 3,6,9 - triazaundecan -1,11- dicarbonsäure - bis (t butylester ) und 2,77 g (24,08 mmol) N - Hydroxysuccinimid, gelöst in 150 ml Dimethylformamid, gibt man bei 0° C 8,25 g (40 mmol) N,N- Dicyclohexylcabodiimid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chomatographiert am Kieselgel (Laufmittel: = Dichlormethan / Ethanol = 20:1).
Ausbeute: 31,61 g (91% der Theorie) eines zähen öls.

| Elementaranalyse : | | | | | |
|---|---|---|---|---|---|
| ber. | C 40,80 | H 6,71 | F 22,39 | N 6,80 | S 2,22 |
| gef. | C 40,72 | H 6,82 | F 22,30 | N 6,75 | S 2,14 |

### Beispiel 2b

### 6-N-[6- Carbonylmethyl - 3,9- bis (carboxylatomethyl)-3,6,9-triazaundecandicarbonsäure-1-carboxy-11-carboxylato-]-2-N-(3,6,9,12,15-pentaoxahexadecanoyl)-lysin-[1-(4-perfluoroctylsulfonyl)-piperazin]-amid, Gd-Komplex, Natriumsalz

30 g (20,8 mmol) der Titelverbindung aus Beispiel 2a werden in 300 ml Trifluoressigsäure gelöst und 5 Stunden bei Raumtemperatur gerührt. Man dampft zur Trockene ein, nimmt den Rückstand in 300 ml Wasser auf und stellt mit 10% iger aqu. NaOH auf einen pH Wert von 2,5. Anschließend gibt man 3,77 g (10,4 mmol) Gadoliniumoxid zu und rührt 3 Stunden bei 60° C. Man läßt auf Raumtemperatur kommen und stellt mit Natronlauge auf einen pH Wert von 7,4. Es wird zur Trockene eingedampft und der Rückstand an Kieselgel RP-18 aufgereinigt. (Laufmittel: Gradient aus Wasser / Acetonitril).
Ausbeute: 19,18 g (67% der Theorie (eines farblosen, amorphen Feststoffs.
Wassergehalt 9,8 %

| Elementaranalyse: (berechnet auf wasserfreie Substanz) | | | | | | | |
|---|---|---|---|---|---|---|---|
| ber. | C 28,80 | H 4,25 | F 23,47 | N 7,12 | S 2,33 | Gd 11,48 | Na 1,67 |
| gef. | C 28,67 | H 4,34 | F 23,38 | N 7,03 | S 2,27 | Gd 11,37 | Na 1,74 |

### Beispiel 3a

### Lysin-[1-(4-perfluoroctylsulfonyl-piperazin]-amid

20 g (24,08 mmol) der Titelverbindung aus Beispiel 1c werden in 300 ml Ethanol gelöst und 4 g Palladium- Katalysator (10% Pd / C) zugegeben. Man hydriert bei Raumtemperatur. Man filtriert vom Katalysator ab und dampft das Filtrat im Vakuum zur Trockene ein. Ausbeute: 16,77 g (quantitativ) eines farblosen Feststoffs.

| Elementaranalyse: | | | | | |
|---|---|---|---|---|---|
| ber. | C 31,04 | H 3,04 | F 46,38 | N 8,04 | S 4,60 |
| gef. | C 30,97 | H 3,15 | F 46,31 | N 7,98 | S 4,51 |

### Beispiel3b

### 2,6-N,N'-Bis [1,4,7-tris (carboxylatomethyl)-1,4,7,10-tetraazacyclododecan-10-(pentanoyl-3-aza-4-oxo-5-methyl-5-yl)]-lysin-[1-(4-perfluoroctyllsulfonyl-piperazin]-amid, Gd-Komplex

10 g (14,36 mmol) der Titelverbindung aus Beispiel 3a, 3,34 g (29 mmol) N-Hydroxysuccinimid, 2,54 g (mmol) Lithiumchlorid und 18,26 g (29 mmol) 1,4,7-Tris (carboxylatomethyl)-10-(3-aza-4-oxo-5-methyl-5yl) 1,4,7,10-tetraazacyclododecan-Gd-Komplex werden unter leichter Erwärmung in 200 ml Dimethylsulfoxid gelöst. Bei 10° C gibt man 12,38 g (60 mmol) N,N-Dicyclohexlcarbodümid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (Kiesel- gel RP-18, Laufmittel: Gradient aus Wasser / Ethanol / Acetonitril). Ausbeute : 19,02 g (69 % der Theorie) eines farblosen Feststoffs
Wassergehalt: 11,3 %

| Elementaranalyse: ( berechnet auf wasserfreie Substanz) | | | | | | |
|---|---|---|---|---|---|---|
| ber. | C 35,03 | H 4,04 | F 16,82 | N 10,21 | S 1,67 | Gd 16,38 |
| gef. | C 34,96 | H 4,13 | F 16,74 | N 10,16 | S 1,61 | Gd 16,33 |

### Beispiel 4a

### 2-[4-(3-Oxapropionsäureethylester)]-phenylessigsäuremethylester

Zu 200 g (1,204 mol) 4-Hydroxyphenylessigsäuremethylester, 212 g (2 mol) Natriumcarbonat in 2000 ml Aceton gibt man 233,8 g (1,4 mol) 2-Bromessigsäureethylester und kocht 5 Stunden unter Rückfluß. Man filtriert den Feststoff ab und dampft im Vakuum zur Trockene ein. Der Rückstand wird an Kieselgel chromatographiert (Laufmittel: -n-Hexan / Essigsäureethylester = 15:1).
Ausbeute : 288,5 g (95 % der Theorie) eines farblosen Öls.

| Elementaranalyse : | | |
|---|---|---|
| ber. | C 61,90 | H 6,39 |
| gef. | C 61,75 | H 6,51 |

### Beispiel 4b

### 2-[4-(3-Oxapropionsäureethylester)]-phenyl-2-bromessigsäuremethylester

Zu 285 g (1,13 mol) der Titelverbindung aus Beispiel 4a, gelöst, in 2000 ml Tetrachlorkohlenstoff, gibt man 201 g (1,13 mol) N-Bromsuccinimid und 100 mg Dibenzylperoxid und kocht 8 Stunden unter Rückfluß. Man kühlt im Eisbad, filtriert das ausgefallene Succinimid ab und dampft das Filtrat im Vakuum zur Trockene ein. Der Rückstand wird an Kieselgel gereinigt (Laufmittel:-n-Hexan / Aceton =15:1).
Ausbeute : 359,2 g (96% derTheorie) eines farblosen, zähen Öls.

| Elementaranalyse : | | | |
|---|---|---|---|
| ber. | C 47,28 | H 4,57 | Br 24,16 |
| gef. | C 47,19 | H 4,71 | Br 24,05 |

### Beispiel 4c

### 2-[4-(3-Oxapropionsäureetilylester)]-phenyl-2-[1-(1,4,7,10-tetraazacydododecan 7-yl]-essigsäuremethylester

Zu 603 g (3,5 mol) 1,4,7,10-Tetraazacyclododecan in 6000 ml Chloroform gibt man 350 g (1,057 mol) der Titelverbindung aus Beispiel 4b und rührt über Nacht bei Raumtemperatur. Man extrahiert 3 mal mit 3000 ml Wasser, trocknet die organische Phase über Magnesiumsulfat und dampft im Vakuum zur Trockene ein. Der Rückstand wird ohne weitere Aufreinigung in die nächste Reaktion (3d) eingesetzt.
Ausbeute: 448 g (quantitativ) eines zähen Öls.

| Elementaranalyse : | | | |
|---|---|---|---|
| ber. | C 59,70 | H 8,11 | N 13,26 |
| gef. | C 59,58 | H 8,20 | N 13,18 |

### Beispiel 4d

### 2-[4-(3-Oxapropionsäure)]-phenyl-2-[1,4,7-tris(carboxymethyl) -1,4,7,10-tetraazacyclododecan-10-yl]-essigsäure

445 g (. 1,053 mol) der Titelverbindung aus Beispiel 4c und 496 g (5,27 mol) Chloressigsäure werden in in 4000 ml Wasser gelöst. Man stellt mit 30% iger aqu.Natronlauge auf einen pH Wert von 10. Man erhitzt auf 70° C und hält den pH-Wert durch Zugabe von 30% iger aqu.Natronlauge auf pH 10. Man rührt 8 Stunden bei 70° C. Man stellt anschließend auf einen pH Wert von 13 und kocht 30 Minuten unter Rückfluß. Die Lösung wird im Eisbad gekühlt und durch Zugabe von konz.Salzsäure auf einen pH Wert von 1 gestellt. Man dampft im Vakuum zur Trockene ein. Der Rückstand wird in 4000 ml Methanol aufgenommen und eine Stunde bei Raumtemperatur ausgerührt. Man filtriert vom ausgefallenem Kochsalz ab, dampft das Filtrat zur Trockene ein und reinigt den Rückstand an Kieselgel RP-18 (Laufmittel: Gradient aus Wasser / Ethanol /Acetonitril).
Ausbeute: 403 g (69 % der Theorie) eines farblosen Feststoffs.
Wassergehalt: 10,2 %

| Elementaranalyse: (berechnet auf wasserfreie Substanz) | | | |
|---|---|---|---|
| ber. | C 51,98 | H 6,18 | N 10,10 |
| gef. | C 51,80 | H 6,31 | N 10,01 |

### Beispiel 4e

### 2-[4-(3-Oxapropionsäure)]-phenyl-2-[1,4,7-tris(carboxymethyl) -1,4,7,10-tetraaza-cyclododecan-10-yl]-essigsäure, Gd-Komplex

Zu 400 g (721,3 mmol) der Titelverbindung aus Beispiel 4d in 2000 ml Wasser gibt man 130,73 g (360,65 mmol) Gadoliniumoxid und rührt 5 Stunden bei 80° C. Die Lösung wird filtriert und das Filtrat gefriergetrocknet.
Ausbeute: 511 g (quantitativ) eines amorphen Feststoffs.
Wassergehalt: 11,0 %

| Elementaranalyse: (berechnet auf wasserfreie Substanz) | | | | |
|---|---|---|---|---|
| ber. | C 40,67 | H 4,41 | Gd 22,19 | N 7,98 |
| gef. | C 40,51 | H 4,52 | Gd 22,05 | N 8,03 |

### Beispiel 4f

### 2,6-N,N'-Bis {2-[4-(3-oxapropionyl)-phenyl]-2-[1,4,7-tris(carboxylatomethyl)-1,4,7,10-tetraazacyclododecan-10-yl]-essigsäure]-lysin-[4-perfluor-octylsulfonyl)-piperazin]-amid, Digadolinium-Komplex, Dinatriumsalz

10 g (14,36 mmol) der Titelverbindung aus Beispiel 3a 3,45 g (30 mmol) N-Hydroxysuccinimid, 2,54 g (60 mmol) Lithiumchlorid und 21,26 g (30 mmol) der Titelverbindung aus Beispiel 4e werden unter leichter Erwärmung in 250 ml Dimethylsulfoxid gelöst. Bei 10° C gibt man 16,51 g (80 mmol) N,N-Dicyclohexylcarbodiimid zu und rührt anschließend über Nacht bei Raumtemperatur. Man gießt die Lösung in 2000 ml Aceton und rührt 10 Minuten. Der ausgefallene Feststoff wird abfiltriert und anschließend durch Chromatographie gereinigt (Kieselgel RP-18, Laufmittel: Gradient aus Wasser / Ethanol / Acetonitril) Man löst in wenig Wasser, stellt mit Natronlauge auf einen pH Wert von 7,4 und gefriergetrocknet.
Ausbeute: 21,02 g (69% der Theorie) eines farblosen Feststoffs.
Wassergehalt: 11,2%

| Elementaranalyse: (berechnet auf wasserfreie Substanz | | | | | | | |
|---|---|---|---|---|---|---|---|
| ber. | C 37,36 | H 3,66 | F 15,22 | Gd 14,82 | N 7,92 | Na 2,17 | S 1,51 |
| gef. | C 37,28 | H 3,74 | F 15,14 | Gd 14,75 | N 8,03 | Na 2,23 | S 1,46 |

### Beispiel 5a

### 2,6-N,N' - Bis [6-carbonylmethyl-3,9-bis (t butyloxycarbonylmethyl) 3,6,9-triazaundecan-1,11-dicarbonsäure-bis (t butylester)]-lysin-[1-(4-perfluoroctylsulfonyl)-piperazin]-amid

Zu einer Lösung aus 10g (14,36 mmol) der Titelverbindung aus Beispiel 3a, 18,53 g (30 mmol) 3,9-Bis(t butyloxycarbonylmethyl) -6-carboxymethyl-3,6,9-tnazaundecan - 1,11-dicarbonsäure-bis(t butylester) und 3,45 g (30 mol) N-Hydroxysuccinimid gelöst in 150ml Dimethylformaid gibt man bei 0°C 10,32 g (50 mmol) N,N Dicyclohexylcarbodiimid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chromatographiert an Kieselgel (Laufmittel: Dichlormethan / Ethanol = 20:1)
Ausbeute: 19,60 g (72 % der Theorie) eines zähen Öls.

| Elementaranalyse | | | | | |
|---|---|---|---|---|---|
| ber. | C 49,41 | H 6,75 | F17,03 | N 7,39 | S 1,69 |
| gef. | C 49,35 | H 6,82 | F16,92 | N 7,32 | S 1,62 |

### Beispiel 5b

### 2,6-N,N-Bis [6-carbonylmethyl-3,9-bis (carboxylatomethyl)-3,6,9-triazaundecandicarbonsäure-1-carboxy-11-carboxylato-lysin-[1-(4-perfluoroctylsulfonyl)-piperazin]-amid,Gd-Komplex, Natriumsalz]

15 g (7,91mol) der Titelverbindung aus Beispiel 5a werden in 50 ml Chloroform gelöst und 200 ml Trifluoressigsäure zugegeben. Man rührt 10 Minuten bei Raumtemperatur. Es wird im Vakuum zur Trockene eingedampft und der Rückstand in 150 ml Wasser gelöst. Man gibt 2,87g (7,91 mmol) Gadoliniumoxid zu und rührt 5 Stunden bei 80° C. Man läßt auf Raumtemperatur abkühlen und stellt mit 2N Natronlauge auf pH 7,4. Die Lösung wird im Vakuum zur Trockene eingedampft und an RP-18 gereinigt ( Laufmittel: Gradient aus Wasser / Ethanol / Acetonitril)
Ausbeute: 8,11 g (57% der Theorie) eines farblosen amorphen Feststoffs.
Wassergehalt: 9,6%

| Elementaranalyse: (berechnet auf wasserfreie Substanz) | | | | | | |
|---|---|---|---|---|---|---|
| ber. | C 30,70 | H 3,08 | Gd 17,48 | N 7,78 | Na 2,56 | S 1,78 |
| gef. | C 30,58 | H 3,19 | Gd 17,42 | N 7,71 | Na 2,68 | S 1,72 |

### Beispiel 6a

### 6-N-Benzyloxycarbonyl-2-N-[6-carboxylmethyl-3,9-bis(t butyloxycarbonylmethyl)-3,6,9-triazaundecan-1, 11-dicarbonsäure-bis(t butylester)]-lysin-[l (4- perfluoroctylsulfonyl)-piperazin]-amid

Zu einer Lösung aus 20 g (24,08 mmol) der Titelverbindung aus Beispiel 1c, 14,88 g (24,08 mmol) 3,9-Bis(t butyloxycarbonylmethyl) -6-carboxymethyl-3,6,9-triazaundecan-1,11-dicarbonsäure-bis (t butylester) und 2,88 g (25mol) N-Hydroxysuccinimid gelöst in 100 ml Dimethylformamid gibt man bei 0° C 8,25 g 40 mol)N,N-Dicyclohexylcarbodiimid zu. Man rührt 3 Stunden bei 0° C, anschließend über Nacht bei Raumtemperatur. Man filtriert vom ausgefallenem Harnstoff ab, dampft das Filtrat im Vakuum zur Trockene ein und chromatographiert am Kieselgel (Laufmittel: Dichlor-Methan / Ethanol = 20:1). Ausbeute: 27,21 g (79 % der Theorie) eines zähen Öls

| Elementaranalyse : | | | | | |
|---|---|---|---|---|---|
| ber. | C47,03 | H 5,64 | F 22,58 | N 6,85 | S 2,24 |
| gef. | C46,94 | H 5,58 | F 22,65 | N 6,84 | S 2,31 |

### Beispiel 6b

### 2-N-[Carbonylmethyl-3,9-bis (t butyloxycarbonylmethyl)-3,6,9-triazaundecan-1,11-dicarbonsäure-bis(t butylester)]-lysin-[1-(4-perlfluoroctylsulfonyl)-piperazin]-amid

25 g (17,48 mmol) der Titelverbindung aus Beispiel 6a werden in 350 ml Ethanol gelöst und 5 g Palladium -Katalysator (10% Pd / C) zugegeben. Man hydriert bei Raumtemperatur. Man filtriert vom Katalysator ab und dampft das Filtrat im Vakuum zur Trockene ein.
Ausbeute: 22,66 g (quantitativ) eines farblosen Feststoffs.

| Elementaranalyse : | | | | | |
|---|---|---|---|---|---|
| ber. | C 44,48 | H 5,75 | F 24,92 | N 7,56 | S 2,47 |
| gef. | C 44,59 | H 5,81 | F 25,03 | N 7,46 | S 2,52 |

### Beispiel 6c

### 6-N-[1,4,7-Tris(carboxyl atomethyt)-1,4,7,10-tetraazacyclododecan-10-(pentanoyl-3-aza-4-oxo-5-methyl-5yl)]-2-N-[6-carbonylmethyl-3,9-bis (t butyloxycarbonylmethyl) 3,6,9-triazaundecan-1, 11-dicarbonsäure bis (t butylester)]-lysin-[1-(4-perfluoroctylsulfonyl) -piperazin-]-amid , Gd- Komplex

20 g (15,43 mmol) der Titelverbindung aus Beispiel 6b, 1,78 g (15,43 mmol) N-Hydroxysuccininmid, 1,48 g (35 mmol) Lithiumchlorid und 9,72 g (15,43 mmol) 1,4,7-Tris (carboxylatomethyl)-10-(3-aza-4oxo-5methyl-5yl)-pentansäure-1,4,7,10-tetraazacyclododecan, Gd-Komplex werden unter leichter Erwärmung in 150 ml Dimethylsulfoxid gelöst. Bei 10° C gibt man 5,16 g (25 mmol) N,N-Dicyclohexylcarbodümid zu und rührt anschließend über Nacht bei Raumtemperatur . Man gießt die Lösung in 2500 ml Aceton und rührt 10 Minuten. Der ausgefallene Feststoff wir abfiltriert und anschließend durch Chromatographie gereinigt ( Kieselgel RP-18, Laufmittel: Gradient aus Wasser / Ethanol / Acetonitril).
Ausbeute: 22,94 g (78 % der Theorie) eines farblosen Feststoffs.
Wassergehalt: 7,9 %

| Elementaranalyse: (berechnet auf wasserfreie Substanz) | | | | | | |
|---|---|---|---|---|---|---|
| ber. | C 42,22 | H 5,29 | F 16,95 | Gd 8,25 | N 8,82 | S 1,68 |
| gef. | C 42,15 | H 5,41 | F 16,87 | Gd 8,13 | N 8,70 | S 1,60 |

### Beispiel 6d

### 6-N-[1,4,7-Tris (carboxylatomethyl)-1,4,7,10-tetraazacyclododecan-10-(3-aza-4-oxo-5-methyl-5-yl-pentanoyl)]-2-N-[6-carbonylmethyl-3,9- bis(carboxylatomethyl) 3,6,9-triazaundecan dicarbonsäure -carboxy-11-carboxylato-z]-lysin-[1-(4-perfluoroctylsulfonyl)-piperazin]-amid, Digadoliniumkomplex, Natriumsalz

20 g (10,49 mmol) der Titelverbindung aus Beispiel 6c werden in 200 ml Trifluoressigsäure gelöst. Man rührt 60 Minuten bei Raumtemperatur. Es wird im Vakuum zur Trockene eingedampft und der Rückstand in 150 ml Wasser gelöst. Man gibt 1,90 g (5,25 mmol) Gadoliniumoxid zu und rührt 5 Stunden bei 80° C. Man läßt auf Raumtemperatur abkühlen und stellt mit Natronlauge auf einen pH Wert von 7,4. Die Lösung wird im Vakuum zur Trockene eingedampft und an Kieselgel RP-18 gereinigt.(Laufmittel: Gradient aus Wasser /Ethanol /Acetonitril)
Ausbeute: 11,89 g (61 % der Theorie) eines farblosen amorphen Feststoffs.
Wassergehalt: 10,2%

| Elementaranalyse: (berechnet auf wasserfreie Substanz) | | | | | | | |
|---|---|---|---|---|---|---|---|
| ber. | C 32,97 | H 3,47 | F 17,39 | Gd 16,93 | N 9,05 | Na 1,24 . | S 1,73 |
| gef. | C 32,90 | H 3,53 | F 17,31 | Gd 16,87 | N 8,92 | Na 1,33 | S 1,67 |

### Beispiel 7a

### 5,6-Bis (benzyloxy)-3oxa-hexansäure-t butylester

100 g (376,2 mmol) 1,2-Di-O-benzyl-glycerin [hergestellt nach Chem. Phys. Lipids (1987), 43(2), 113-27] und 5 g Tetrabutylammoniumhydrogensulfat werden in einer Mischung aus 400 ml Toluol und 200 ml 50 % iger aqu.Natronlauge gelöst. Bei 0° C tropft man über 30 Minuten 78g (400 mmol) 2-Bromessigsäure-t butylester zu und rührt anschließend 3 Stunden bei 0° C. Die organische Phase wird abgetrennt, über Magnesiumsulfat getrocknet und im Vakuum zur Trockene eingedampft. Der Rückstand wird an Kieselgel chromatographiert. (Laufmittel: N-Hexan / Aceton=20:1).
Ausbeute: 133,4 g (94 % der Theorie) eines farblosen Öls.

| Elementaranalyse: | | |
|---|---|---|
| ber. | C 71,48 | H 7,82 |
| gef. | C 71,61 | H 7,92 |

### Beispiel 7b

### 5,6-Bis (benzyloxy)-3-oxa-hexansäure

130 g (336,4 mmol) der Titelverbindung aus Beispiel 7a werden in 200 ml Dichlormethan gelöst und bei 0° C 100 ml Trifluoressigsäure zugesetzt. Man rührt 4 Stunden bei Raumtemperatur und dampft anschließend zur Trockene ein. Der Rückstand wird aus Pentan /Diethylether kristallisiert.
Ausbeute: 102,2 g (92 % der Theorie) eines wachsartigen Feststoff

| Elementaranalyse: | | |
|---|---|---|
| ber. | C 69,07 | H 6,71 |
| gef. | C 69,19 | H 6,82 |

### Beispiel 7c

### 6-N-Benzyloxycarbonyl-2-N-[1,4,7-tris (carboxylatomethyl) 1 ,4,7, 10-tetraazacyclododecan-10-(pentanoyl-3-aza-4-oxo-5-methyl-5-yl)]-lysin-[1-(4-perfluoroctylsulfonyl)-piperazin]-amid, Gd-Komplex

50 g (60,20 m mmol) der Titelverbindung aus Beispiel 1c, 6,93 g (60,20 mmol) N-Hydroxysuccinimid, 5,09 g (120 mmol) Lithiumchlorid und 37,91 g (60,20 mmol) 1,4,7-Tris (carboxylatomethyl)-1,4,7,10-tetraazacyclododecan-10-pentanoyl-3-aza-4-oxo-5-methyl-5yl ), Gd-Komplex werden unter leichter Erwärmung in 400 ml Dimethylsulfoxid gelöst. Bei 10° C gibt man 20,63 g (100 mmol) N,N- Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (Kieselgel RP-18, Laufmittel: Gradient aus Wasser / Ethanol / Acetonitril).
Ausbeute: 75,53 g (87 % der Theorie) eines farblosen Feststoffs.
Wassergehalt: 10,1 %

| Elementaranalyse: (berechnet auf wasserfreie Substanz) | | | | | | |
|---|---|---|---|---|---|---|
| ber. | C 37,48 | H 3,84 | F 22,39 | Gd 10,90 | N 8,74 | S 2,22 |
| gef. | C 37,39 | H 4,02 | F 22,29 | Gd 10,75 | N 8,70 | S 2,22 |

### Beispiel 7d

### 2-N-[1,4,7-Tris(carboxylatomethyl)-1,4,7,10-tetraazacyclododecan-10-(pentanoyl-- 3aza-4-oxo-5methyl-5yl]-lysin-[1-(4-perfluoroctylsulfonyl)-piperazin]-amid, Gd-komplex

70 g (48,53 mmol) der Titelverbindung aus Beispiel 7c werden in 500 ml Wasser/100m1 Ethanol gelöst und 5 g Palladium-Katalysator (10 % Pd / C) zugegeben. Man hydriert bei Raumtemperatur. Man filtriert vom Katalysator ab und dampft das Filtrat im Vakuum zur Trockene ein
Ausbeute: 63,5 g (quantitativ) eines farblosen Feststoffs.
Wassergehalt : 9,8 %

| Elementaranalyse: (berechnet auf wasserfreie Substanz) | | | | | | |
|---|---|---|---|---|---|---|
| ber. | C 37,48 | H 3,84 | F 22,39 | Gd 10,90 | N 8,74 | S 2.22 |
| gef | C 37,39 | H 4,03 | F 22,31 | Gd 10,78 | N 8,65 | S 2.20 |

### Beispiel 7e

### 6-N-[5,6-Bis (benzyloxy)-3-oxahexanoyl]-2-N-[1,4,7-tris (carboxylatomethyl)-1,4,710-tetraazacyclododecan-10-(pentanoyl-3aza-4-oxo-5-methyl-5yI)] -lysin-[1-(4-perfluoroctylsulfonyl)-piperazin]-amid, Gd-Komplex

10 g (7,64 mmol) der Titelverbindung aus Beispiel 7d, 3,30 g (10 mmol) der Titelverbindung aus Beispiel 7b, 0,85 g (20 mmol) Lithiumchlorid und 1,15 g (10 mmol) N-Hydroxysuccinimid werden unter leichter Erwärmung in 150 ml Dimethylsulfoxid gelöst. Bei 10° C gibt man 3,10 g (15mmol) N,N' -Dicyclohexylcarbodiimid zu und rührt 8 Stunden bei Raumtemperatur. Die Reaktionslösung wird in 2000 ml Aceton gegossen und der ausgefallene Niederschlag isoliert. Die wird an Kieselgel RP-18 aufgereinigt (Laufmittel : Gradient aus Wasser / Ethanol / Acetonitril).
Ausbeute: 11,14 g (90 % der Theorie) eines farblosen, amorphen Feststoffs.
Wassergehalt: 4,3 %

| Elementaranalyse: (berechnet auf wasserfreie Substanz) | | | | | | |
|---|---|---|---|---|---|---|
| ber. | C 41,51 | H 4,29 | F 19,93 | N 7,78 | Gd 9,70 | S 1,98 |
| gef. | C 41,45 | H 4,38 | F 19,84 | N 7,70 | Gd 9,58 | S 1,90 |

### Beispiel 7f

### 6-N-(5,6,-Dihydroxy-3-oxahexanoyl)-2-N-[1,4,7-tris carboxylatomethyl)-1,4,7,10-tetraazacyclododecan-10-pentanoyl-3-aza-4-oxo-5-methyl-5-yl)]-lysin [1-(4-perfluoroctylsulfonyl)-piperazin]-amid, Gd-Komplex

10g (6,17 mmol) der Titelverbindung aus Beispiel 7e werden in 200 ml Ethanol gelöst und 3 g Palladium-Katalysator (10% Pd / C) zugegeben. Man hydriert bei Raumtemperatur. Man filtriert vom Katalysator ab und dampft das Filtrat im Vakuum zur Trockene ein. Ausbeute: 8,89 g (quantitativ) eines farblosen Feststoffs.
Wassergehalt: 3,1 %

| Elementaranalyse: (berechnet auf wasserfreie Substanz) | | | | | | |
|---|---|---|---|---|---|---|
| ber. | C 35,03 | H 3,99 | F 22,42 | Gd 10,92 | N 8,75 | S 2,23 |
| gef. | C 34,95 | H 4,12 | F 22,30 | Gd 10,78 | N 8,71 | S 2,18 |

### Beispiel 8a

### 6-N-Benzyloxycarbonyl-2-N[-5,6-bis(benzyloxy)-3-oxa-hexanoyl]-lysin-[1-(4-perfluoroctylsulfonyl)-piperazin]-amid

Zu einer Lösung aus 20 g (24,08 mmol) der Titelverbindung aus Beispiel 1c, 9,91 g (30 mmol) der Titelverbindung aus Beispiel 7b und 3,45 g (30 mmol) N-Hydroxysuccinimid, gelöst in 100 ml Dimethylformamid, gibt man bei 0° C 9,28 g (45 mmol) N,N-Dicyclohexylcarbodümid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chromatographiert an Kieselgel (Laufmittel: Dichlor-methan / Ethanol = 20:1) Ausbeute:24,50 g (89 % der Theorie) eines zähen Öls.

| Elementaranalyse: | | | | | |
|---|---|---|---|---|---|
| ber. | C 47,29 | H 4,14 | F 28,26 | N 4,90 | S 2,81 |
| gef. | C 47,14 | H 4,26 | F 28,17 | N 4,91 | S 2,69 |

### Beispiel 8b

### 2-N-(5,6-Dihydroxy-3-oxahexanoyl) -lysin-[1-(4-perfluoroetylsulfonyl)-piperazin]-amid

20 g (17,5 mmol) der Titelverbindung aus Beispiel 8a werden in 300 ml Ethanol gelöst und 5 g Palladium -Katalysator (10% Pd/C) zugegeben. Man hydriert bei Raumtemperatur. Man filtriert vom Katalysator ab und dampft das Filtrat im Vakuum zur Trockene ein. Ausbeute: 17,65 g (quantitativ) eines farblosen Feststoff.

| Elementaranalyse: | | | | | |
|---|---|---|---|---|---|
| ber. | C 44,05 | H 4,10 | F 32,02 | N 5,55 | S 3,18 |
| gef. | C 43,96 | H 4,21 | F 31,94 | N 5,48 | S 3,24 |

### Beispiel 8c

### 6-N-[1,4,7-tris(carboxylatomethyl)-1,4,7,10-tetraazacyclododecan-10-(pentanoyl-3-aza-4-oxo-5-methyl-5yI)]- lysin-[1-(4-perlfluoroctylsulfonyl)-piperazin]-amid , Gd-Komplex

15 g (14,87 mmol) der Titelverbindung aus Beispiel 8b 1,73 g (15 mmol) N-Hydroxysuccinimid, 1,27 g (30 mmol.) Lithiumchlorid und 9,48 g (15 mmol) 1,4,7-Tris ( carboxylatomethyl)-10-(3-aza-4-oxo-5-methyl-5-yl)-pentansäure -1,4,7,10-tetraazacyclododecan, Gd-komplex werden unter leichter Erwärmung in 100 ml Dimethylsulfoxid gelöst. Bei 10° C gibt man 5,16 g (25 mol) N,N-Dicyclohexylcarbodiimid zu und rührt anschließend über Nacht bei Raumtemperatur.Man gießt die Lösung in 1500 ml Aceton und rührt 10 Minuten. Der ausgefallenen Feststoff wird abfiltriert und anschließend durch Chromatographie gereinigt (Kieselgel RP-18 Laufmittel: Gradient aus Wasser / Ethanol / Acetonitril).
Ausbeute: 19,28 g (80 % der Theorie) eines farblosen Feststoffs.
Wassergehalt: 10,3 %

| Elementaranalyse: (berechnet auf wasserfreie Substanz) | | | | | | |
|---|---|---|---|---|---|---|
| ber. | C 41,51 | H 4,29 | F 19,93 | Gd 9,70 | N 7,78 | S 1,98 |
| gef. | C 41,37 | H 4,40 | F 19,88 | Gd 9,58 | N 7,67 | S 1,85 |

### Beispiel 9a

### 6-N-Benzyloxycarbonyl-2-N-[2,6-N,N'-bis(benzyloxycarbonyl)-lysyl]-lysin-[1-(4-perfluoroctylsulfonyl)-piperazin]-amid

20 g (24,08 mmol) der Titelverbindung aus Beispiel 1c und 2,53 g (25 mmol) Triethylamin werden in 200 ml Tetrahydrofuran (THF) gelöst und 14,46 g (27 mmol) Di-N,N'-Z-Lysinparanitrophenolester zugegeben.Man rührt 5 Stunden bei 50° C. Man dampft im Vakuum zur Trockene ein und chromatographiert den Rückstand an Kieselgel. Laufmittel:Dichlormethan / Methanol = 20:1).
Ausbeute: 28,07 g (95% der Theorie) eines farblosen Feststoff.

| Elementaranalyse: | | | | | |
|---|---|---|---|---|---|
| ber. | C 46,99 | H 4,19 | F 26,32 | N 6,85 | S 2,61 |
| gef. | C 47,08 | H 4,32 | F 26,21 | N 6,75 | S 2,54 |

### Beispiel 9b

### 2-N-(Lysyl)-lysin - [1-(4-perfluoroctylsulfonyl)- piperazin]-amid, Trihydrobromid

Zu 25 g (20,37 mmol) der Titelverbindung aus Beispiel 9a gibt man 100 ml Bromwasserstoffsäure in Eisessig (48 %) und rührt 2 Stunden bei 40° C. Man kühlt auf 0° C ab, tropft 1500 ml Diethylester zu und filtriert den ausgefallenen Feststoff ab. Nach Trocknen im Vakuum (60° C) erhält man 21,52 g (99 % der Theorie) leicht gelb gefärbten, kristallinen Feststoff.

| Elementaranalyse: | | | | | | |
|---|---|---|---|---|---|---|
| ber. | C 27,01 | H 3,40 | Br. 22,46 | F 30,26 | N 7,87 | S 3,00 |
| gef. | C 26,92 | H 3,53 | Br. 22,15 | F 30,14 | N 7,69 | S 2,87 |

### Beispiel 9c

### 6-N-[1,4,7-Tris(Carboxylatomethyl)-1,4,7,10-tetraazacyclododecan-10-(pentanoyl-3-aza-4-oxo-5-methyl-5yl)]-2-N-]2,6-N,N'- bis[1,4,7-tris carboxylatomethyl)-1,4,7,10-tetraazacyclododecan-10-(pentanoyl- 3-aza-4-oxo-5-methyl-5yl)]-lysyl]-lysin-[1-(4-perfluoroctylsulfonyl)-piperazin]-amid , Trigadolinium-Komplex

31,49 g (50 mmol) 1,4,7-Tris(Carboxylatomethyl)-10-(3-aza-4-oxo-5-methyl-5-yl) pentansäure, Gd-Komplex 6,91 g (60 mmol) N-Hydroxysuccinimid und 4,24 g (100 mmol) Lithiumchlorid werden in 350 ml Dimethylsulfoxid unter leichten Erwärmen gelöst.Bei 10° C gibt man 16,51 g (80 mmol) N,N- Dicyclohexylcarbodiimid zu und rührt 5 Stunden bei 10° C. Zu dieser Mischung gibt man 10 g (9,37 mmol) der Titelverbindung aus Beispiel 9b und 3,03 g (30 mmol) Triethylamin und rührt 12 Stunden bei 60° C. Man läßt auf Raumtemperatur abkühlen und gießt die Mischung in 3000 ml Aceton. Der ausgefallene Niederschlag wird abfiltriert, an Kieselgel RP-18 gereinigt (Laufmittel : Gradient aus Wasser / Ethanol /Acetonitril). Ausbeute: 16,7 g (67% der Theorie) eines farblosen Feststoffs.
Wassergehalt : 7,9 %

| Elementaranalyse: (berechnet auf wasserfreie Substanz) | | | | | | |
|---|---|---|---|---|---|---|
| ber. | C 36,58 | H 4,43 | F 12,14 | Gd 17,74 | N 11,06 | S 1,14 |
| gef. | C 36,47 | H 4,54 | F 12,03 | Gd 17,65 | N 10,95 | S 1,21 |

### Beispiel 10a

### 1,7-Bis (benzyloxycarbonyl)-4-(3,6,9,12,15-pentaoxahexadecanoyl)- 1,4,7,10-tetraazacyclododecan

Zu 18,13 g (68,1mmol) 3,6,9,12,15-Pentaoxahexadecansäure und 30 g (68,1 mmol) 1,7 Di-Z-Cyclen hergestellt nach Z.Kovacs und A. D. Sherry, J.Chem. Soc .chem. Commun. (1995),2,185, in 300 ml Tetrahydrofuran, gibt man bei 0° C 24,73 g (100 mmol) EEDQ (2-Ethoxy-1,2-dihydrochinolin-1-carbonsäureethylester) zu und rührt über Nacht bei Raumtemperatur.Man dampft im Vakuum zur Trockene ein und chromatographiert an Kieselgel (Laufmittel: Dichlormethan / Methanol = 20:1) Ausbeute: 19,13 g (42 % der Theorie) eines farblosen Feststoffs

| Elementaranalyse: | | | |
|---|---|---|---|
| ber. | C 61,03 | H 7,61 | N 8,13 |
| gef. | C 60,92 | H 7,75 | N 8,04 |

### Beispiel 10b

### 1,7-Bis (benzyloxycarbonyl)-4-(3,6,9,12,15-pentaoxahexadecanoyl)-10-(2H,2H,4H,5H,5H-3-oxa-perfluortridecanoyl)-1,4,7,10-tetraazacyclododecan

Zu 18 g (26,91 mmol) der Titelverbindung aus Beispiel 10a und 14,05 g (26,91 mmol) 2H,2H,4H,4H,5H,5H-3-Oxa-perfluortridecansäure, hergestellt nach DE 19603033, in 300 ml Tetrahydrofuran, gibt man bei 0° C 12,36 g (50 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel : Dichlormethan / Methanol = 20:1)
Ausbeute: 21,51 g (67% der Theorie) eines farblosen Feststoffs

| Elementaranalyse : | | | | |
|---|---|---|---|---|
| ber. | C 47,32 | H 4,82 | F 27,07 | N 4,70 |
| gef. | C 47,26 | H 5,01 | F 26,94 | N 4,59 |

### Beispiel 10c

### 1- (3,6,9,12,15-Pentaoxahexadecanoyl)-7-(2H,2H,4H,4H,5H,5H-3-oxaperfluortridecanoyl)-1,4,7,10-tetraazacyclododecan

20 g (16,77 mmol) der Titelverbindung aus Beispiel 1d werden in 200 ml Ethanol gelöst und 2,5 g Palladium-Katalysator (10 % Pd / C) zugegeben. Man hydriert bei Raumtemperatur. Man filtriert vom Katalysator ab und dampft das Filtrat im Vakuum zur Trockene ein.
Ausbeute: 15,5 g (quantitativ) eines farblosen Feststoffs.

| Elementaranalyse : | | | | |
|---|---|---|---|---|
| ber. | C 40,27 | H 4,90 | F 34,93 | N 6,06 |
| gef. | C 40,15 | H 4,99 | F 34,87 | N 5,94 |

### Beispiel 10d

### 1,7-Bis(1,4,7-tris (carboxylatomethyl)-1,4,7,10-tetraazacyclododecan-10-(pentanoyl-3-aza-4-oxo-5-methyl-5-yl)]-4-(3,6,9,12,15-pentaoxahexadecanoyl)-10-(2H,2H,4H,4H,5H,5H,-3-oxaperfluortridecanoyl)-1,4,7,10-tetraazacyclododecan , Gd-Komplex

15 g (16,22 mmol) der Titelverbindung aus Beispiel 10c, 4,60 g (40 mmol) N-Hydroxysuccinimid, 3,39 g (80 mmol) Lithiumchlorid und 25,19 g (40 mmol) 1,4,7-Tris (carboxylatomethyl)- 10-(3-aza-4-oxo-5-methyl-5-yl) pentansäure, Gd-Komplex werden unter leichter Erwärmung in 300 ml Dimethylsulfoxid gelöst. Bei 10° C gibt man 24,73 g (100 mmol) EEDQ zu und rührt anschließend über Nacht bei Raumtemperatur. Man gießt die Lösung in 3000 ml Aceton und rührt 10 Minuten.
Der ausgefallene Feststoff wird abfiltriert und anschließend durch Chromatographie gereinigt Kieselgel RP-18) Laufmittel: Gradient aus Wasser / Ethanol / Acetonitril).
Ausbeute: 19.86 g (57 % der Theorie) eines farblosen Feststoffs
Wassergehalt: 11,3%

| Elementaranalyse: (berechnet auf wasserfreie Substanz) | | | | | |
|---|---|---|---|---|---|
| ber. | C 38,58 | H 4,74 | F 15,04 | Gd 14,64 | N 9,13 |
| gef. | C 38,47 | H 4,91 | F 14,95 | Gd 14,57 | N 9,04 |

### Beispiel 11a

### 3,5-Dinitrobenzoesäure-1-[(4-perfluoroctysulfonyl)-piperazin]-amid

Zu 20 g (35,2 mmol) Perfluoroctylsulfonylpiperazin und 8,1 g (80 mmol) Triethylamin, gelöst in 200 ml Dichlormethan tropft man bei 0° C eine Lösung aus 8,76 g (38 mmol) 3,5-Dinitrobenzoylchlorid in 55 ml Dichlormethan und rührt 3 Stunden bei 0° C. Man gibt 200 ml 5% ige aqu.Salzsäure zu und rührt 5 Minuten bei Raumtemperatur. Die organische Phase wird abgetrennt, über Magnesiumsulfat getrocknet und im Vakuum zur Trockene eingedampft. Der Rückstand wird an Kieselgel chromatographiert
(Laufmittel: Dichlormethan / Aceton =15:1)
Ausbeute: 24,96 g (93% der Theorie) eines farblosen Feststoffs.

| Elementaranalyse: | | | | | |
|---|---|---|---|---|---|
| ber. | C 29,35 | H 1,45 | F 42,37 | N 7,35 | S 4,21 |
| gef. | C 29,28 | H 1,61 | F 42,15 | N 7,25 | S 4,15 |

### Beispiel 11b

### 3,5 Diaminobenzoesäure-[1-(4-perfluoroctylsulfonyl)-piperazin]-amid

20 g (26,23 mmol) der Titelverbindung aus Beispiel 11a werden in 400 ml Ethanol gelöst und 6 g Palladium-Katalysator (10 % Pd / C) zugegeben.Man hydriert bei Raumtemperatur. Man filtriert vom Katalysator ab und dampft das Filtrat im Vakuum zur Trockene ein. Ausbeute: 18,43 g (quantitativ) eines Cremefarbenen Feststoffs.

| Elementaranalyse: | | | | | |
|---|---|---|---|---|---|
| ber. | C 32,49 | H 2,15 | F 45,98 | N 7,98 | S 4,57 |
| gef. | C 32,29 | H 2,35 | F 45,69 | N 7,81 | S 4,40 |

### Beispiel 11c

### 3,5-N,N'-Bis[1,4,7-tris(carboxylatomethyl)-1,4,7,10-tetraazacyclododecan-10-(pentanoyl-3-aza-4-oxo-5-methyl-5-yl-)]-benzoesäure-[1-(4-perfluoroctylsulfonyl)-piperazin]-amid, Gd-Komplex

10 g (14,24 mmol) der Titelverbindung aus Beispiel 11b, 3,45 g (30 mmol) N-Hydroxysuccinimid, 2,54 g (60 mol) Lithiumchlorid und 18,89 g (30 mmol) 1,4,7-Tris (carboxylatomethyl)-10-(3-aza-4-oxo-5-methyl-5yl)-pentansäure, Gd-Komplex werden unter leichter Erwärmung in 200 ml Dimethylsulfoxid gelöst. Bei 10° C gibt man 10,32 g (50 mmol) N,N-Dicyclohexylcarbodümid zu und rührt anschließend über Nacht bei Raumtemperatur. Man gießt die Lösung in 2000 ml Aceton und rührt 10 Minuten. Der ausgefallene Feststoff wird abfiltriert und anschließend durch Chromatographie gereinigt (Kieselgel RP-18, Laufmittel: Gradient aus Wasser / Ethanol /Acetonitril). Ausbeute: 19,74 g (72 % der Theorie) eines farblosen Feststoffs.
Wassergehalt: 11,8 %

| Elementaranalyse: (berechnet auf wasserfreie Substanz) | | | | | | |
|---|---|---|---|---|---|---|
| ber. | C 35,55 | H 3,72 | F 16,77 | Gd 16,33 | N 10,18 | S 1,67 |
| gef. | C35,48 | H 3,84 | F 16,58 | Gd 16,24 | N 10,07 | S 1,58 |

### Beispiel 12

### a) 3-Oxa-2H,2H,4H,4H,5H,5H-perfluortridecancarbonsäure-t-butylester

25,0 g (53,8 mmol) 1H,1H,2H,2H-Perfluoro-1-decanol [kommerziell erhältlich bei der Firma Lancaster] werden in 250 ml absolutem Toluol gelöst und bei Raumtemperatur mit einer katalytischen Menge (ca. 0,75 g) Tetra-n-butyl-ammoniumhydrogensulfat versetzt.
Anschließend gibt man bei 0°C insgesamt 7,55 g (134,6 mmol; 2,5 equ. bezogen auf die eingesetzte Alkoholkomponente) fein gepulvertes Kaliumhydroxidpulver hinzu, gefolgt von 15,73 g (80,7 mmol; 1,5 equ. bezogen auf die eingesetzte Alkoholkomponente) Bromessigsäure-tert.-butylester und lässt noch 2 Stunden bei 0°C nachrühren. Die so erhaltene Reaktionslösung wird für 12 h bei Raumtemperatur nachgerührt und zum Zwecke der Aufarbeitung wird mit insgesamt 500 ml Essigsäureethylester und 250 ml Wasser versetzt. Die org. Phase wird abgetrennt und zweimal mit Wasser gewaschen. Nach dem Trocknen der organischen Phase über Natriumsulfat wird vom Salz abgesaugt und das Solvens im Vakuum abgezogen. Der verbleibende ölige Rückstand wird an Kieselgel unter Verwendung von Essigsäureethylester/Hexan (1:10) als Eluent gereinigt.
Ausbeute: 26,3 g (84,6% d. Th.) der oben genannten Titelverbindung als farbloses und stark viskoses Öl.

| Elementaranalyse: | | | |
|---|---|---|---|
| ber.: | C 33,23 | H 2,61 | F 55,85 |
| gef.: | C 33,29 | H 2,61 | F 55,90 |

### b) 3-Oxa-2H,2H,4H,4H,5H,5H-perfluortridecancarbonsäure

20,0 g (34,58 mmol) der Titelverbindung aus Beispiel 12a) werden in 200 ml eines Gemisches, bestehend aus Methanol und 0,5 molarer Natronlauge im Verhältnis von 2:1 unter Rühren bei Raumtemperatur suspendiert und anschließend auf 60 °C erwärmt. Nach einer Reaktionszeit von 12 h bei 60 °C wird das nun klare Reaktionsgemisch zur Aufarbeitung durch Versetzen mit Amberlite^{®} IR 120 (H⁺-Form)-Kationenaustauscherharz neutralisiert, vom Austauscher abgesaugt und das so erhaltene methanolisch-wässrige Filtrat im Vakuum bis zur Trockne abgezogen. Der erhaltene amorph-ölige Rückstand wird an Kieselgel unter Verwendung von Essigsäureethylester/n-Hexan (1:3) als Eluent gereinigt.
Ausbeute: 16,0 g (88,6% d. Th.) der oben genannten Titelverbindung als farbloses und stark viskoses Öl.

| Elementaranalyse: | | | |
|---|---|---|---|
| ber.: | C 27,60 | H 1,35 | F 61,85 |
| gef.: | C 27,58 | H 1,36 | F 61,90 |

### c) 1,7-Bis{[1,4,7-tris(carboxylatomethyl)-10-(3-aza-4-oxo-5-methyl-5-yl-pentanoyl)]-1,4,7,10-tetraazacyclododecan}-diethylentriamin, Digadolinium-Komplex

2,48 g [(3,94 mmol); 2,05 Molequivalente bezogen auf eingesetztes Diethylentriamin] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 167 mg wasserfreies Lithiumchlorid (3,94 mmol) werden bei 40 C° in 40 ml absolutem Dimethylsulfoxid unter Rühren gelöst und bei dieser Temperatur mit insgesamt 453 mg (3,94 mmol) N-Hydroxysuccinimid versetzt. Nach dem Abkühlen auf Raumtemperatur wird die so erhaltene Reaktionslösung mit 814 mg (3,946 mmol) N,N'-Dicyclohexylcarbodiimid versetzt und für 2 Stunden bei Raumtemperatur gerührt. Die erhaltene Suspension des Aktivestetrs wird anschließend mit 198,3 mg (1,92 mmol) Diethylentriamin, gelöst in 5 ml absolutem Dimethylsulfoxid, versetzt und für 12 Stunden bei Raumtemperatur gerührt. Zum Zwecke der Aufarbeitung wird das Reaktionsgemisch mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON^{®} YM-3
Ultrafiltrationsmembran (cut off 3000 Da) entsalzt und von niedermolekularen Bestandteilen gereinigt. Das Retentat wird anschließend gefriergetrocknet.
Ausbeute: 1,85 g (72,7 % d. Th.) als farbloses Lyophilisat.
H₂O-Gehalt (Karl-Fischer): 3.89 % .

| Elementaranalyse (berechnet auf wasserfreie Substanz): | | | |
|---|---|---|---|
| ber.: | C 38,03 | H 5,24 N 13,73 | Gd 23,71 |
| gef.: | C 37,98 | H 5,20 N 13,69 | Gd 23,78 |

### d) 1,7-Bis{[1,4,7-tris(carboxylatomethyl)-10-(3-aza-4-oxo-5-methyl-5-yl-pentanoyl)]-1,4,7,10-tetraazacyclododecan}-4-(3-oxa-2H,2H,4H,4H,5H,5H-perfluortridecanoyl)-diethylentriamin ,Digadolinium-Komplex

In eine Lösung von 3,23 g (2,44 mmol) der Titelverbindung aus Beispiel 12c), in einem Gemisch aus 30 ml Dimethylsulfoxid und 3 ml Tetrahydrofuran, werden bei 50°C und unter Stickstoffatmosphäre 1,27 g (2,44 mmol) der Titelverbindung aus Beispiel 12b), gelöst in einem Gemisch aus 15 ml Tetrahydrofuran und 15 ml Dimethylsulfoxid , tropfenweise hinzugegeben. Anschließend gibt man bei 0°C insgesamt 1,80 g (3,66 mmol) EEDQ [2-Ethoxy-1-ethoxycarbonyl-1,2-dihydrochinolin] portionsweise hinzu und läßt über Nacht bei Raumtemperatur rühren. Die erhaltene Reaktionslösung wird anschließend mit ausreichend Aceton bis zur vollständigen Fällung der oben genannten Titelverbindung versetzt, der Niederschlag abgesaugt, getrocknet, in Wasser aufgenommen, von unlöslichen Bestandteilen abfiltriert und das Filtrat über eine AMICON^{®} YM-3 Ultrafiltrationsmembran (cut off 3000 Da) ultrafiltriert, was sowohl zur vollständigen Entsalzung , als auch zur Reinigung der Titelverbindung von niedermolekularen Bestandteilen dient. Das Retentat wird anschließend gefriergetrocknet.
Ausbeute: 3,54 g (79,4 % d. Th.) als farbloses Lyophilisat.
H₂O-Gehalt (Karl-Fischer): 5,87 % .

| Elementaranalyse (berechnet auf wasserfreie Substanz): | | | | | |
|---|---|---|---|---|---|
| ber.: | C 35,43 | H 4,07 | N 9,95 | F 17,64 | Gd 17,18 |
| gef.: | C 35,42 | H 4,01 | N 9,89 | F 17,67 | Gd 17,18 |

### Beispiel 13

### a) 2-N-Trifluoracetyl -6-N- benzyloxycarbonyl-L-lysin

100,0 g (356,7 mmol) 6-N-Benzyloxycarbonyl-L-lysin werden in einer Mischung aus 1000 ml Trifluoressigsäureethylester und 500 ml Ethanol gelöst und 24 Stunden bei Raumtemperatur gerührt. Man dampft zur Trockne ein und kristallisiert den Rückstand aus Diisopropylether.
Ausbeute: 128,9 g (96 % der Theorie) eines farblosen kristallienen Pulvers.
Schmelzpunkt: 98,5 °C.

| Elementaranalyse : | | | | |
|---|---|---|---|---|
| ber.: | C 51,07 | H 5,09 | N 7,44 | F 15,14 |
| gef.: | C 51,25 | H 5,18 | N 7,58 | F 15,03 |

### b) 2-N-Trifluoracetyl -6-N- benzyloxycarbonyl-L-lysin [1-(4-perfluoroctylsulfonyl)-piperazin]-amid

Zu 125,0 g (332,Ommol) der Titelverbindung aus Beispiel 1a) und 188,7 g (332,0 mmol) 1-Perfluoroctylsulfonylpiperazin (hergestellt nach DE 19603033) in 750 ml Tetrahydrofuran ,gibt man bei 0°C 164,2 g (0,664 mmol) EEDQ (2-Ethoxy-1,2-dihydrochinolin -1-carbonsäureethylester) zu und rührt über Nacht bei Raumtemperatur. Man dampft im Vakuum zur Trockene ein und chromatographiert an Kieselgel (Laufmittel: Dichlormethan / Methanol = 20:1).
Ausbeute : 286,0 g (93% der Theorie) eines farblosen Feststoffs.
Schmelzpunkt: 92 °C .

| Elementaranalyse: | | | | | |
|---|---|---|---|---|---|
| ber.: | C 36,30 | H 2,83 | N 6,05 | F 41,01 | S 3,46 |
| gef.: | C 36,18 | H 2,94 | N 5,98 | F 40,87 | S 3,40 |

### c) 6-N- Benzyloxycarbonyl-L-lysin-[1-(4-perfluoroctylsulfonyl)-piperazin]-amid

In eine Lösung aus 280,0 g (302,2mol) der Titelverbindung aus Beispiel 1b) in 2000ml Ethanol, leitet man bei 0°C für eine Stunde Ammoniak-Gas ein. Man rührt anschließend 4 Stunden bei 0°C. Es wird zur Trockene eingedampft und der Rückstand aus Wasser ausgerührt . Man filtriert den Feststoff ab und trocknet im Vakuum bei 50 °C .
Ausbeute: 243,5 g (97,0 % der Theorie) eines amorphen Feststoffs.

| Elementaranalyse: | | | | | |
|---|---|---|---|---|---|
| ber.: | C 37,60 | H 3,28 | N 6,75 | F 38,89 | S 3,86 |
| gef.: | C 37,55 | H 3,33 | N 6,68 | F 38,78 | S 3,81 |

### d) L-Lysin-[1-(4-perfluoroctylsulfonyl)-piperazin]-amid

In 1000 ml Ethanol werden 200,0 g (240,8 mmol) der unter 13c) hergestellten Verbindung gelöst, mit 5,0 g Pearlman-Katalysator (Pd 20% , C) versetzt und solange bei Raumtemperatur unter einer Wasserstoffatmosphäre (latm) hydriert, bis keine Wasserstoffaufnahme mehr zu beobachten ist. Man saugt vom Katalysator ab,wäscht gründlich mit Ethanol nach (dreimal mit jeweils ca. 100 ml) und engt im Vakuum zur Trockne ein. Die Titelverbindung wird als stark viskoses und gelblich gefärbtes Öl erhalten.
Ausbeute: 162,5 g (96,9 % d.Th.).

| Elementaranalyse: | | | | | |
|---|---|---|---|---|---|
| ber.:C | 31,04 | H 3,04 | N 8,05 | F 46,38 | S 4,60 |
| gef.:C | 31,11 | H 3,09 | N 8,08 | F 46,33 | S 4,62 |

### e) 6N-2N-Bis-{4-[2,3-bis-(N,N-bis(t-butyloxycarbonylmethyl)-amino)-propyl]-phenyl}-3-oxa-propionyl-L-lysin-[1-(4-perfluoroctylsulfonyl)-piperazin]-amid

5,25 g (7,72 mmol) der 4-[2,3-Bis-(N,N-bis(t-butyloxycarbonylmethyl)-amino)-propyl]-phenyl}-3-oxa-propionsäure und 781,0 mg (7,72 mmol) Triethylamin werden in 50 ml Methylenchlorid gelöst. Bei -15°C tropft man eine Lösung aus 1,16 g (8,5 mmol) Chlorameisensäureisobutylester in 10 ml Methylchlorid innerhalb 5 Minuten hinzu und rührt noch weitere 20 Minuten bei -15°C. Anschließend kühlt man die Lösung auf -25°C ab und tropft eine Lösung, bestehend aus 2,68 g (3,86 mmol) der Titelverbindung aus Beispiel 13d) und 2,12 g (21,0 mmol) Triethylamin, in 70 ml Tetrahydrofuran innerhalb von 30 Minuten hinzu und rührt im Anschluß noch 30 Minuten bei -15° C und anschließend noch über Nacht bei Raumtemperatur nach. Zur Aufarbeitung wird das Lösungsmittel im Vakuum abgezogen und der verbleibende ölige Rückstand in 250 ml Chloroform aufgenommen. Man extrahiert die Chloroformphase zweimal mit je 100 ml einer 10 %igen wässrigen AmmoniumchloridLösung, trocknet die organische Phase über Magnesiumsulfat und dampft im Vakuum zur Trockne ein. Der Rückstand wird an Kieselgel chromatographiert (Laufmittel:
Methylenchlorid/Ethanol= 20:1).
Ausbeute: 5,37 g (68,8 % d. Th.) eines farblosen und sehr zähen Öls.

| Elementaranalyse: | | | | | |
|---|---|---|---|---|---|
| ber.: | C 52,27 | H 6,43 | N 5,54 | F 15,97 | S 1,59 |
| gef.: | C 52,22 | H 6,51 | N 5,49 | F 15,99 | S 1,63 |

### f) 6N-2N-Bis-{4-[2,3-bis-(N,N-bis(carboxylatomethyl)-amino)-propyl]-phenyl}-3-oxa-propionyl-L-lysin-[1-(4-perfluoroctylsulfonyl)-piperazin]-amid, Octa-Natriumsalz

5,0 g (2,47 mmol) der Titelverbindung aus Beispiel 13e) werden in 60 ml absolutem Dichlormethan gelöst. Anschließend wird bei 0°C mit insgesamt 75 ml Trifluoressigsäure tropfenweise versetzt. Nach einer Reaktionszeit von 12 Stunden bei Raumtemperatur wird im Vakuum bis zur Trockne eingeengt. Der verbleibende Rückstand wird mit 100 ml Wasser versetzt und erneut im Vakuum bis zur Trockne abgezogen. Der so erhaltene Rückstand wird in 200 ml destilliertem Wasser gelöst und die wässrige Produktlösung der oben genannten Titelverbindung zweimal mit jeweils 60 ml Diethylether extrahiert. Die resultierende wässrige Produktlösung wird durch Versetzen mit Wasser auf ein Gesamtvolumen von 300 ml aufgefüllt, von unlöslichen Bestandteilen abfiltriert und das Filtrat über eine AMICON^{®} YM-3 Ultrafiltrationsmembran (cut off 3000 Da) ultrafiltriert, was sowohl zur vollständigen Entsalzung , als auch zur Reinigung der Titelverbindung von niedermolekularen Bestandteilen dient. Das Retentat wird wird durch Versetzen mit Wasser auf ein Gesamtvolumen von 200 ml aufgefüllt und mit 15 % iger Natronlauge wird der pH-Wert dieser Lösung anschließend auf 10,0 eingestellt. Die basische , wässrige Produktlösung wird im Anschluss gefriergetrocknet.
Man erhält 4,0 g (92,8 % d. Th) der Titelverbindung in Form des Octa-Natriumsalzes als amorphes Lyophilisat.
Wassergehalt: 5,37 %

| Elementaranalyse (berechnet auf wasserfreie Substanz): | | | | | | |
|---|---|---|---|---|---|---|
| ber.: | C 38,46 | H 3,28 | N 6,41 | F 18,47 | S 1,83 | Na 10,52 |
| gef.: | C 38,42 | H 3,31 | N 6,39 | F 18,51 | S 1,87 | Na 10,38 |

### g)) 6N-2N-Bis-{4-[2,3-bis-(N,N-bis(carboxymethyl)-amino)-propyl]-phenyl}-3-oxa-propionyl-L-lysin-[1-(4-perfluoroctylsulfonyl)-piperazin]-amid, Di-Mangankomplex, Tetra-Natriumsalz

1,94 g (1,11 mmol) der Titelverbindung aus Beispiel 13f) werden in 100 ml destilliertem Wasser gelöst und die resultierende Lösung wird durch Versetzen mit 1 molarer wässriger Salzsäure auf einen pH-Wert von 4,0 gebracht. Bei 80°C wird nun portionweise mit 0,25 g (2,22 mmol) Mangau-II-carbonat versetzt. Anschließend wird die so erhaltene Reaktionslösung für 5 Stunden unter Rückfluß gekocht. Nach dem Abkühlen auf Raumtemperatur wird der pH-Wert der wässrigen Produktlösung unter kräftigem Rühren durch Versetzen mit 1 N Natronlauge auf 7,2 eingestellt und über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 1,80 g (92,0 % d. Th.) der Titelverbindung als farbloses Lyophilisat.
H₂O-Gehalt (Karl-Fischer): 7,28%.

| Elementaranalyse (berechnet auf wasserfreie Substanz): | | | | | | | |
|---|---|---|---|---|---|---|---|
| ber.: | C 38,07 | H 3,25 | F 18,28 | Mn 6,22 | N 6,34 | Na 5,20 | S 1,81 |
| gef.: | C 38,01 | H 3,29 | F 18,29 | Mn 6,21 | N 6,36 | Na 5,28 | S 1,78 |

### Beispiel 14

### a) 6-N-(Benzyloxycarbonyl)-2-N - [(N-pteroyl) - L-glutaminyl]-lysin-[1-(4-perfluoroctylsulfonyl)-piperazin]-amid

20 g (45,31 mmol) Folsäure werden in 300 ml Dimethylsulfoxid gelöst und bei 10° C 9,49 g (46 mmol) N,N -Dicyclohexylcarbodiimid zugegeben .Man rührt über Nacht bei Raumtemperatur. Zu dieser Mischung gibt man 29,1-g (35 mmol) der Titelverbindung aus Beispiel 1c und 20 ml Pyridin und rührt 3 Stunden bei 50° C. Es wird auf Raumtemperatur abgekühlt und eine Mischung aus 1500 ml Diethylether/ 1500 ml Aceton zugeben. Der ausgefallene Niederschlag wird abfiltriert und an (RP-18) gereinigt (Laufmittel =Gradient aus Wasser / Ethanol / Tetrahydrofuran).
Ausbeute : 21,59 g (38 % der Theorie), gelber Feststoff.
Wassergehalt : 2,1 %

| Elementaranalyse (berechnet auf wasserfreie Substanz): | | | | | |
|---|---|---|---|---|---|
| ber. | C 43,10 | H 3,54 | F 25,76 | N 11,29 | S 2,56 |
| gef. | C 43,02 | H 3,62 | F 25,68 | N 11,21 | S 2,48 |

### b) 2-N-[(N-Pteroyl)-L-glutaminyl]-lysin-[1-(4-perfluoroctylsulfonyl) piperazin]-amid

Zu 20 g (15,95 mmol) der Titelverbindung aus Beispiel 14 a gibt man 200 ml Bromwasserstoffsäure in Eisessig (48 %) und rührt 2 Stunden bei 40 °C. Man kühlt auf 0°C ab , tropft 2000 ml Diethylether zu und filtriert den ausgefallenen Feststoff ab. Nach Trocknen im Vakuum (60 °C) erhält man 18,96 g (99 % der Theorie) eines gelb gefärbten, kristallinen Feststoffs.

| Elementaranalyse: | | | | | | |
|---|---|---|---|---|---|---|
| ber. | C 37,01 | H 3,27 | Br 6,65 | F 26,90 | N 12,83 | S 2,67 |
| gef. | C 36,91 | H 3,42 | Br 6,31 | F 29,75 | N12,72, | S 2,56 |

### c) 6-N-[1,4,7-Tris (carboxylatomethyl)-1,4,7,10-tetraazacyclododecan -10-(pentanoyl-3-aza-4oxo-5-methyl-5yl]-2-N-[(N-pteroyl]-L-glutaminyl] lysin-[1-(4-perfluoroctylsulfonyl)-piperazin]-amid, Gd-Komplex

0,92 g (8 mmol)N-Hydroxysuccinimid, 0,68 g (16 mol) Lithiumchlorid und 5,04 g (8 mmol) 1,4,7-Tris(carboxylatomethyl-10-(3-aza-4-oxo-5-methyl-5-yl)- 1,4,7-10-tetraazacyclododecan, Gd-Komplex werden unter leichter Erwärmung in 80 ml Dimethylsulfoxid gelöst. Bei 10° C gibt man 2,06 g (10 mol) N , N-Dicyclohexylcarbodiimid zu und rührt anschließend 3 Stunden bei Raumtemperatur. Zu dieser Reaktionslösung gibt man 5 g (4,16 mmol) der Titelverbindung aus Beispiel 14 b und 10 ml Pyridin, Man rührt über Nacht bei Raumtemperatur. Man gießt die Lösung in 1000 ml Aceton und rührt 10 Minuten. Der ausgefallene Feststoff wird abfiltriert und anschließend durch Chomatographie gereinigt (Kieselgel R P -18, Laufmittel : Gradient aus Wasser /Ethanol /Acetonitril. Man löst in etwas Wasser, stellt den pH Wert mit Natronlauge auf 7,4 und gefriergetrocknet. Ausbeute : 3,87 g (53 % der Theorie) eines gelben Feststoffs Wassergehalt: 5,8 %,

| Elementaranalyse : (auf wasserfreie Substanz berechnet) | | | | | | | |
|---|---|---|---|---|---|---|---|
| ber. | C 38,36 | H 3,74 | F 18,42 | Gd 8,97 | N 12,78 | Na 1,31 | S 1,83 |
| gef. | C 38,28 | H 3,85 | F 18,33 | Gd 8,85 | N 12,69 | Na 1,42 | S 1,75 |

### Beispiel 15

### a) 6-N- Benzyloxycarbonyl- 2-N- (3,6,9,12- tetraoxatridecanoyl)-lysin [1- (4-perfluoroctylsulfonyl) - piperazin] - amid

Zu 50 g (60,20 mmol) der Titelverbindung aus Beispiel 1c und 7,10 g (70 mmol) Triethylamin, gelöst in 350 ml Dichlormethan, tropft man bei 0° C eine Lösung aus 16,85 g (70 mmol) 3,6,9,12 Tetraoxatridecansäurechlorid in 50 ml Dichlormethan und rührt 3 Stunden bei 0° C. Man gibt 200 ml 5% ige aqu Salzsäure zu und rührt 5 Minuten bei Raumtemperatur. Die organische Phase wird abgetrennt, über Magnesiumsulfat getrocknet und im Vakuum zur Trockene eingedampft. Der Rückstand wird an Kieselgel chromatographiert (Laufmittel: Dichlormethan / Aceton = 15:1
Ausbeute: 30,94 g (92% der Theorie) eines farblosen, zähen Öls.

| Elementaranalyse: | | | | | |
|---|---|---|---|---|---|
| ber. | C 40,63 | H 4,19 | F 31,21 | N 5,41 | S 3,10 |
| gef. | C 40,75 | H 4,08 | F 31,29 | N 5,58 | S 3,25 |

### b) 2-N-(3,6,9,12- tetraoxatridecanoyl)-lysin [1-(4-perfluoroctylsulfonyl) -piperazin]-amid

53,96 g (52,15 mmol) der Titelverbindung aus Beispiel 15a werden in 500 ml Ethanol gelöst und 6 g Palladium-Katalysator (10 % Pd / C) zugegeben. Man hydriert bei Raumtemperatur. Man filtriert vom Katalysator ab und dampft das Filtrat im Vakuum zur Trockene ein. Ausbeute: 43,0 g (quantitativ) eines farblosen Feststoffs.

| Elementaranalyse: | | | | | |
|---|---|---|---|---|---|
| ber. | C 36,01 | H 4,14 | F 35,86 | N 6,22 | S 3,56 |
| gef. | C 27,60 | H 5,13 | F 39,09 | N 6,68 | S 3,81 |

### c) 6-N-[1,4,7-Tris (carboxylatomethyl) -1,4,7,10- tetraazacyclododecan-10 -(pentanoyl- 3-aza- 4- oxo- 5- methyl- 5-yl)] - 2-N- (3,6,9,12- tetraoxatridecanoyl)-lysin [1-(4-perfluoroctylsulfonyl)-piperazin ]-amid, Gd-Komplex

21,84 g (24,25 mmol) der Titelverbindung aus Beispiel 15b, 2,79 g (24,25 mmol) N-Hydroxysuccinimid, 2,12 g (50 mmol) Lithiumchlorid und 15,27g (24,25 mmol) 1,4,7-Tris (carboxylatomethyl)-10-[(3-aza- 4- oxo- 5- methyl- 5-yl)]-pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser / Ethanol / Acetonitril).Ausbeute: 28,21 g (81 % der Theorie) eines farblosen Feststoffs.
Wassergehalt: 11,0 %

| Elementaranalyse (auf wasserfreie Substanz berechnet) | | | | | | |
|---|---|---|---|---|---|---|
| ber. | C 36,53 | H 4,33 | F 21,36 | N 8,34 | S 2,12 | Gd 10,40 |
| gef. | C 31,74 | H 4,98 | F 22,39 | N 8,69 | S 2,15 | Gd 10,87 |

### Beispiel 16

### a) 6-N- Benzyloxycarbonyl- 2-N- (propyl-3-sulfonsäure) - lysin [1- (4- perfluoroctylsulfonyl)- piperazin]-amid

Zu 50 g (60,20 mmol) der Titelverbindung aus Beispiel 1c und 7,10 g (70 mmol) Triethylamin, gelöst in 250 ml ,trockenem Tetrahydrofuran, tropft man bei 50° C eine Lösung aus 7,33g (60 mol) Propansulton in 50 ml Tetrahydrofuran und rührt 3 Stunden bei 60° C. Man gibt 200 ml 5% i ge aqu Salzsäure zu und rührt 5 Minuten bei Raumtemperatur. Die organische Phase wird abgetrennt, über Magnesiumsulfat getrocknet und im Vakuum zur Trockene eingedampft. Der Rückstand wird an Kieselgel chromatographiert (Laufmittel: Dichlormethan / Aceton =15:1
Ausbeute: 45,16g (79 % der Theorie) eines farblosen, zähen Öls.

| Elementaranalyse: | | | | | |
|---|---|---|---|---|---|
| ber. | C 36,56 | H 3,49 | F 33,90 | N 5,88 | S 6,73 |
| gef. | C 36,72 | H 3,35 | F 33,79 | N 5,78 | S 6,75 |

### b) 2-N-(propyl-3-sulfonsäure)- lysin [1-(4- perfluoroctylsulfonyl) piperazin]-amid

49,68 g (52,15 mmol) der Titelverbindung aus Beispiel 16a werden in 500 ml Ethanol gelöst und 6 g Palladium-Katalysator (10 % Pd / C) zugegeben. Man hydriert bei Raumtemperatur. Man filtriert vom Katalysator ab und dampft das Filtrat im Vakuum zur Trockene ein. Ausbeute: 42,69 g (quantitativ) eines farblosen Feststoffs.

| Elementaranalyse: | | | | | |
|---|---|---|---|---|---|
| ber. | C 30,81 | H 3,32 | F 39,46 | N 6,84 | S 7,83 |
| gef. | C 30,64 | H 4,1 | F 39,29 | N 6,68 | S 7,89 |

### c) 6-N-[1,4,7-Tris (carboxylatomethyl) -1,4,7,10- tetraazacyclododecan-10 -(pentanoyl- 3-aza- 4- oxo- 5- methyl- 5-yl)] - 2-N- (propyl-3-sulfonsäurel)-lysin [1-(4--perfluoroctylsulfonyl)-piperazin]-amid, Gd-Komplex

19,85 g (24,25 mmol) der Titelverbindung aus Beispiel 16b, 2,79 g (24,25 mmol) N-Hydroxysuccinimid, 2,12 g (50 mmol) Lithiumchlorid und 15,27g (24,25 mmol) 1,4,7-Tris (carboxylatomethyl)-10-[(3-aza- 4- oxo- 5- methyl-5-yl)]-pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser / Ethanol / Acetonitril).
Ausbeute: 28,13 g (81 % der Theorie) eines farblosen Feststoffs.
Wassergehalt: 11,0 %

| Elementaranalyse (auf wasserfreie Substanz berechnet) | | | | | | |
|---|---|---|---|---|---|---|
| ber. | C 33,27 | H 3,70 | F 22,36 | N 8,73 | S 4,44 | Gd 10,89 |
| gef. | C 32,41 | H 3,88 | F 22,49 | N 8,69 | S 4,35 | Gd 10,97 |

### Beispiel 17

### a) 6-N- Benzyloxycarbonyl- 2-N,N-bis (propyl-3-sulfonsäure)- lysin [1- (4-perfluoroctylsulfonyl)-piperazin] - amid

Zu 50 g (60,20 mmol) der Titelverbindung aus Beispiel 1c und 12,14 g (120 mmol) Triethylamin, gelöst in 250 ml trockenem Tetrahydrofuran tropft man bei 50° C eine Lösung aus 14,65 g (120 mmol) 1,3-Propansulton in 100 ml Tetrahydrofuran und rührt 3 Stunden bei 60° C. Man gibt 400 ml 5% ige aqu Salzsäure zu, rührt 5 Minuten bei Raumtemperatur, versetzt mit Natriumchlorid., trennt die organische Phase ab, trocknet sie über Magnesiumsulfat und dampft sie im Vakuum zur Trockene .ein. Der Rückstand wird an Kieselgel chromatographiert (Laufmittel: Dichlormethan / Aceton = 15:1
Ausbeute: 52,24 g (81 % der Theorie) eines farblosen, zähen Öls.

| Elementaranalyse: | | | | | |
|---|---|---|---|---|---|
| ber. | C 35,76 | H 3,66 | F 30,05 | N 5,21 | S 8,95 |
| gef. | C 35,75 | H 3,55 | F 30,19 | N 5,08 | S 9,04 |

### b) 2-N,N bis(propyl-3-sulfonsäure) - lysin [1-(4- perfluoroctylsulfonyl) piperazin] - amid

53,74 g (52,15 mmol) der Titelverbindung aus Beispiel 17a werden in 500 ml Ethanol gelöst und 6 g Palladium-Katalysator (10 % Pd / C) zugegeben. Man hydriert bei Raumtemperatur. Man filtriert vom Katalysator ab und dampft das Filtrat im Vakuum zur Trockene ein.
Ausbeute: 49,06 g (quantitativ) eines farblosen Feststoffs.

| Elementaranalyse: | | | | | |
|---|---|---|---|---|---|
| ber. | C 30,64 | H 3,54 | F 34,33 | N 5,96 | S 10,23 |
| gef. | C 30,69 | H 3,71 | F 34,19 | N 6,08 | S 10,38 |

### c) 6-N-[1,4,7-Tris (carboxylatomethyl) -1,4,7,10- tetraazacyclododecan-10 -(pentanoyl- 3-aza- 4- oxo- 5- methyl- 5-yl)]-2-N,N bis(propyl-3-sulfonsäure)-lysin [1-(4-perfluoroctylsulfonyl)-piperazin]-amid, Gd-Komplex, Dinatriumsalz

38,76 g ( 24,25 mmol) der Titelverbindung aus Beispiel 17b 2,79 g (24,25 mmol) N-Hydroxysuccinimid, 2,12 g (50 mmol) Lithiumchlorid und 15,27g (24,25 mmol) 1,4,7-Tris (carboxylatomethyl) -10-[(3-aza-4-oxo-5-methyl-5-yl)]-pentansäure]- 1,4,7,10-tetraazacyclododecan, Gd-Komplex werden unter leichter Erwärmung in 200 ml Dimethylsulfoxid gelöst. Bei 10° C gibt man 8,25 g (40 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser / Ethanol / Acetonitril).Ausbeute: 31,63 g (81 % der Theorie) eines farblosen Feststoffs. Wassergehalt: 11,0 %

| | | | | | | | |
|---|---|---|---|---|---|---|---|
| Elementaranalyse (auf wasserfreie Substanz berechnet) | | | | | | | |
| ber.: | C 32,07 | H 3,57 | F 20,06 | N 7,83 | S 5,97 | Gd 9,76 | Na 2,86 |
| gef. | C 31,94 | H 3,48 | F 20,19 | N 7,69 | S 5,85 | Gd 9,87 | Na 2,99 |

### Beispiel 18

### a) N-Trifluoracetyl- L-glutaminsäure-5-benzylester

100 g (421,5 mmol) L-Glutaminsäure-5-benzylester werden in einer Mischung aus 1000 ml Trifluoressigsäureethylester / 500 ml Ethanol gelöst und 24 Stunden bei Raumtemperatur gerührt. Man dampft zur Trockene ein und kristallisiert den Rückstand aus Diisopropylether.
Ausbeute: 140,47 g (96 % der Theorie) eines farblosen kristallienen Pulvers.

| Elementaranalyse: | | | | |
|---|---|---|---|---|
| ber. | C 50,46 | H 4,23 | F 17,10 | N 4,20 |
| gef. | C 51,35 | H 4,18 | F 17,03 | N 4,28 |

### b) 2-N-Trifluoacetyl-L-glutaminsäure-5-benzylester-N-bis(2-hydroxyethyl)-amid

Zu einer Lösung aus 24,9 g (24,08 mmol) der Titelverbindung aus Beispiel 18a , 2,53 g (24,08 mmol) Diethanolamin und 2,77 g (24,08 mmol) N - Hydroxysuccinimid, gelöst in 150 ml Dimethylformamid, gibt man bei 0° C 8,25 g (40 mmol) N,N-Dicyclohexylcabodiimid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chomatographiert am Kieselgel (Laufmittel: = Dichlormethan / Ethanol = 20:1).
Ausbeute: 9,11 g (90% der Theorie) eines zähen öls.

| Elementaranalyse : | | | | |
|---|---|---|---|---|
| ber. | C 51,43 | H 5,51 | F 13,56 | N 6,66 |
| gef. | C 51,22 | H 5,41 | F 13,40 | N 6,75 |

### c) N-Trifluoracetyl-L-glutaminsäure-N bis(2-hydroxyethyl)-monoamid

21,92 g (52,15 mmol) der Titelverbindung aus Beispiel 18b werden in 500 ml Ethanol gelöst und 3 g Palladium-Katalysator (10 % Pd/C) zugegeben. Man hydriert bei Raumtemperatur. Man filtriert vom Katalysator ab und dampft das Filtrat im Vakuum zur Trockene ein. Ausbeute: 43,0 g (quantitativ) eines farblosen Feststoffs.

| Elementaranalyse: | | | | |
|---|---|---|---|---|
| ber. | C 40,01 | H 5,19 | F 17,26 | N 8,48 |
| gef. | C 39,84 | H 5,13 | F 17,09 | N 8,68 |

### d) Trifluoracetyl-L-glutaminsäure-N-bis(2-hydroxyethyl)-amid-5-[1-(4-perfluoroctylsulfonyl)-piperazin]-amid

Zu 10,96g (33,2 mmol) der Titelverbindung aus Beispiel 18a und 18,87g (33,2 mmol) 1-Perfluoroctylsulfonyl - piperazin , (hergestellt nach DE 19603033) in 80 ml Tetrahydrofuran, gibt man bei 0° C 16,42 g (66,4 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel :
Dichlormethan / Methanol =20:1)
Ausbeute: 30,93 g (93 % der Theorie) eines farblosen Feststoffs.

| Elementaranalyse: | | | | | |
|---|---|---|---|---|---|
| ber. | C 39,61 | H 2,89 | F 35,66 | N 6,19 | S 3,54 |
| gef. | C 39,68 | H 2,74 | F 35,81 | N 6,13 | S 3,40 |

### e) L-Glutaminsäure-N- bis(2-hydroxyethyl)-amid-5- [1-(4- perfluoroctylsulfonyl)-piperazin] - amid

In eine Lösung aus 30,24 g (30,22 mmol) der Titelverbindung aus Beispiel 18b in 200 ml Ethanol, leitet man bei 0° C für eine Stunde Ammoniak-Gas ein. Man rührt anschließend 4 Stunden bei 0° C. Es wird zur Trockene eingedampft und der Rückstand aus Wasser ausgerührt. Man filtriert den Feststoff ab und trocknet im Vakuum (50° C).
Ausbeute : 26,55 g (97 % der Theorie) eines amorphen Feststoffs.

| Elementaranalyse: | | | | | |
|---|---|---|---|---|---|
| ber. | C 41,12 | H 2,89 | F 35,66 | N 6,19 | S 3,54 |
| gef. | C 41,15 | H 2,83 | F 35,78 | N 6,28 | S 3,71 |

### f) N-[1,4,7-Tris (carboxylatomethyl) -1,4,7,10- tetraazacyclododecan-10 -(pentanoyl- 3-aza-4- oxo- 5- methyl- 5-yl)] -L-glutaminsäure-N-bis(2-hydroxyethyl)-amid-5-[1-(4-perfluoroctylsulfonyl)-piperazin]-amid, Gd-Komplex

211,96 g (24,25 mmol) der Titelverbindung aus Beispiel 18e, 2,79 g (24,25 mmol) N-Hydroxysuccinimid, 2,12 g (50 mmol) Lithiumchlorid und 15,27g (24,25 mmol) 1,4,7-Tris (carboxylatomethyl) -10 -[(3-aza- 4- oxo- 5- methyl- 5-yl)]- pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol) N,NDicyclohexylcarbodümid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser /Ethanol / Acetonitril).
Ausbeute: 27,43 g (81 % der Theorie) eines farblosen Feststoffs.
Wassergehalt: 11,0 %

| Elementaranalyse (auf wasserfreie Substanz berechnet) | | | | | | |
|---|---|---|---|---|---|---|
| ber. | C 34,41 | H 3,83 | F 23,13 | N 9,03 | S 2,30 | Gd 11,26 |
| gef. | C 34,34 | H 3,98 | F 23,29 | N 9,19 | S 2,15 | Gd 11,07 |

### Beispiel 19

### a) N-Trifluoracetyl-L-glutaminsäure-5-benzylester-N-dimethyl-bis(1,1-dihydroxymethyl)-amid[

Zu einer Lösung aus 8,03g (24,08 mmol) der Titelverbindung aus Beispiel 18a, 3,98 g ( 24,08 mmol) Dimethyl-bis(1,1-dihydroymethyl)-amin und 2,77 g (24,08 mmol) N - Hydroxysuccinimid, gelöst in 150 ml Dimethylformamid, gibt man bei 0° C 8,25 g (40 mmol) N,N- Dicyclohexylcabodiimid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chromatographiert am Kieselgel (Laufmittel: Dichlormethan/ Ethanol = 20:1).
Ausbeute: 110,53 g (91% der Theorie) eines zähen Öls.

| Elementaranalyse : | | | | |
|---|---|---|---|---|
| ber. | C 50,00 | H 5,66 | F 11,86 | N 7,18 |
| gef. | C 50,17 | H 5,82 | F 11,80 | N 7,15 |

### b) N-Trifluoracetyl-L-glutaminsäure-5-benzylester- [1-(4- perfluoroctylsulfonyl) -piperazin] - amid

25,05 g (52,15 mmol) der Titelverbindung aus Beispiel 19a werden in 500 ml Ethanol gelöst und 6 g Palladium-Katalysator (10 % Pd /C) zugegeben. hydriert bei Raumtemperatur. Man filtriert vom Katalysator ab und dampft das Filtrat im Vakuum zur Trockene ein.
Ausbeute: 20,36 g (quantitativ) eines farblosen Feststoffs.

| Elementaranalyse: | | | | |
|---|---|---|---|---|
| ber. | C 40,00 | H 5,42 | F 14,60 | N 7,18 |
| gef. | C 40,10 | H 5,53 | F 14,69 | N 7,28 |

### c) N-Trifluoracetyl-L.glutaminsäure-N-dimethyl-bis(1,1-dihydroxymethyl)-amid-5- [1-(4-perfluöroctylsulfonyl) piperazin]-amid

Zu 12,96 g (33,2 mmol) der Titelverbindung aus Beispiel 19b und 18,87g (33,2 mmol) 1-Pertluoroctylsulfonyl - piperazin, (hergestellt nach DE 19603033) in 800 ml Tetrahydrofuran, gibt man bei 0° C 16,42 g (66,4 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel : Dichlormethan /Methanol =20:1)
Ausbeute: 28,42 g (91% der Theorie) eines farblosen Feststoffs.

| Elementaranalyse: | | | | | |
|---|---|---|---|---|---|
| ber. | C 31,93 | H 3,00 | F 40,40 | N 5,96 | S 3,41 |
| gef. | C 32,08 | H 2,94 | F 40,57 | N 5,88 | S 3,31 |

### d) L-Glutaminsäure-N-[dimethyl-bis(1;1-dihydroxymethyl)]-amid-5-[(1- 4-perfluoroctylsulfonyl)- piperazin] - amid

In eine Lösung aus 28,41 g (30,2 mmol) der Titelverbindung aus Beispiel 19c in 200 ml Ethanol, leitet man bei 0°C für eine Stunde Ammoniak-Gas ein. Man rührt anschließend 4 Stunden bei 0° C. Es wird zur Trockene eingedampft und der Rückstand aus Wasser ausgerührt. Man filtriert den Feststoff ab und trocknet im Vakuum (50° C).
Ausbeute : 24,74 g (97 % der Theorie) eines amorphen Feststoffs.

| Elementaranalyse: | | | | | |
|---|---|---|---|---|---|
| ber. | C 32,71 | H 3,46 | F 38,24 | N 6,63 | S 3,80 |
| gef. | C 32,75 | H 3,33 | F 38,38 | N 6,68 | S 3,81 |

### e) 2-N-[1,4,7-Tris (carboxylatomethyl) -1,4,7,10- tetraazacyclododecan-10-(pentanoyl-3-aza- 4- oxo- 5- methyl- 5-yl)] L-glutaminsäure-N-[dimethyl-bis(1,1-dihydroxymethyl)-amid]-5- [1-(4-perfluoroctylsulfonyl)-piperazin]-amid, Gd-Komplex

20,48 g (24,25 mmol) der Titelverbindung aus Beispiel 19d 2,79 g (24,25 mmol) N-Hydroxysuccinimid, 2,12 g (50 mmol) Lithiumchlorid und 15,27g (24,25 mmol) 1,4,7-Tris (carboxylatomethyl)-10-[(3-aza- 4- oxo- 5- methyl- 5-yl)]- pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser/Ethanol/Acetonitril).
Ausbeute: 29,05 g (83% der Theorie) eines farblosen Feststoffs.
Wassergehalt: 11,0 %

| Elementaranalyse (auf wasserfreie Substanz berechnet) | | | | | | |
|---|---|---|---|---|---|---|
| ber. | C 34,12 | H 3,91 | F 22,38 | N 8,73 | S 2,22 | Gd 10,90 |
| gef. | C 34,24 | H 3,98 | F 22,39 | N 8,69 | S 2,15 | Gd 10,87 |

### Beispiel 20

### a) N-Trifluormethylacetyl-L-glutaminsäure-5-benzylester-[1-(4-perfluoroctylsulfonyl)-piperazin]-amid

Zu 11,06 g (33,2 mmol) der Titelverbindung aus Beispiel 18a und 18,87g (33,2 mmol) 1-Perfluoroctylsulfonyl - piperazin , (hergestellt nach DE 19603033) in 80 ml Tetrahydrofuran, gibt man bei 0° C 16,42 g (66,4 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel :
Dichlormethan / Methanol =20:1)
Ausbeute: 27,28 g (93 % der Theorie) eines farblosen Feststoffs.

| Elementaranalyse: | | | | | |
|---|---|---|---|---|---|
| ber. | C 35,35 | H 2,40 | F 43,01 | N 4,76 | S 3,63 |
| gef. | C 35,48 | H 2,51 | F 42,87 | N 4,73 | S 3,50 |

### b) N-Trifluoracetyl-L-glutaminsäure-5-[1-[4-perfluoroctylsulfonyl)-piperazin]-amid

21,92 g (52,15 mmol) der Titelverbindung aus Beispiel 18a werden in 500 ml Ethanol gelöst und 3 g Palladium-Katalysator (10 % Pd / C) zugegeben. Man hydriert bei Raumtemperatur. Man filtriert vom Katalysator ab und dampft das Filtrat im Vakuum zur Trockene ein. Ausbeute: 41,37 g (quantitativ) eines farblosen Feststoffs.

| Elementaranalyse: | | | | | |
|---|---|---|---|---|---|
| ber. | C 28,76 | H 1,91 | F 47,89 | N 5,30 | S 4.04 |
| gef. | C 28,84 | H 2,03 | F 47,79 | N 5,28 | S 4,19 |

### c) N-Trifluoacetyl-L-glutaminsäure-N-bis(2-hydroxyethyl)-amid-5-[1-(4-perfluoroctylsulfonyl)-piperazin]-amid

Zu einer Lösung aus 24,9 g (24,08 mmol) der Titelverbindung aus Beispiel 18a , 2,53 g (24,08 mmol) Diethanolamin und 2,77 g (24,08 mmol) N - Hydroxysuccinimid, gelöst in 150 ml Dimethylformamid, gibt man bei 0° C 8,25 g (40 mmol) N,N-Dicyclohexylcarbodümid zu. Man rührt 3 Stunden bei 0° C, anschließend über Nacht bei Raumtemperatur. Man filtriert vom ausgefallenen Harnstoff ab, dampft das Filtrat im Vakuum zur Trockene ein und chomatographiert am Kieselgel (Laufmittel: = Dichlormethan / Ethanol = 20:1).
Ausbeute: 9,11 g (90% der Theorie) eines zähen Öls.

| Elementaranalyse : | | | | | |
|---|---|---|---|---|---|
| ber. | C 31,37 | H 2,75 | F 43,15 | N 6,36 | S 3,64 |
| gef. | C 31,22 | H 2,61 | F 43,30 | N 6,25 | S 3,81 |

### d) L-Glutaminsäure-N- bis(2-hydroxyethyl)-amid-5- [1- (4- perfluoroctylsulfonyl)-piperazin] . - amid

In eine Lösung aus 26,61 g (30,22 mmol) der Titelverbindung aus Beispiel 18c in 200 ml Ethanol, leitet man bei 0° C für eine Stunde Ammoniak-Gas ein. Man rührt anschließend 4 Stunden bei 0° C. Es wird zur Trockene eingedampft und der Rückstand aus Wasser ausgerührt. Man filtriert den Feststoff ab und trocknet im Vakuum (50° C).
Ausbeute : 23,93 g (97 % der Theorie) eines amorphen Feststoffs.

| Elementaranalyse: | | | | | |
|---|---|---|---|---|---|
| ber. | C 30,89 | H 3,09 | F 39,56 | N 6,86 | S 3,93 |
| gef. | C 30,75 | H 3,13 | F 39,78 | N 6,75 | S 3,81 |

### e) N-[1,4,7-Tris (carboxylatomethyl) -1,4,7,10- tetraazacyclododecan-10 -(pentanoyl- 3-aza-4-oxo-5-methyl-5-yl)] -L-glutaminsäure-N-bis(2-hydroxyethyl)-amid-5- [1-(4-perfluoroctylsulfonyl)-piperazin]-a.mid, Gd-Komplex

16,43 g (24,25 mmol) der Titelverbindung aus Beispiel 20d, 2,79 g (24,25 mmol) N-Hydroxysuccinimid, 2,12 g (50 mmol) Lithiumchlorid und 15,27g (24,25 mmol) 1,4,7-Tris (carboxylatomethyl) -10 -[(3-aza- 4- oxo- 5- methyl- 5-yl)]- pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol) N,NDicyclohexylcarbodümid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt (Kieselgel RP-18, Laufmittel: Gradient aus Wasser /Ethanol / Acetonitril).Ausbeute: 28,10 g (83 % der Theorie) eines farblosen Feststoffs. Wassergehalt: 11,0 %

| Elementaranalyse (auf wasserfreie Substanz berechnet) | | | | | | |
|---|---|---|---|---|---|---|
| ber. | C 34,41 | H 3,83 | F 23,13 | N 9,03 | S 2,30 | Gd 11,26 |
| gef. | C 34,44 | H 4,98 | F 23,19 | N 8,89 | S 2,15 | Gd 11,17 |

### Beispiel 21

### a) N-Trifluoracetyl-glutaminsäure-5-benzylester-[1-(4-perfluoroctylsulfonyl)-piperazin]-amid

Zu 11,06g (33,2 mmol) der Titelverbindung aus Beispiel 18a und 18,87g (33,2 mmol) 1-Perfluoroctylsulfonyl - piperazin , (hergestellt nach DE 19603033) in 80 ml Tetrahydrofuran, gibt man bei 0° C 16,42 g (66,4 mmol) EEDQ (2-Ethoxy-1,2-dihydrochinolin-1-carbonsäureethylester) zu und rührt über Nacht bei Raumtemperatur. Man dampft im Vakuum zur Trockene ein und chromatographiert an Kieselgel (Laufmittel :
Dichlormethan Methanol =20:1)
Ausbeute: 27,28 g (93 % der Theorie) eines farblosen Feststoffs.

| Elementaranalyse: | | | | | |
|---|---|---|---|---|---|
| ber. | C 35,35 | H 2,40 | F 43,01 | N 4,76 | S 3,63 |
| gef. | C 35,48 | H 2,54 | F 42,87 | N 4,73 | S 3,40 |

### b) N-Trifluoracetyl-L-glutaränsäure-5-[1-[4-perfluoroctylsulfonyl)-piperazin]-amid

21,92 g (52,15 mmol) der Titelverbindung aus Beispiel 21a werden in 500 ml Ethanol gelöst und 3 g Palladium-Katalysator (10 % Pd / C) zugegeben. Man hydriert bei Raumtemperatur. Man filtriert vom Katalysator ab und dampft das Filtrat im Vakuum zur Trockene ein. Ausbeute: 41,37 g (quantitativ) eines farblosen Feststoffs.

| Elementaranalyse: | | | | | |
|---|---|---|---|---|---|
| ber. | C 28,76 | H 1,91 | F 47,89 | N 5,30 | S 4.04 |
| gef. | C 28,84 | H 1,81 | F 47,79 | N 5,28 | S 4.16 |

### Beispiel 22

### Organverteilung (einschließlich Tumor- und Lymphknotenanreicherung) nach intravenöser Gabe des erfindungsgemäßen Kontrastmittels aus Beispiel 3 in Prostatakarzinom-tragenden Ratten.

Nach intravenöser Applikation von 100 µmol Gesamtgadolinium/kg KGW der Titelverbindung aus Beispiel 3 in Ratten (Cop-Inzucht, mit 12 Tage zuvor i.m. implantiertem Prostatakarzinom Dunning R3327 MAT-Lu) wurde 10 Minuten, 1 und 24 Stunden nach Applikation der Metallgehalt in verschiedenen Organen, im Tumor sowie in den Lymphknoten (gepoolt als mesenteriale und periphere Lymphknoten) bestimmt (MW ± SD, n=3).

**Titelverbindung aus Beispiel 3**

| | Gd-Konzentration [*µ*mol/l] | | | | | | | | | % Dosis pro Gesamtgewebe | | | | | | | | |
|---|---|---|---|---|---|---|---|---|---|---|---|---|---|---|---|---|---|---|
| | 10min p.i. | | | 1h p.i. | | | 24h p.i. | | | 10min p.i. | | | 1h p.i. | | | 24h p.i. | | |
| Leber | 137 | ± | 39 | 136 | ± | 1 | 172 | ± | 6 | 3,79 | ± | 1,12 | 3,93 | ± | 0,20 | 5,37 | ± | 0,63 |
| Milz | 184 | ± | 58 | 161 | ± | 3 | 161 | ± | 19 | 0,25 | ± | 0,07 | 0,23 | ± | 0,01 | 0,25 | ± | 0,01 |
| Pankreas | 99 | ± | 26 | 95 | ± | 15 | 55 | ± | 7 | 0,25 | ± | 0,08 | 0,23 | ± | 0,07 | 0,18 | ± | 0,01 |
| Niere | 359 | ± | 88 | 394 | ± | 41 | 292 | ± | 18 | 1,70 | ± | 0,39 | 2,00 | ± | 0,21 | 1,38 | ± | 0,07 |
| Lunge | 344 | ± | 95 | 321 | ± | 16 | 146 | ± | 19 | 1,30 | ± | 0,33 | 1,27 | ± | 0,05 | 0,56 | ± | 0,03 |
| Herz | 177 | ± | 46 | 151 | ± | 7 | 65 | ± | 12 | 0,40 | ± | 0,11 | 0,33 | ± | 0,01 | 0,15 | ± | 0,03 |
| Gehirn | 16 | ± | 5 | 16 | ± | 3 | 5 | ± | 0 | 0,09 | ± | 0,02 | 0,10 | ± | 0,02 | 0,03 | ± | 0,00 |
| Muskel** | 41 | ± | 12 | 40 | ± | 4 | 13 | ± | 2 | 0,12 | ± | 0,04 | 0,08 | ± | 0,01 | 0,03 | ± | 0,01 |
| Tumor | 82 | ± | 32 | 126 | ± | 10 | 100 | ± | 6 | 0,20 | ± | 0,07 | 0,40 | ± | 0,12 | 0,46 | ± | 0,42 |
| Femur | 61 | ± | 10 | 64 | ± | 5 | 33 | ± | 1 | 0,50 | ± | 0,06 | 0,50 | ± | 0,02 | 0,26 | ± | 0,01 |
| mes. LK | 155 | ± | 40 | 160 | ± | 5 | 127 | ± | 7 | 0,11 | ± | 0,04 | 0,10 | ± | 0,01 | 0,09 | ± | 0,01 |
| periph. LK | 115 | ± | 27 | 186 | ± | 6 | 108 | ± | 6 | 0,13 | ± | 0,03 | 0,19 | ± | 0,03 | 0,11 | ± | 0,02 |
| Magen (entleert) | 90 | ± | 26 | 93 | ± | 3 | 48 | ± | 8 | 0,47 | ± | 0,16 | 0,50 | ± | 0,06 | 0,27 | ± | 0,03 |
| Darm (entleert) | 146 | ± | 37 | 130 | ± | 7 | 101 | ± | 12 | 2,48 | ± | 0,56 | 1,85 | ± | 0,27 | 1,63 | ± | 0,15 |
| Blut* | 621 | ± | 137 | 534 | ± | 12 | 169 | ± | 16 | 35,18 | ± | 7,43 | 30,63 | ± | 1,05 | 9,58 | ± | 1,02 |
| Restkörper | -- | ± | -- | - | ± | - | 103 | ± | 7 | -- | ± | -- | - | ± | -- | 31,0 5 | ± | 4,60 |
| Harn 0-24 h | -- | - | -- | - | - | - | 60 | ± | 19 | -- | - | -- | - | - | -- | 36,3 8 | ± | 2,36 |
| Faeces 0-24 h | -- | - | - | -- | - | -- | 561 | ± | 28 | -- | - | - | -- | - | - | 8,91 | ± | 2,29 |
| | | | | | | Summe der Organe *** | | | | 46,35 | ± | 9,98 | 41,79 | ± | 1,76 | 50,9 5 | ± | 4,52 |
| | | | | | | Bilanz | | | | -- | ± | -- | -- | ± | -- | 96, 24 | ± | 3,47 |
| * 58ml Blut/kg KGW | | | | | | | | | | | | | | | | | | |
| ** nur Gewebealiquot v. rechten Unterschenkelmuskel | | | | | | | | | | | | | | | | | | |
| *** Summe Organe 10 und 60 min p.i. ohne Restkörper | | | | | | | | | | | | | | | | | | |

## Patentansprüche

1. Perfluoralkylhaltige Komplexe mit polaren Resten der allgemeinen Formel I 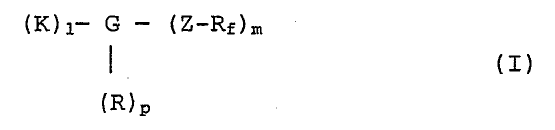 in der
R_{f} eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
K für einen Metallkomplex der allgemeinen Formel II steht, 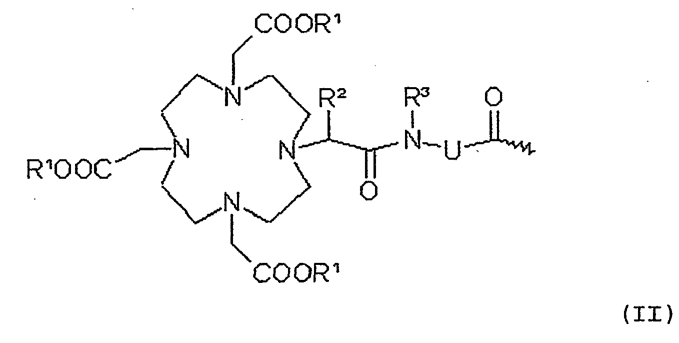
in der
R¹ ein Wasserstoffatom oder ein Metallionenäquivalent der Ordnungszahlen 21-29, 31-33, 37-39, 42-44, 49 oder 57-83 bedeutet,
mit der Maßgabe, daß mindestens zwei R¹ für Metallionenäquivalente stehen
R² und R³ unabhängig voneinander Wasserstoff, C₁-C₇-Alkyl, Benzyl, Phenyl, -CH₂OH oder -CH₂OCH₃ darstellen und
U -C₆H₄-O-CH₂-ω-, -(CH₂)₁₋₅-ω, eine Phenylengruppe, -CH₂-NHCO-CH₂-CH(CH₂COOH) -C₆H₄-ω-, -C₆H₄- (OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3 - (CH₂)₀₋₅COOH-Gruppen substituierte C₁-C₁₂-Alkylen-oder C₇-C₁₂-C₆H₄-0-Gruppe darstellt, wobei ω für die Bindungsstelle an -CO- steht,
oder
der allgemeinen Formel III 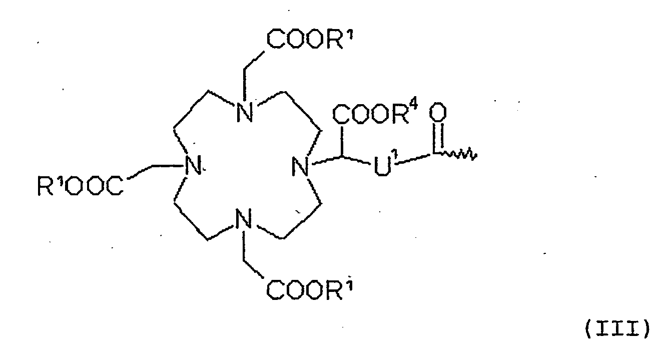 in der R¹ die oben genannte Bedeutung hat, R⁴ Wasserstoff oder ein unter R¹ genanntes Metallionenäquivalent darstellt und U¹-C₆H₄-O-CH₂-ω- darstellt, wobei ω die Bindungsstelle an -CO-bedeutet
oder der allgemeinen Formel IV 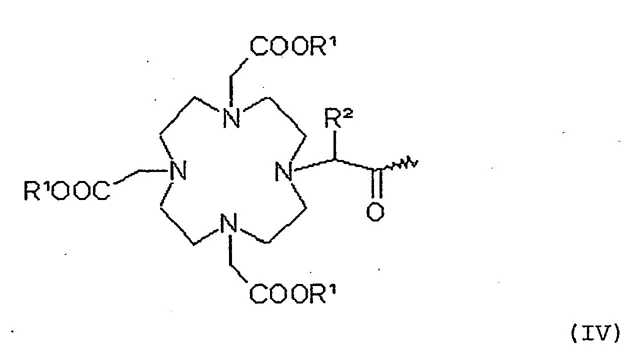 in der R¹ und R² die oben genannte Bedeutung haben
oder der allgemeinen Formel V A oder V B 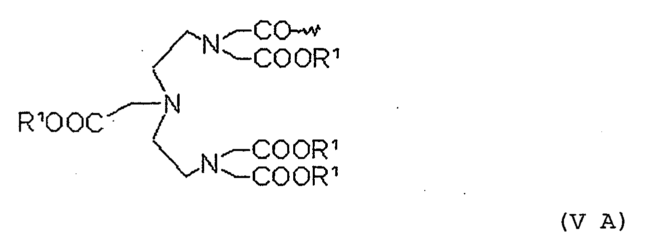 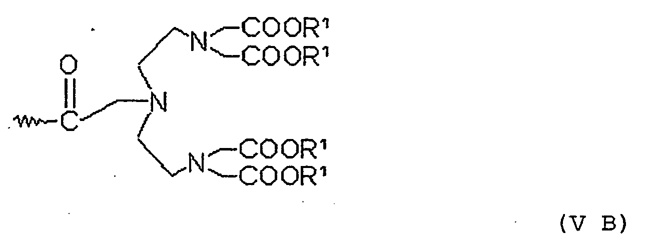 in der R¹ die oben genannte Bedeutung hat,
oder der allgemeinen Formel VI 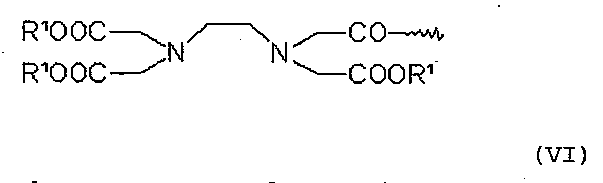 in der R¹ die oben genannte Bedeutung hat,
oder der allgemeinen Formel VII 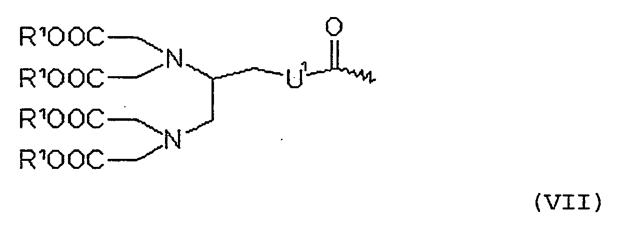 in der R¹ die oben genannte Bedeutung hat und
U¹ -C₆H₄-O-CH₂-ω- darstellt, wobei ω die Bindungsstelle an -CO-bedeutet
und im Rest K gegebenenfalls vorhandene freie Säuregruppen gegebenenfalls als Salze organischer und/oder anorganischer Basen oder Aminosäuren oder Aminosäureamide vorliegen können,
G einen mindestens dreifach funktionalisierten Rest ausgewählt aus den nachfolgenden Resten a) bis g) darstellt
(a) 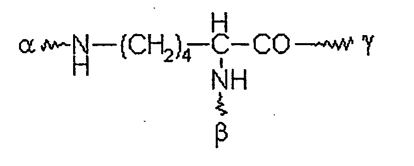
(b) 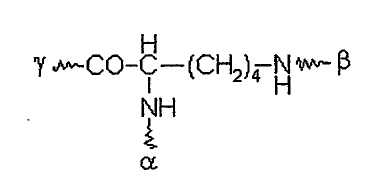
(c) 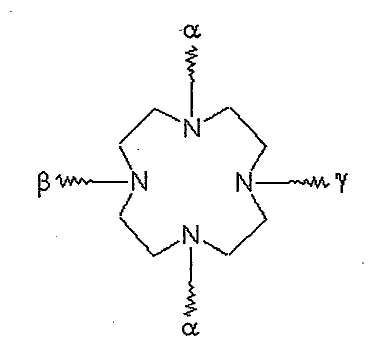
(d) 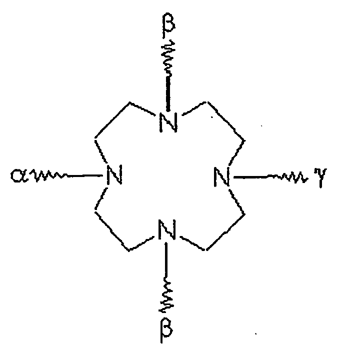
(e) 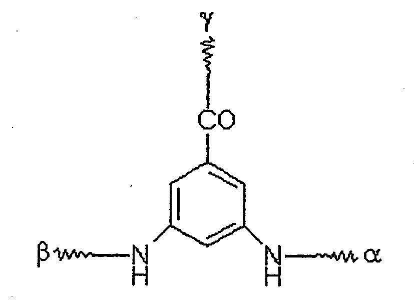
(f) 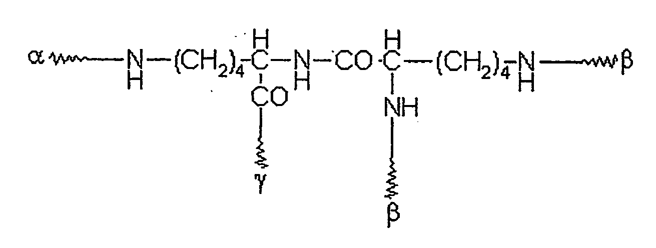
(g) 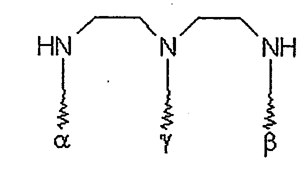
(h) 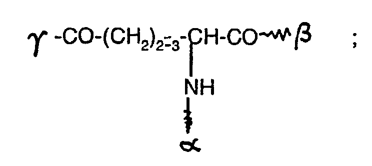
(i) 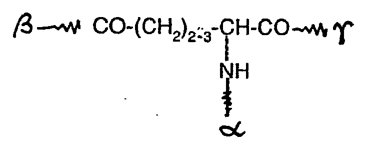
wobei α die Bindungsstelle von G an den Komplex K bedeutet, β die Bindungsstelle von G zum Rest R ist und γ die Bindungsstelle von G zum Rest Z darstellt
Z für 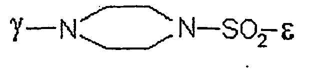 γ-C(O) CH₂O(CH₂)₂-ε,
steht, wobei γ die Bindungsstelle von Z zum Rest G darstellt und ε die Bindungsstelle von Z an den perfluorierten Rest R_{f} bedeutet
R einen polaren Rest ausgewählt aus den Komplexen K der allgemeinen Formeln II bis VII darstellt, wobei R¹ hier ein Wasserstoffatom oder ein Metallionenäquivalent der Ordnungszahlen 20-29, 31-33, 37-39, 42-44, 49 oder 57-83 bedeutet,
und die Reste R², R³, R⁴, U und U¹ die oben angegebene Bedeutung aufweisen, wobei für den Fall, dass G den Rest (c) oder (d) und R einen Komplex ausgewählt aus den allgemeinen Formeln II und V darstellen, darf R nicht identisch mit dem Rest K der allgemeinen Formel I sein, wenn Z für δ-C(O)CH₂O(CH₂)₂-ε steht,
oder
den Folsäurerest
oder
eine über -CO- , SO₂- oder eine direkte Bindung an den Rest G gebundene Kohlenstoffkette mit 2-30 C-Atomen bedeutet, geradlinig oder verzweigt, gesättigt oder ungesättigt,
gegebenenfalls unterbrochen durch 1-10 Sauerstoffatome, 1-5 -NHCO-Gruppen, 1-5 -CONH-Gruppen, 1-2 Schwefelatome, 1-5 -NH-Gruppen oder 1-2 Phenylengruppen, die gegebenenfalls mit 1-2 OH-Gruppen, 1-2 NH₂-Gruppen, 1-2 -COOH-Gruppen, oder 1-2 -SO₃H-Gruppen substituiert sein können
oder
gegebenenfalls substituiert mit 1-8 OH-Gruppen, 1-5-COOH-Gruppen, 1-2 SO₃H-Gruppen, 1-5 NH₂-Gruppen, 1-5 C₁-C₄-Alkoxygruppen,
und
l, m, p unabhängig voneinander die ganzen Zahlen 1 oder 2 bedeuten.

2. Metallkomplexe nach Anspruch 1,
**dadurch gekennzeichnet, daß**
das Metallionenäquivalent R¹ im Rest K ein Element der Ordnungszahlen 21-29, 39, 42, 44 oder 57-83 ist.

3. Metallkomplexe nach Anspruch 1,
**dadurch gekennzeichnet, daß**
das Metallionenäquivalent R¹ im Rest K ein Element der Ordnungszahlen 27, 29, 31-33, 37-39, 43, 49, 62, 64, 70, 75 und 77 ist.

4. Metallkomplexe nach einem der Ansprüche 1 bis 3,
**dadurch gekennzeichnet, daß**
K für einen Metallkomplex der allgemeinen Formel II, .III , VB oder VII steht.

5. Metallkomplexe nach einem der Ansprüche 1 bis 4,
**dadurch gekennzeichnet, daß**
der polare Rest R die Bedeutung des Komplexes K hat.

6. Metallkomplexe nach Anspruch 5,
**dadurch gekennzeichnet, dass**
als polare Reste R die Komplexe K der allgemeinen Formeln II, III, V A oder VII vorhanden sind.

7. Metallkomplexe nach Anspruch 5 oder 6,
**dadurch gekennzeichnet, dass**
R¹ ein Metallionenäquivalent der Ordnungszahlen 20, 25 oder 64 bedeutet.

8. Metallkomplexe nach einem der Ansprüche 1 bis 4,
**dadurch gekennzeichnet, daß**
der polare Rest R folgende.Bedeutungen hat:
-C (O) CH₂CH₂SO₃H
-C (O) CH₂OCH₂CH₂OCH₂CH₂OH
-C (O) CH₂OCH₂CH₂OH
-C (O) CH₂OCH₂CH (OH) CH₂OH
-C (O) CH₂NH-C (O) CH₂COOH
-C (O) CH₂CH (OH) CH₂OH
-C(O)CH₂OCH₂COOH
-SO₂CH₂CH₂COOH
-C (O) -C₆H₃- (m-COOH)₂
-C (O) CH₂O (CH₂)₂-C₆H₃- (m-COOH) ₂
-C (O) CH₂O-C₆H₄-m-SO₃H
-C (O) CH₂NHC (O) CH₂NHC (O) CH₂OCH₂COOH
-C (O) CH₂OCH₂CH₂OCH₂COOH
-C (O) CH₂OCH₂CH (OH) CH₂O-CH₂CH₂OH
-C (O) CH₂OCH₂CH (OH) CH₂OCH₂-CH (OH) -CH₂OH
-C(O)CH₂SO₃H
-C (O)CH₂CH₂COOH
-C(O)CH(OH)CH(OH)CH₂OH
-C (O) CH₂O[(CH₂)₂O]₁₋₉-CH₃
-C (O) CH₂O [(CH₂)₂O]₁₋₉-H
-C(O)CH₂OCH(CH₂OH)₂
-C(O)CH₂OCH(CH₂OCH₂COOH)₂
-C(O)-C₆H₃-(m-OCH₂COOH)₂
-CO-CH₂O-(CH₂)₂O(CH₂)₂O-(CH₂)₂O(CH₂)₂OCH₃
vorzugsweise -C(O)CH₂O[(CH₂)₂O]₄-CH₃.

9. Metallkomplexe nach einem der Ansprüche 1 bis 4,
**dadurch gekennzeichnet, daß**
der polare Rest R der Folsäurerest ist.

10. Metallkomplexe nach einem der Ansprüche 1 bis 9,
**dadurch gekennzeichnet, daß**
G in der allgemeinen Formel I den Lysinrest (a) oder (b) darstellt.

11. Metallkomplexe nach einem der Ansprüche 1 bis 10,
**dadurch gekennzeichnet, daß**
U im Metallkomplex K die Gruppe -CH₂- oder -C₆H₄-O-CH₂-ω darstellt, wobei ω für die Bindungsstelle an -CO- steht.

12. Verwendung von Metallkomplexen nach Anspruch 2 zur Herstellung von Kontrastmitteln zur Anwendung in der NMR-und Röntgendiagnostik.

13. Verwendung von Metallkomplexen nach Anspruch 12 zur Herstellung von Kontrastmitteln zum Infarkt- und Nekrose-Imaging.

14. Verwendung von Metallkomplexen nach Anspruch 3 zur Herstellung von Kontrastmitteln zur Anwendung in der Radiodiagnostik und Radiotherapie.

15. Verwendung von Metallkomplexen nach Anspruch 2 zur Herstellung von Kontrastmitteln für die Lymphographie zur Diagnose von Veränderungen des Lymphsystems.

16. Verwendung von Metallkomplexen nach Anspruch 2 zur Herstellung von Kontrastmitteln für die Anwendung in der indirekten Lymphographie.

17. Verwendung von Metallkomplexen nach Anspruch 2 zur Herstellung von Kontrastmitteln für die Anwendung in der intravenösen Lymphographie.

18. Verwendung von Metallkomplexen nach Anspruch 2 zur Herstellung von Kontrastmitteln zur Darstellung des Vasalraumes.

19. Verwendung von Metallkomplexen nach Anspruch 2 zur Herstellung von Kontrastmitteln für das Tumorimaging.

20. Pharmazeutische Mittel enthaltend mindestens eine physiologisch verträgliche Verbindung nach den Ansprüchen 1 bis 11, gegebenenfalls mit den in der Galenik üblichen Zusätzen.

21. Verfahren zur Herstellung von perfluoralkylhaltigen Komplexen mit polaren Resten der allgemeinen Formel I 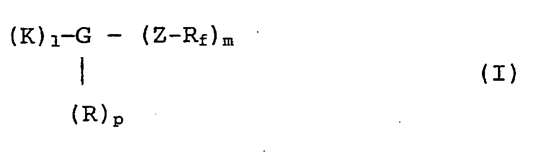 in der K, G, R, Z, R_{f}, l, m und p die in Anspruch 1 angegebene Bedeutung haben,
**dadurch gekennzeichnet, dass**
man in an sich bekannter Weise eine Carbonsäure der allgemeinen Formel IIa 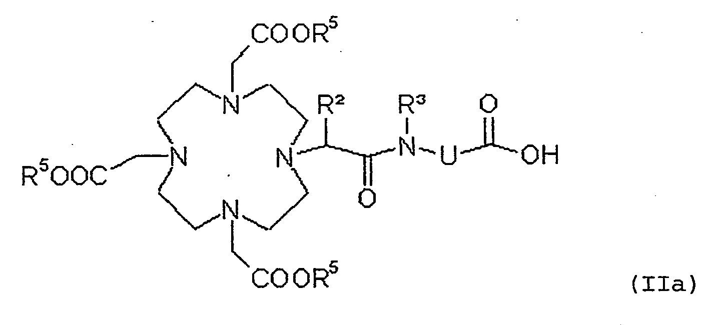 worin R⁵ ein Metallionenäquivalent der Ordnungszahlen 21-29, 31-33, 37-39, 42-44, 49 oder 57-83 oder eine Carboxylschutzgruppe bedeutet, und R², R³ und U die genannte Bedeutung haben
oder eine Carbonsäure der allgemeinen Formel IIIa 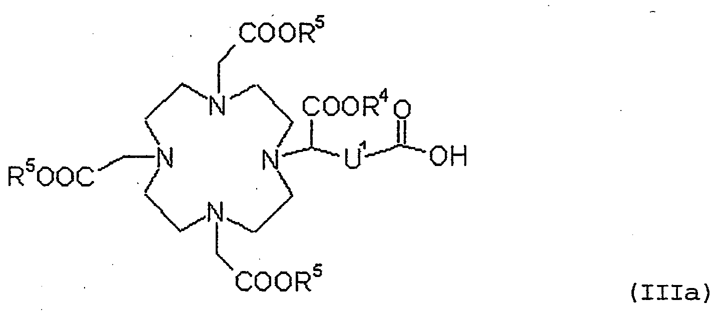 worin R⁴, R⁵ und U¹ die genannte Bedeutung haben
oder eine Carbonsäure der allgemeinen Formel IVa 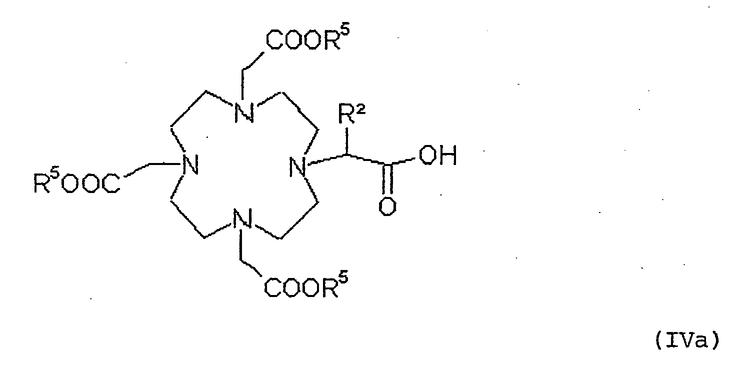 worin R⁵ und R² die genannte Bedeutung haben
oder eine Carbonsäure der allgemeinen Formel Va oder Vb 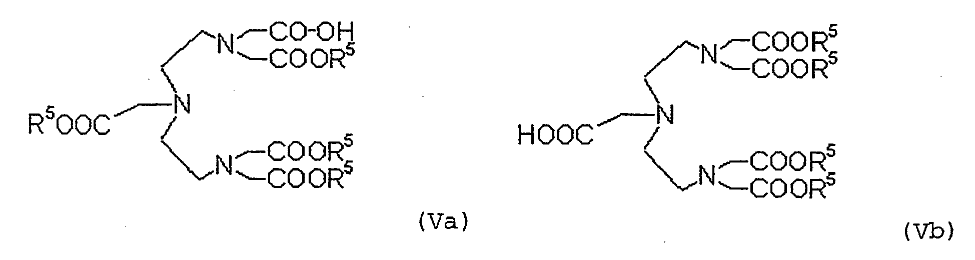 worin R⁵ die genannte Bedeutung hat
oder eine Carbonsäure der allgemeinen Formel VIa 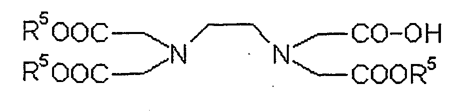 worin R⁵ die genannte Bedeutung hat
oder eine Carbonsäure der allgemeinen Formel VIIa 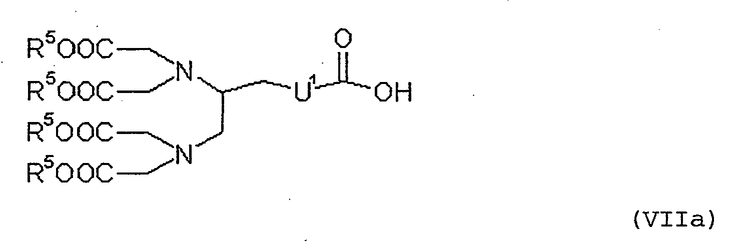 worin R⁵ und U¹ die genannten Bedeutungen haben,
in gegebenenfalls aktivierter Form mit einem Amin der allgemeinen Formel VIII 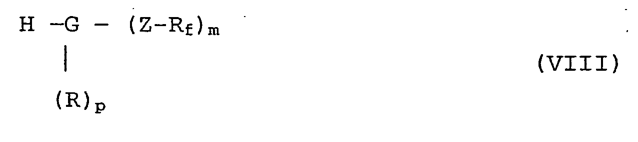 in der G, R, Z, R_{f}, m und p die angegebene Bedeutung haben, in einer Kupplungsreaktion und gegebenenfalls nachfolgender Abspaltung gegebenenfalls vorhandener Schutzgruppen zu einem Metallkomplex der allgemeinen Formel I umsetzt
oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

## Claims

1. Perfluoroalkyl-containing complexes with polar radicals of formula I 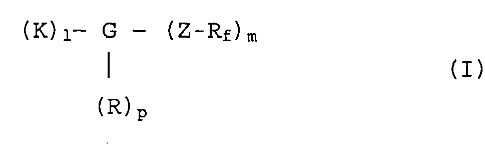 in which R_{f} is a perfluorinated, straight-chain or branched carbon chain with the formula -CₙF₂ₙE, in which E is a terminal fluorine, chlorine, bromine, iodine or hydrogen atom, and n is a number from 4-30,
K is a metal complex of formula II 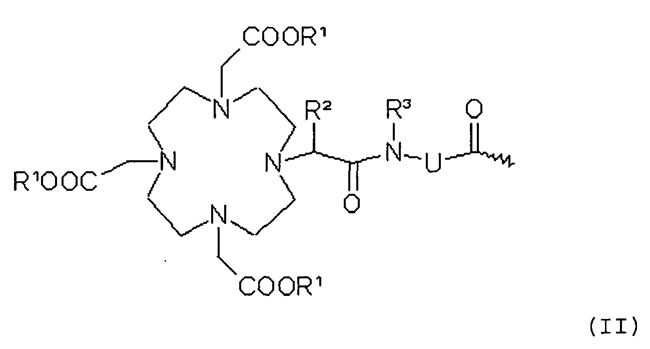 in which R¹ is a hydrogen atom or a metal ion equivalent of atomic numbers 21-29, 31-33, 37-39, 42-44, 49 or 57-83, provided that at least two R¹ are metal ion equivalents,
R² and R³, independently of one another, are hydrogen, C₁-C₇-alkyl, benzyl, phenyl, -CH₂OH or -CH₂OCH₃, and
U is -C₆H₄-O-CH₂-ω-, -(CH₂)₁₋₅-ω, a phenylene group, -CH₂-NHCO-CH₂-CH (CH₂COOH) -C₆H₄-ω-, -C₆H₄- (OCH₂CH₂)₀₋₁-N(CH₂COOH)-CH₂-ω or a C₁-C₁₂ alkylene group or C₇-C₁₂-C₆H₄ -O group that is optionally interrupted by one or more oxygen atoms, 1 to 3 -NHCO groups or 1 to 3 -CONH groups and/or is substituted with 1 to 3 - -(CH₂)₀₋₅COOH groups, where ω stands for the binding site to -CO-, or
of formula III 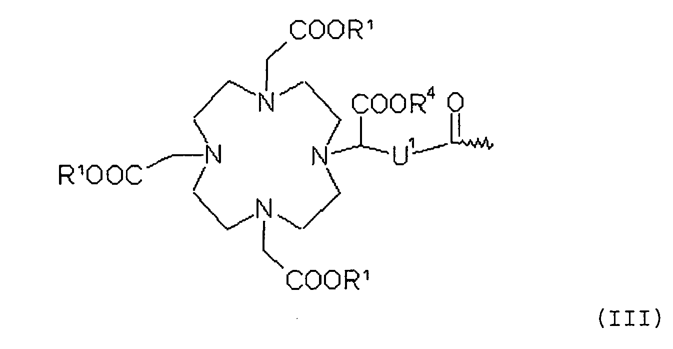 in which R¹ has the above-mentioned meaning, R⁴ is hydrogen or a metal ion equivalent that is mentioned under R¹, and U¹ represents -C₆H₄-O-CH₂-ω-, where ω means the binding site to -CO-, or of formula IV 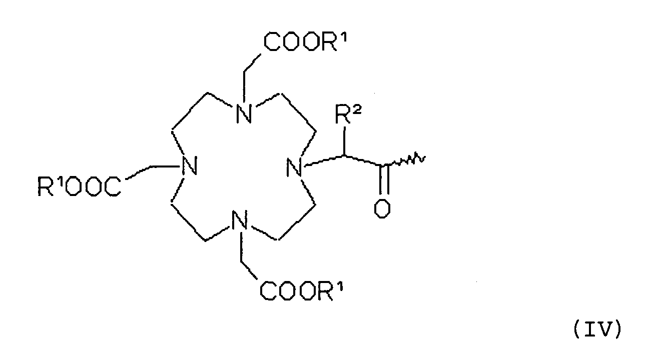 in which R¹ and R² have the above-mentioned meaning
or of formula V A or V B 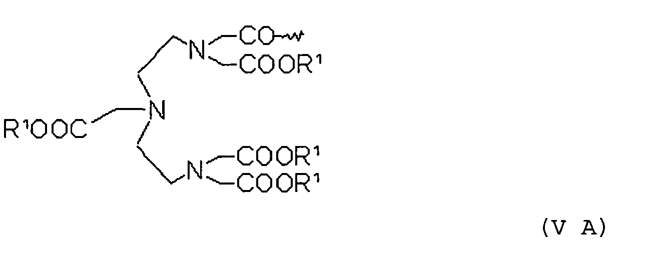 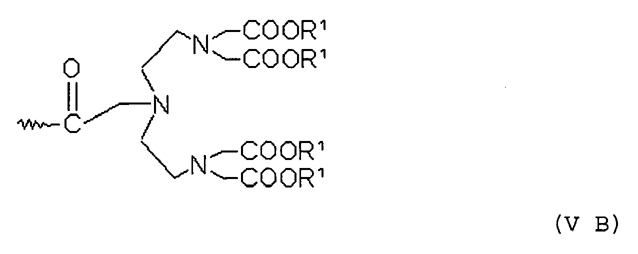 in which R¹ has the above-mentioned meaning, or of formula VI 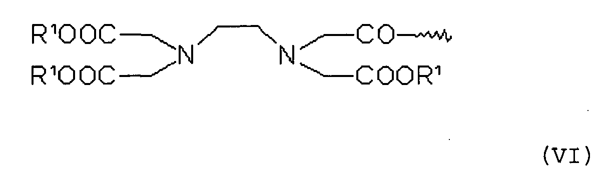 in which R¹ has the above-mentioned meaning, or of formula VII 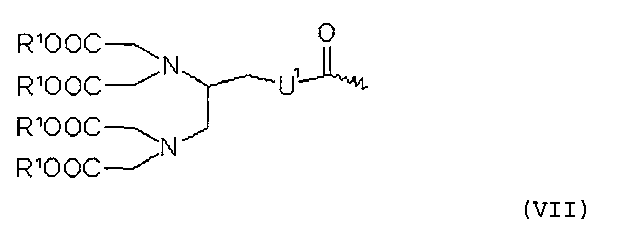 in which R¹ has the above-mentioned meaning, and U¹ is - C₆H₄-O-CH₂-ω-, where ω means the binding site to -CO-, and in radical K, optionally present free acid groups optionally can be present as salts of organic and/or inorganic bases or amino acids or amino acid amides,
G is a radical that is functionalized in at least three places and that is selected from radicals a) to g) below:
(a) 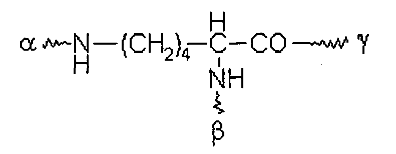
(b) 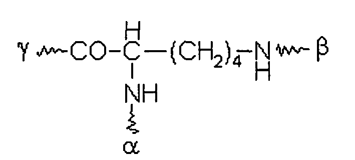
(c) 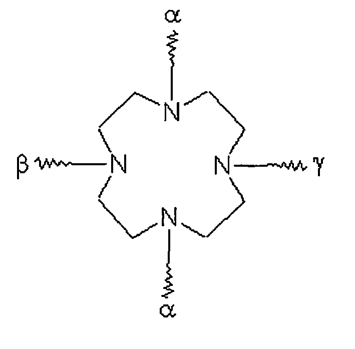
(d) 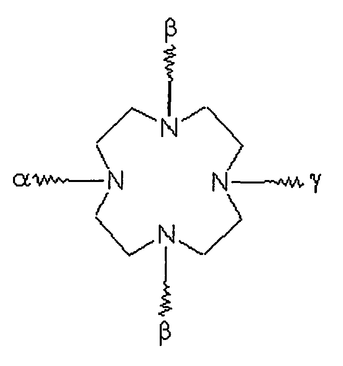
(e) 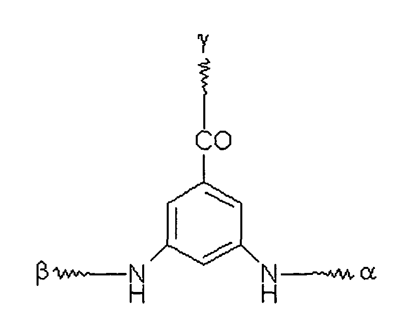
(f) 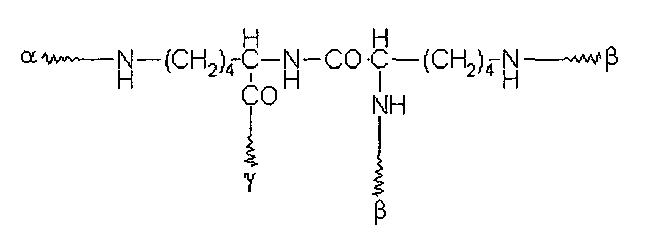
(g) 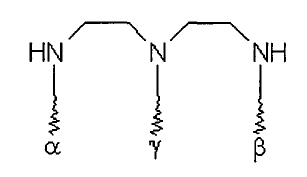
(h) 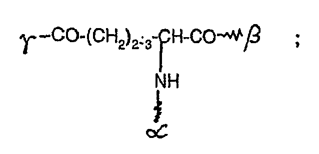
(i) 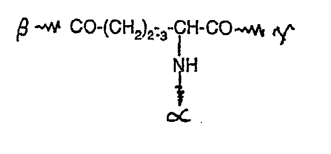
where α is the binding site of G to complex K, β is the binding site of G to radical R and γ represents the binding site of G to radical Z,
Z is 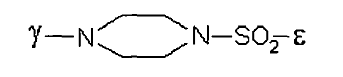
γ-C(O)CH₂ O(CH₂)₂-ε,
where γ is the binding site of Z to radical G, and ε is the binding site of Z to perfluorinated radical R_{f},
R is: a polar radical selected from complexes K of formula II to VII, where R¹ here is a hydrogen atom or a metal ion equivalent of atomic numbers 20-29, 31-33, 37-39, 42-44, 49 or 57-83,
and radicals R², R³, R⁴, U and U¹ have the above-indicated meaning, with the proviso that, when G is the radical (c) or (d) and R is a complex selected from formula II or V, R shall not be identical with the radical K of formula I if Z stands for -C(O)CH₂O(CH₂)₂-ε, or
a folic acid radical, or
a carbon chain with 2-30 C-atoms that is bonded via - CO-, -SO₂- or a direct bond to radical G, in a straight line or branched, saturated or unsaturated,
optionally interrupted by 1-10 oxygen atoms, 1-5 -NHCO groups, 1-5 -CONH groups, 1-2 sulphur atoms, 1-5 -NH groups or 1-2 phenylene groups, which phenylene groups optionally can be substituted with 1-2 OH groups, 1-2 NH₂ groups, 1-2 -COOH groups, or 1-2 -SO₃ H groups,
optionally substituted with 1-8 OH groups, 1-5 -COOH groups, 1-2 SO₃H groups, 1-5 NH₂ groups, 1-5 C₁-C₄-alkoxy groups, and
l, m, and p, independently of one another, mean the whole numbers 1 or 2.

2. Metal complexes according to Claim 1, **characterized in that** metal ion equivalent R¹ in radical K is an element of atomic numbers 21-29, 39, 42, 44 or 57-83.

3. Metal complexes according to Claim 1, **characterized in that** metal ion equivalent R¹ in radical K is an element of atomic numbers 27, 29, 31-33, 37-39, 43, 49, 62, 64, 70, 75 and 77.

4. Metal complexes according to any one of Claims 1 to 3, **characterized in that** K is a metal complex of formula II, III, VB or VII.

5. Metal complexes according to any one of Claims 1 to 4, **characterized in that** polar radical R has the meaning of complex K.

6. Metal complexes according to Claim 5, **characterized in that** polar radical R is one of complexes K of formulae II, III, VA or VII.

7. Metal complexes according to Claim 5 or 6, **characterized in that** R¹ is a metal ion equivalent of atomic numbers 20, 25 or 64.

8. Metal complexes according to any one of Claims 1 to 4, **characterized in that** polar radical R has one of the following meanings:
-C (O) CH₂CH₂SO₃H
-C (O) CH₂OCH₂CH₂OCH₂CH₂OH
-C (O) CH₂OCH₂CH₂OH
-C (O) CH₂OCH₂CH (OH) CH₂OH
-C (O) CH₂NH-C (O) CH₂COOH
-C (O) CH₂CH (OH) CH₂OH
-C (O) CH₂OCH₂COOH
-SO₂CH₂CH₂COOH
-C (O) -C₆Hg- (m-COOH) ₂
-C (O) CH₂O (CH₂)₂-C₆H₃- (m-COOH) ₂
-C (O) CH₂O-C₆H₄-m-SO₃H
-C (O) CH₂NHC (O) CH₂NHC (O) CH₂OCH₂COOH
-C (O) CH₂OCH₂CH₂OCH₂COOH
-C (O) CH₂OCH₂C (OH) CH₂O-CH₂CH₂OH
-C (O) CH₂OCH₂C (OH) CH₂OCH₂-CH (OH) -CH₂OH
-C (O) CH₂SO₃H
-C(O)CH₂CH₂COOH
-C(O)CH(OH)CH(OH)CH₂OH
-C (O) CH₂O [(CH₂)₂O]₁₋₉-CH₃
-C (O) CH₂O [(CH₂)₂O]₁₋₉-H
-C (O) CH₂OCH (CH₂OH)₂
-C (O) CH₂OCH (CH₂OCH₂COOH) ₂
-C (O) -C₆H₃- (m-OCH₂COOH) ₂
-CO-CH₂O- (CH₂)₂O (CH₂) ₂O- (CH₂) ₂O (CH₂) ₂OCH₃
preferably -C (O) CH₂O [(CH₂)₂O]₄-CH₃.

9. Metal complexes according to any one of Claims 1 to 4, **characterized in that** polar radical R is the folic acid radical.

10. Metal complexes according to any one of Claims 1 to 9, **characterized in that** G in formula I is a lysine radical (a) or (b).

11. Metal complexes according to any one of Claims 1 to 10, **characterized in that** U in metal complex K represents the group -CH₂- or -C₆H₄-O-CH₂ -ω, where ω stands for the binding site to -CO-.

12. Use of metal complexes according to Claim 2 in the manufacture of contrast media for use in NMR diagnosis or x-ray diagnosis.

13. Use of metal complexes according to Claim 12 in the manufacture of contrast media for infarction or necrosis imaging.

14. Use of metal complexes according to Claim 3 in the manufacture of contrast media for use in radiodiagnosis or radiotherapy.

15. Use of metal complexes according to Claim 2 in the manufacture of contrast media for lymphography in the diagnosis of changes in the lymphatic system.

16. Use of metal complexes according to Claim 2 in the manufacture of contrast media for use in indirect lympography.

17. Use of metal complexes according to Claim 2 in the manufacture of contrast media for use in intravenous lympography.

18. Use of metal complexes according to Claim 2 in the manufacture of contrast media for visualizing the vascular space.

19. Use of metal complexes according to Claim 2 in the manufacture of contrast media for tumour imaging.

20. Pharmaceutical composition comprising at least one physiologically compatible compound according to Claims 1 to 11, optionally with the additives that are commonly used in galenicals.

21. Process for the production of perfluoroalkyl-containing complexes with polar radicals of formula I of claim 1: 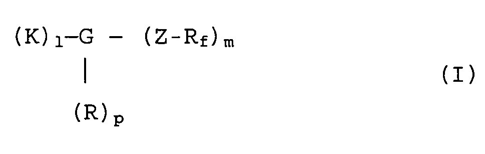 in which K, G, R, Z, R_{f}, l, m and p have the meaning that is indicated in Claim 1, **characterized in that** a carboxylic acid of formula IIa 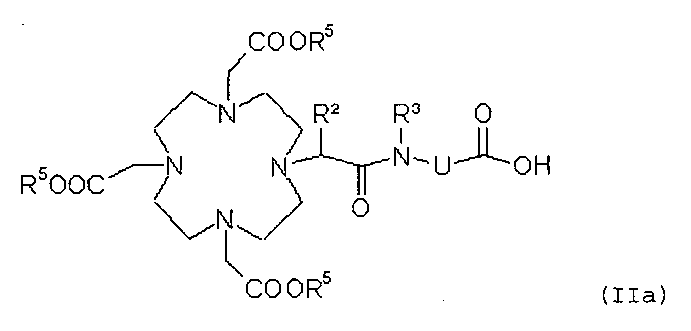 in which R⁵ is a metal ion eqiuvalent of atomic numbers 21-29, 31-33, 37-39, 42-44, 49 or 57-83 or a carboxyl protective group, and R², R³ and U have the abovementioned meaning,
or a carboxylic acid of formula IIIa 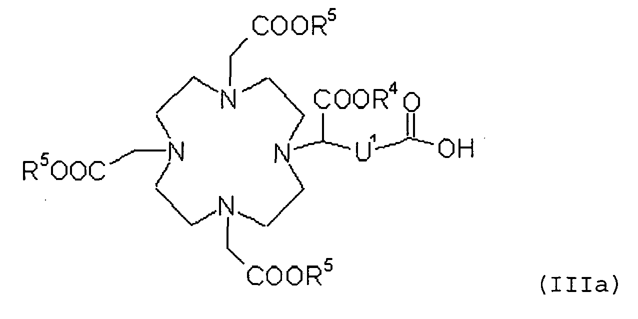 in which R⁴, R⁵, and U¹ have the above-mentioned meaning or a carboxylic acid of formula IVa 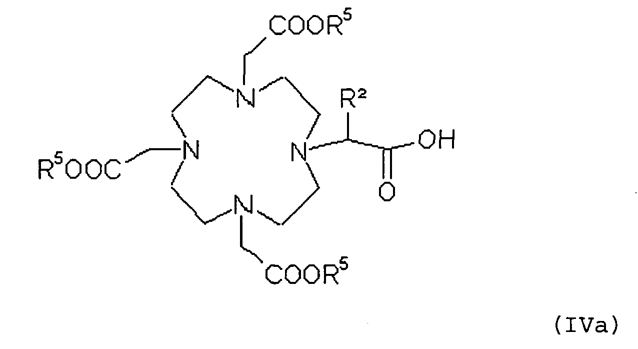 in which R⁵ and R² have the above-mentioned meaning
or a carboxylic acid of formula Va or Vb 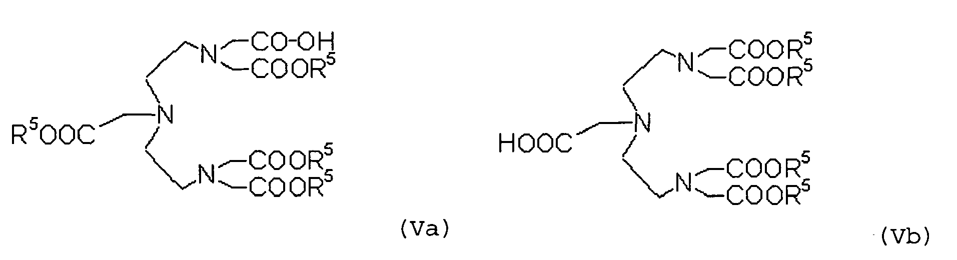 in which R⁵ has the above-mentioned meaning
or a carboxylic acid of formula VIa 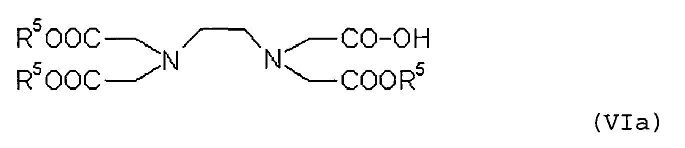 in which R⁵ has the above-mentioned meaning
or a carboxylic acid of formula VIIa 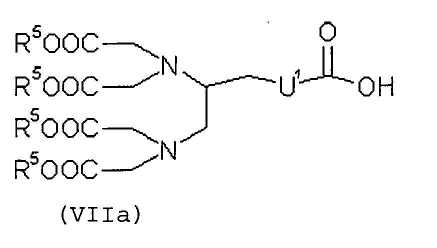 in which R⁵ and U¹ have the above-mentioned meanings, in optionally activated form, is reacted with an amine of formula VIII 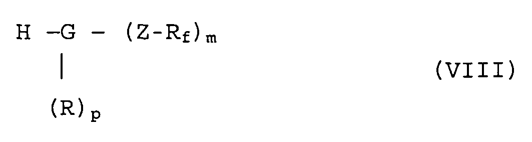 in which G, R, Z, R_{f}, m and p have the indicated meaning, in a coupling reaction and optionally subsequently cleaving optionally present protective groups to provide a metal complex of formula I, or
if R⁵ is a protective group, reacting after cleavage of these protective groups in a subsequent step in a manner known per se with at least one metal oxide or metal salt of an element of atomic numbers 21-29, 31-33, 37-39, 42-44, 49 or 57-83, and then optionally present acidic hydrogen atoms are optionally substituted by cations of inorganic and/or organic bases, amino acids or amino acid amides.

## Revendications

1. Complexes perfluoroalkylés à résidus polaires de formule générale I 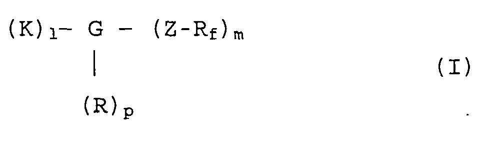 dans laquelle
R_{f} est une chaîne hydrocarbonée perfluorée droite ou ramifiée de formule -CₙF₂ₙE, dans laquelle E représente un atome de fluor, de chlore, de brome, d'iode ou, d'hydrogène en bout de chaîne et n représente les nombres 4-30,
K représente un complexe métallique de formule générale II, 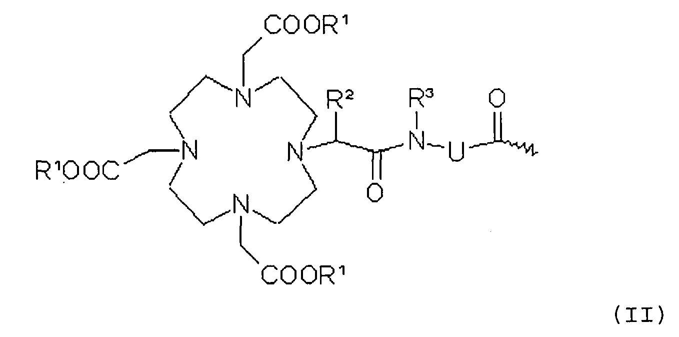
dans laquelle
R¹ représente un atome d'hydrogène ou un équivalent d'ion métallique des nombres atomiques 21-29, 31-33, 37-39, 42-44, 49 ou 57-83,
étant entendu qu'au moins deux radicaux R¹ représentent des équivalents d'ions métalliques,
R² et R³ représentent, indépendamment l'un de l'autre, un atome d'hydrogène, un groupe alkyle en C₁-C₇, benzyle, phényle, -CH₂OH ou -CH₂OCH₃ et
U représente -C₆H₄-O-CH₂-CH₂-ω, - (CH₂)₁₋₅-ω, un groupe phénylène,
-CH₂-NHCO-CH₂-CH(CH₂COOH)-C₆H₄-ω-,
-C₆H₄- (OCH₂CH₂)₀₋₁-N(CH₂COOH)-CH₂-ω ou un groupe alkylène en C₁-C₁₂ ou C₇-C₁₂-C₆H₄-O-éventuellement interrompu par un ou plusieurs atomes d'oxygène, 1 à 3 groupes -NHCO-, 1 à 3 groupes -CONH- et/ou substitué par 1 à 3 groupes - (CH₂)₀₋₅COOH, ω représentant le point de liaison à -CO-
ou
de formule générale III 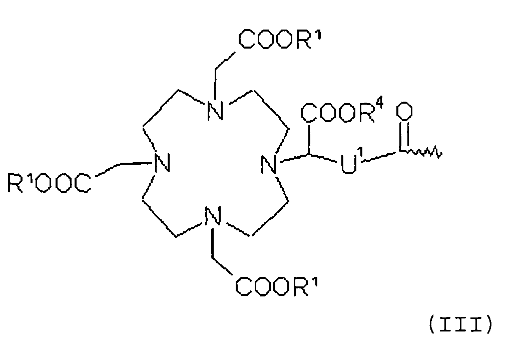 dans laquelle R¹ a la signification donnée ci-dessus, R⁴ représente un atome d'hydrogène ou un équivalent d'ion métallique mentionné sous R¹ et U¹ représente -C₆H₄-O-CH₂-ω-, ω représentant le point de liaison à -CO-,
ou de formule générale IV 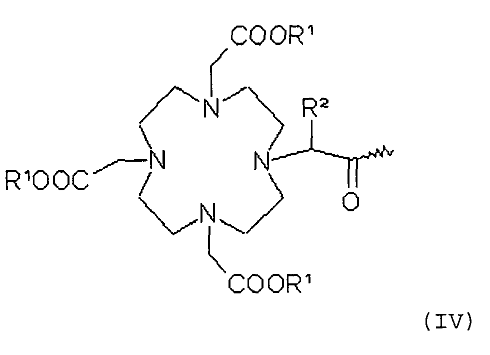 dans laquelle R¹ et R² ont les significations données plus haut
ou de formule générale V A ou V B 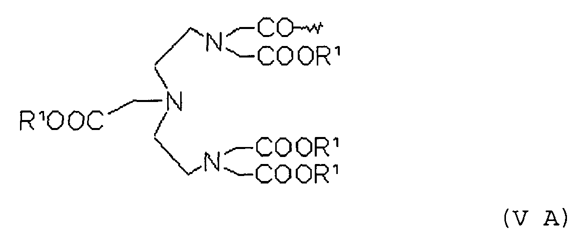 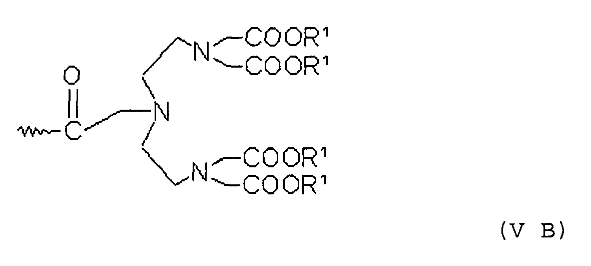 formules dans lesquelles R¹ a la signification donnée plus haut,
ou de formule générale VI 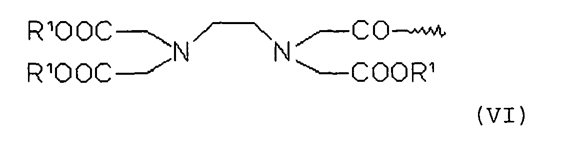 dans laquelle R¹ a la signification donnée plus haut,
ou de formule générale VII 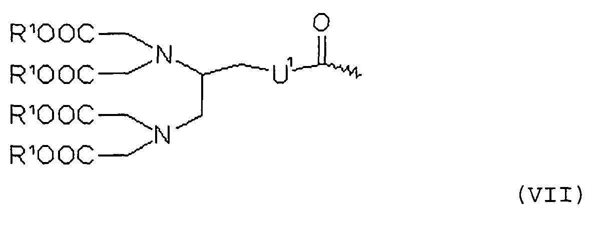 dans laquelle R¹ a la signification donnée plus haut et
U représente -C₆H₄-O-CH₂-ω-, ω représentant le point de liaison à -CO-
et des groupes acides libres éventuellement présents dans le résidu K pouvant éventuellement se trouver sous forme de sels de bases organiques et/ou minérales ou d'acides aminés ou d'amides d'acides aminés,
G représente un radical au moins trois fois fonctionnalisé, choisi parmi les radicaux a) à g) suivants
(a) 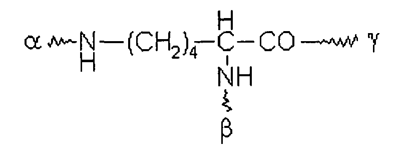
(b) 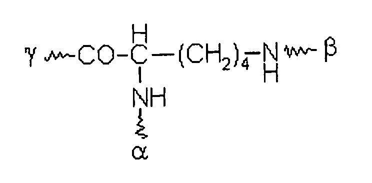
(c) 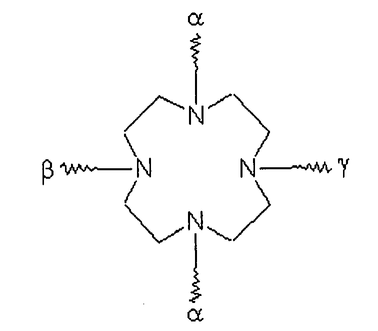
(d) 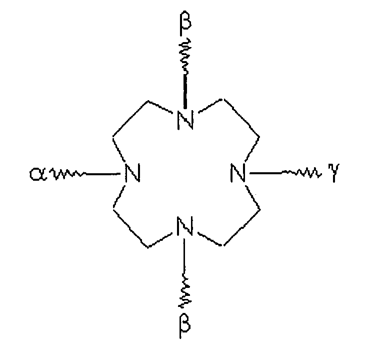
(e) 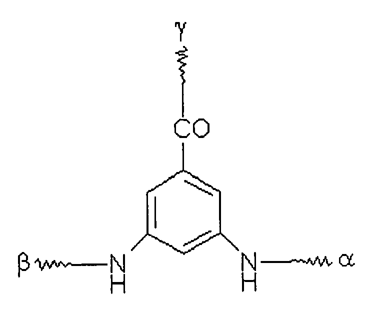
(f) 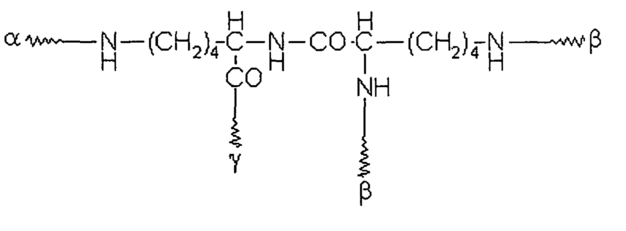
(g) 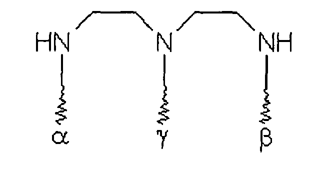
(h) 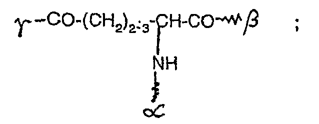
(i) 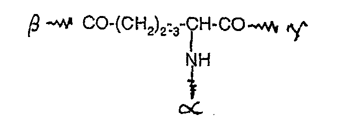
α représentant le point de liaison de G au complexe K, β représentant le point de liaison de G au radical R et γ représentant le point de liaison de G au radical Z
Z représente 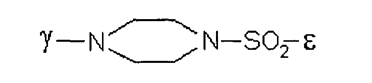
γ- C (O) CH₂O (CH₂)₂ - ε,
γ représentant le point de liaison de Z au radical G et ε représentant le point de liaison de Z au radical perfluoré R_{f},
R représente un radical polaire choisi parmi les complexes K de formules générales II à VII, R¹ représentant en ce cas un atome un atome d'hydrogène ou un équivalent d'ion métallique des nombres atomiques 20-29, 31-33, 37-39, 42-44, 49 ou 57-83,
et les radicaux R², R³, R⁴, U et U¹ ont les significations données plus haut, dans le cas où G représente le radical (c) ou (d) et R un complexe choisi parmi les formules générales II et V, R ne pouvant pas être identique au résidu K de formule générale I lorsque Z représente δ-C(O)CH₂O(CH₂)₂-ε,
ou
le reste d'acide folique
ou
une chaîne carbonée droite ou ramifiée, saturée ou insaturée, ayant 2-30 atomes de carbone, liée au radical G par -CO-, SO₂- ou une liaison directe,
éventuellement interrompue par 1-10 atomes d'oxygène, 1-5 groupes -NHCO-, 1-5 groupes -CONH-, 1-2 atomes de soufre, 1-5 groupes -NH- ou 1-2 groupes phénylène, qui peuvent éventuellement être substitués par 1-2 groupes OH, 1-2 groupes NH₂, 1-2 groupes -COOH ou 1-2 groupes -SO₃H
ou
éventuellement substitué par 1-8 groupes OH, 1-5 groupes -COOH, 1-2 groupes SO₃H, 1-5 groupes NH₂, 1-5 groupes alcoxy en C₁-C₄,
et
l, m, p représentent chacun indépendamment le nombre entier 1 ou 2.

2. Complexes métalliques selon la revendication 1, **caractérisés en ce que** l'équivalent d'ion métallique R¹ dans le résidu K est un élément des nombres atomiques 21-29, 39, 42, 44 ou 57-83.

3. Complexes métalliques selon la revendication 1, **caractérisés en ce que** l'équivalent d'ion métallique R¹ dans le résidu K est un élément des nombres atomiques 27, 29, 31-33, 37-39, 43, 49, 62, 64, 70, 75 et 77.

4. Complexes métalliques selon l'une quelconque des revendications 1 à 3, **caractérisés en ce que** K représente un complexe métallique de formule générale II, III, VB ou VII.

5. Complexes métalliques selon l'une quelconque des revendications 1 à 4, **caractérisés en ce que** le résidu polaire R a la signification du complexe K.

6. Complexes métalliques selon la revendication 5, **caractérisés en ce que** les complexes K de formules générales II, III, V A ou VII sont présents en tant que résidus polaires R.

7. Complexes métalliques selon la revendication 5 ou 6, **caractérisés en ce que** R¹ représente un équivalent d'ion métallique des nombres atomiques 20, 25 ou 64.

8. Complexes métalliques selon l'une quelconque des revendications 1 à 4, **caractérisés en ce que** le résidu polaire R a les significations suivantes :
-C(O) CH₂CH₂SO₃H
-C(O) CH₂OCH₂CH₂OCH₂CH₂OH
- C(O) CH₂OCH₂CH₂OH
-C(O) CH₂OCH₂CH (OH) CH₂OH
-C(O) CH₂NH-C(O)CH₂COOH
-C(O)CH₂CH(OH)CH₂OH
-C(O) CH₂OCH₂ COOH
- SO₂CH₂CH₂COOH
-C(O) C₆H₃ - (m-COOH) ₂
- C(O) CH₂O (CH₂)₂-C₆H₃- (m-COOH) ₂
- C (O) CH₂O-C₆H₄-m-SO₃H
- C(O) CH₂NHC (O) CH₂NHC (O) CH₂OCH₂COOH
-C (O) CH₂OCH₂CH₂OCH₂COOH
-C (O) CH₂OCH₂CH (OH) CH₂O-CH₂CH₂OH
-C (O) CH₂OCH₂CH (OH) CH₂OCH₂-CH (OH) -CH₂OH
-C (O) CH₂SO₃H
-C (O) CH₂CH₂COOH
-C(O)CH(OH)CH(OH)CH₂OH
-C (O) CH₂O [(CH₂) ₂O] ₁₋₉-CH₃
-C (O) CH₂O [(CH₂)₂O]₁₋₉-H
-C (O) CH₂OCH (CH₂OH) ₂
-C(O) CH₂OCH (CH₂OCH₂COOH) ₂
-C (O)-C₆H₃- (m-OCH₂COOH)₂
-CO-CH₂O- (CH₂) ₂O (CH₂) ₂O- (CH₂) ₂O (CH₂)₂OCH₃
de préférence -C(O)CH₂O[(CH₂)₂O]₄-CH₃.

9. Complexes métalliques selon l'une quelconque des revendications 1 à 4, **caractérisés en ce que** le résidu polaire R est le reste de l'acide folique.

10. Complexes métalliques selon l'une quelconque des revendications 1 à 9, **caractérisés en ce que** G dans la formule I représente le reste lysine (a) ou (b).

11. Complexes métalliques selon l'une quelconque des revendications 1 à 10, **caractérisés en ce que** U représente dans le complexe métallique K le groupe -CH₂- ou -C₆H₄-O-CH₂-ω, ω représentant le point de liaison à -CO-.

12. Utilisation de complexes métalliques selon la revendication 2, pour la préparation de produits de contraste destinés à être utilisés dans le diagnostic radiographique ou par RMN.

13. Utilisation de complexes métalliques selon la revendication 12, pour la préparation de produits de contraste destinés à être utilisés dans l'imagerie d'infarctus et de nécroses.

14. Utilisation de complexes métalliques selon la revendication 3, pour la préparation de produits de contraste destinés à être utilisés dans le diagnostic radiologique et la radiothérapie.

15. Utilisation de complexes métalliques selon la revendication 2, pour la préparation de produits de contraste pour la lymphographie destinée au diagnostic d'altérations du système lymphatique.

16. Utilisation de complexes métalliques selon la revendication 2, pour la préparation de produits de contraste destinés à être utilisés dans la lymphographie indirecte.

17. Utilisation de complexes métalliques selon la revendication 2, pour la préparation de produits de contraste destinés à être utilisés dans la lymphographie intraveineuse.

18. Utilisation de complexes métalliques selon la revendication 2, pour la préparation de produits de contraste destinés à la représentation de l'espace vasculaire.

19. Utilisation de complexes métalliques selon la revendication 2, pour la préparation de produits de contraste pour l'imagerie de tumeurs.

20. Composition pharmaceutique contenant au moins un composé physiologiquement acceptable selon les revendications 1 à 11, éventuellement avec les additifs usuels en galénique.

21. Procédé pour la préparation de complexes perfluoroalkylés à résidus polaires de formule générale I 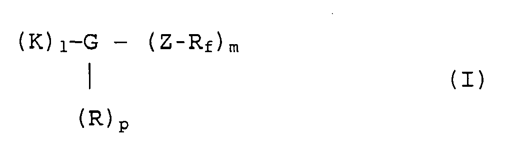 dans laquelle K, G, R, Z, R_{f}, l, m et p ont les significations données dans la revendication 1,
**caractérisé en ce qu'**on fait réagir d'une façon connue en soi un acide carboxylique de formule générale IIa 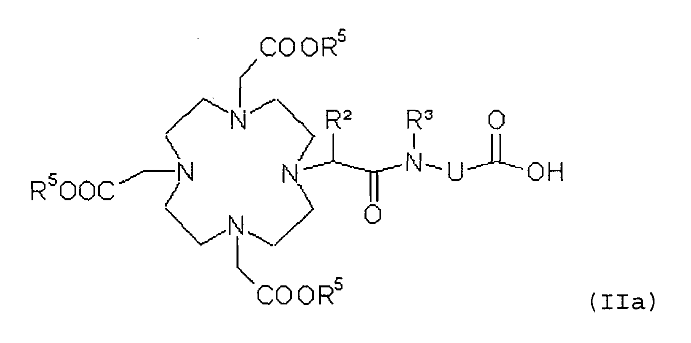 dans laquelle R⁵ représente un équivalent d'ion métallique des nombres atomiques 21-29, 31-33, 37-39, 42-44, 49 ou 57-83, ou un groupe protecteur de fonction carboxy, et R² R³ et U ont les significations données
ou un acide carboxylique de formule générale IIIa 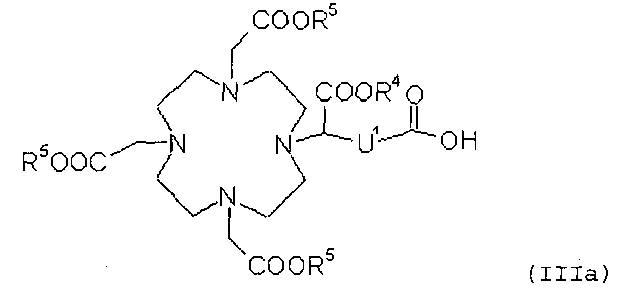 dans laquelle R⁴, R⁵ et U¹ ont les significations données
ou un acide carboxylique de formule générale IVa 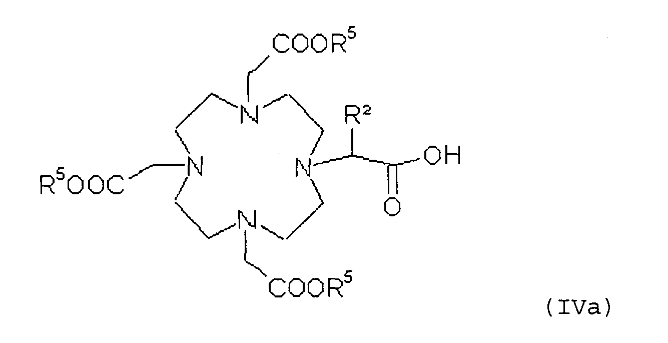 dans laquelle R⁵ et R² ont les significations données
ou un acide carboxylique de formule générale Va ou Vb 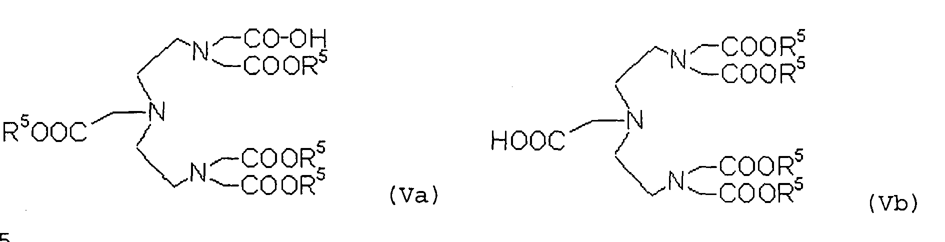 formules dans lesquelles R⁵ a la signification donnée
ou un acide carboxylique de formule générale VIa 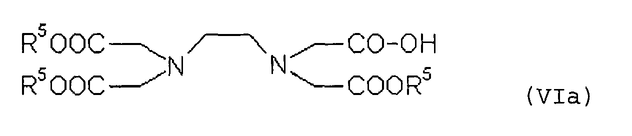 dans laquelle R⁵ a la signification donnée
ou un acide carboxylique de formule générale VIIa 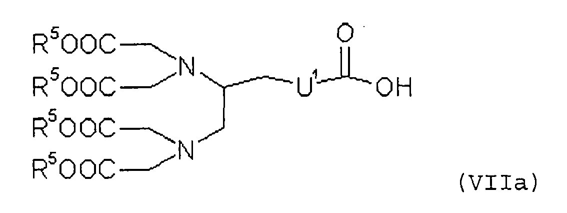 dans laquelle R⁵ et U¹ ont les significations données,
sous forme éventuellement activée, avec une amine de formule générale VIII 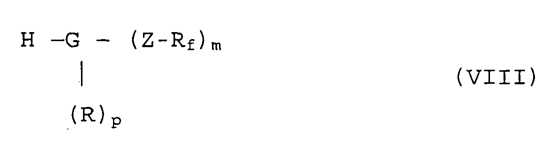 dans laquelle G, R, Z, R_{f}, m et p ont les significations données, dans une réaction de couplage et éventuellement élimination subséquente de groupes protecteurs éventuellement présents, pour obtenir un complexe métallique de formule générale I
ou
lorsque R⁵ a la signification d'un groupe protecteur, après élimination de ces groupes protecteurs on le fait réagir dans une étape subséquente, d'une façon connue en soi, avec au moins un oxyde métallique ou sel métallique d'un élément des nombres atomiques 21-29, 31-33, 37-39, 42-44, 49 ou 57-83, et ensuite, si on le désire, on remplace des atomes d'hydrogène acides éventuellement présents par des cations de bases organiques et/ou minérales, d'acides aminés ou d'amides d'acides aminés.
